# EUROPEAN PATENT SPECIFICATION

(11) **EP 4 410 804 B1**
(45) Date of publication and mention of the grant of the patent: **18.02.2026**
(21) Application number: 22876257.1
(22) Date of filing: 27.09.2022
(51) Int. Cl.: C12Q 1/6806, C07H 21/04, C12M 1/00

(54) **NUCLEIC ACID STRAND CLEAVING METHOD, NUCLEIC ACID STRAND CLEAVING DEVICE, DOUBLE-STRANDED DNA PRODUCTION METHOD, AND DOUBLE-STRANDED DNA PRODUCTION DEVICE**
NUKLEINSÄURESTRANGSPALTUNGSVERFAHREN, NUKLEINSÄURESTRANGSPALTUNGSVORRICHTUNG, VERFAHREN ZUR HERSTELLUNG VON DOPPELSTRÄNGIGER DNA UND VORRICHTUNG ZUR HERSTELLUNG VON DOPPELSTRÄNGIGER DNA
PROCÉDÉ DE CLIVAGE DE BRIN D'ACIDE NUCLÉIQUE, DISPOSITIF DE CLIVAGE DE BRIN D'ACIDE NUCLÉIQUE, PROCÉDÉ DE PRODUCTION D'ADN DOUBLE BRIN ET DISPOSITIF DE PRODUCTION D'ADN DOUBLE BRIN

(30) Priority: 29.09.2021 JP 2021158659
(43) Date of publication of application: 07.08.2024
(73) Proprietor: Japan Science and Technology Agency, Kawaguchi-shi, Saitama 332-0012 (JP)
(72) Inventor: ABE Hiroshi, Nagoya-shi, Aichi 464-8601 (JP); INAGAKI Masahito, Nagoya-shi, Aichi 464-8601 (JP); HASHIYA Fumitaka, Nagoya-shi, Aichi 464-8601 (JP); HIRAOKA Haruka, Nagoya-shi, Aichi 464-8601 (JP); ABE Naoko, Nagoya-shi, Aichi 464-8601 (JP)
(74) Representative: Grünecker Patent- und Rechtsanwälte PartG mbB
(86) International application number: PCT/JP2022/036009
(87) International publication number: WO 2023/054391

(56) References cited:
- EP-A1- 4 006 041
- WO-A1-2015/164957
- WO-A1-2021/020561
- WO-A1-94/00471
- GB-A- 2 236 852
- MAG M ET AL: "Synthesis and selective cleavage of an oligodeoxynucleotide containing a bridged internucleotide 5'-phosphorothioate linkage", NUCLEIC ACIDS RESEARCH, vol. 19, no. 7, 1 April 1991 (1991-04-01), pages 1437 - 1441, XP093311655, ISSN: 0305-1048, DOI: 10.1093/nar/19.7.1437
- SUN MAOZHONG ET AL: "Site-selective photoinduced cleavage and profiling of DNA by chiral semiconductor nanoparticles", vol. 10, no. 8, 20 July 2018 (2018-07-20), London, pages 821 - 830, XP093311591, ISSN: 1755-4330, Retrieved from the Internet <URL:http://www.nature.com/articles/s41557-018-0083-y.pdf> DOI: 10.1038/s41557-018-0083-y
- MOUSAVI-KHATTAT MOHAMMAD ET AL: "A comparative study of stability, antioxidant, DNA cleavage and antibacterial activities of green and chemically synthesized silver nanoparticles", ARTIFICIAL CELLS, NANOMEDICINE AND BIOTECHNOLOGY, vol. 46, no. sup3, 12 November 2018 (2018-11-12), US, pages 1022 - 1031, XP093311620, ISSN: 2169-1401, DOI: 10.1080/21691401.2018.1527346
- HU SHENGQIANG ET AL: "Phosphorothioate DNA Mediated Sequence-Insensitive Etching and Ripening of Silver Nanoparticles", FRONTIERS IN CHEMISTRY, vol. 7, 16 April 2019 (2019-04-16), Switzerland, XP093311634, ISSN: 2296-2646, DOI: 10.3389/fchem.2019.00198
- COOK A F, HOLMAN M J, NUSSBAUM A L: "Nucleoside S-alkyl phosphorothioates. II. Preparation and chemical and enzymatic properties", JOURNAL OF THE AMERICAN CHEMICAL SOCIETY, vol. 91, no. 6, 12 March 1969 (1969-03-12), pages 1522 - 1527, XP002597133, ISSN: 0002-7863, DOI: 10.1021/ja01034a040
- COSSTICK RICHARD, VYLE JOSEPH S.: "Synthesis and phosphorus–sulphur bond cleavage of 3′-thiothymidylyl(3′-5′)thymidine", JOURNAL OF THE CHEMICAL SOCIETY, no. 15, 1 January 1988 (1988-01-01), GB , pages 992 - 993, XP093053165, ISSN: 0022-4936, DOI: 10.1039/C39880000992
- VYLE J S ET AL: "Sequence- and strand-specific cleavage in oligodeoxyribonucleotides and DNA containing 3'-thiothymidine.", BIOCHEMISTRY, vol. 31, no. 11, 24 March 1992 (1992-03-24), pages 3012 - 3018, XP002417213, ISSN: 0006-2960, DOI: 10.1021/bi00126a024
- ELZAGHEID MOHAMED I, MATTILA KATI, OIVANEN MIKKO, JONES BRYAN C N M, COSSTICK RICHARD, LÖNNBERG HARRI: "Hydrolytic Reactions of the cis-Methyl Ester of 3Ј-Deoxy-3Ј-thiothymidine 3Ј,5Ј-Cyclic(phosphorothiolate)", EUROPEAN JOURNAL OF ORGANIC CHEMISTRY, vol. 2000, no. 10, 1 May 2000 (2000-05-01), pages 1987 - 1991, XP093053169

## Description

### Technical Field

The present invention relates to a nucleic acid strand cleaving method, a nucleic acid strand cleaving device, a double-stranded DNA production method, and a double-stranded DNA production device.

### Background Art

In the field of molecular biology or the like, a vector obtained by incorporating a target DNA into a host has been used in order to perform gene recombination, transformation, or the like. In general, the target DNA is amplified for use by polymerase chain reaction (PCR) in a case where the amount is small. In the PCR, by using a template DNA containing a target DNA sequence and using a primer that binds complementarily to the template DNA, the template DNA is amplified by repeating a cycle of thermal denaturation and annealing multiple times.

An amplification product obtained by amplifying a template DNA by PCR has blunt ends originally, and it is necessary to perform a treatment for binding (ligating) the amplification product to a host DNA such as a plasmid DNA. In this case, there is a technique for constructing a vector by forming the 3' end and 5' end of an amplification product as sticky ends (also referred to as "cohesive ends", "protruding ends" or the like), forming sticky ends also on the host side in a similar manner, and ligating both of the ends.

In order to form a sticky end on DNA, it is necessary to cleave a desired position of one strand of double-stranded DNA to generate an overhang on the DNA strand. In this regard, for example, Non Patent Literature 1 describes that an oligonucleotide containing a 3'-S-phosphorothioate bond was prepared by an automatic DNA synthesizer using 5'-O-monomethoxytritylthymidine 3'-S-(2-cyanoethyl)-N,N-diisopropylphosphorothioamidite, a strand containing a 3'-S-phosphorothioate bond was specifically and chemically cleaved at this site using Ag⁺, and the like (Abstract).

### Citation List

### Non Patent Literature

Non Patent Literature 1: "Sequence-and strand-specific cleavage in oligodeoxyribonucleotides and DNA containing 3'-thiothymidine." Joseph S. Vyle, et al. Biochemistry, 31, 3012 (1992)

Patent Literature

WO 2021/020561

### Summary of Invention

### Technical Problem

In Non Patent Literature 1, a silver ion (Ag⁺) is used as a cleaving agent for cleaving single-stranded DNA. Since silver ions have a positive charge, the silver ions have electrostatic interaction with a functional group having a negative charge such as a phosphate group of DNA or a nucleic acid salt group, and thus the aggregate formation due to non-specific interaction causes a low recovery rate of target DNA. WO 2021/020561 discloses a method for site-specific cleavage of nucleic acids by modifying the nucleic acid with a photodecomposable nitro compound that is removed by photoirradiation.

An object of the present invention is to provide a nucleic acid strand cleaving method and a nucleic acid strand cleaving device which have a high cleavage efficiency and a high recovery rate of a target nucleic acid. Another object of the present invention is to provide a double-stranded DNA production method and a double-stranded DNA production device having a sticky end, the method which have a high sticky end formation efficiency and a high recovery rate of a double-stranded DNA having a target sticky end.

### Solution to Problem

The present inventors have intensively studied to solve the problems. As a result, the present inventors have found that by using metal nanoparticles as a cleaving agent instead of metal ions, the cleavage efficiency of a nucleic acid to be cleaved is good, and the recovery rate of a target nucleic acid is high, thereby completing the present invention.
[1] A nucleic acid strand cleaving method including:
   a nucleic acid preparation step of preparing a nucleic acid to be cleaved having a structure represented by the following Formula (1); and
   a cleaving step of reacting the nucleic acid to be cleaved with a cleaving agent to cleave the nucleic acid to be cleaved at the part X of the Formula (1) to generate a nucleic acid having a structure represented by the following Formula (2), in which
   the cleaving agent is a metal nanoparticle containing an atom selected from the group consisting of silver, mercury, and cadmium. 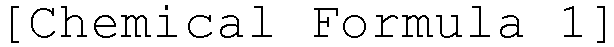 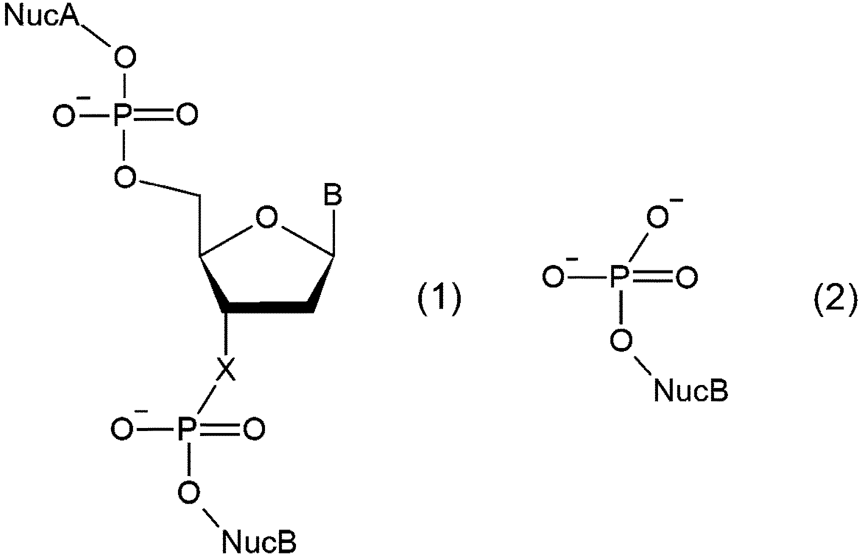 (Here, B represents a base, and X represents sulfur or selenium. NucA is composed of at least one nucleotide and is a part of the nucleic acid to be cleaved, and represents a part on the 5' end side with reference to the X. NucB is composed of at least one nucleotide and is a part of the nucleic acid to be cleaved, and represents a part on the 3' end side with reference to the X.)
[2] The nucleic acid strand cleaving method according to [1], in which the cleaving agent is silver nanoparticles, and the X is sulfur.
[3] The nucleic acid strand cleaving method according to [1], wherein the metal nanoparticles have an average particle size within a range of 1 to 20 nm.
[4] The nucleic acid strand cleaving method according to [1], in which polyethylene glycol is bound to the surface of the metal nanoparticle.
[5] The nucleic acid strand cleaving method according to claim 1, in which in the nucleic acid preparation step, a part or all of the nucleic acid to be cleaved is synthesized by a phosphoramidite method using an amidite reagent represented by the following Formula (3) and Formula (4). 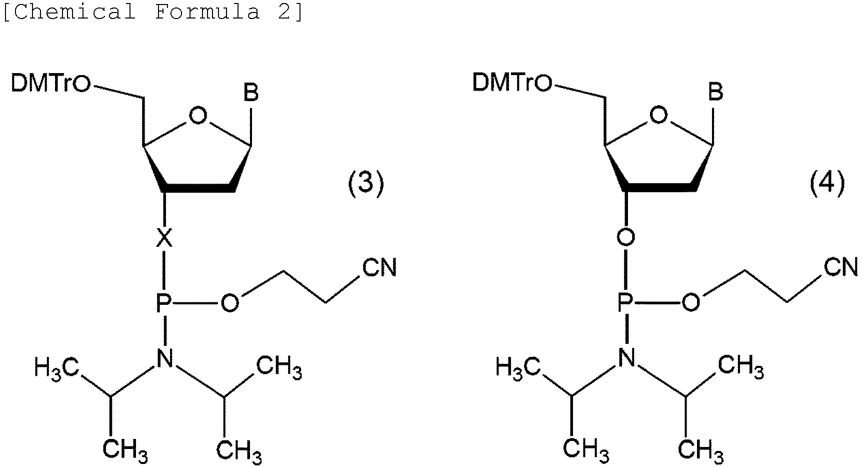 (Here, B represents a base, X represents sulfur or selenium, and DMTr represents a dimethoxytrityl group.)
[6] The nucleic acid strand cleaving method according to [5], in which the nucleic acid preparation step includes synthesizing 5-[3,5-bis (trifluoromethyl)phenyl]-1 H-tetrazole as an activator by a phosphoramidite method.
[7] The nucleic acid strand cleaving method according to [5], in which in the nucleic acid preparation step, a part of the nucleic acid to be cleaved is synthesized by a phosphoramidite method to form a primer, a plurality of cycles of polymerase chain reaction is performed using a template DNA having a sequence complementary to the nucleic acid to be cleaved as a template, and the primer is extended along the template DNA to generate the nucleic acid to be cleaved.
[8] A nucleic acid strand cleaving device including:
   nucleic acid preparation means that prepares a nucleic acid to be cleaved having a structure represented by the following Formula (1); and
   cleaving means that reacts the nucleic acid to be cleaved with a cleaving agent to cleave the nucleic acid to be cleaved at the part X of the Formula (1) to generate a nucleic acid having a structure represented by the following Formula (2), in which
   the cleaving agent is a metal nanoparticle containing an atom selected from the group consisting of silver, mercury, and cadmium. 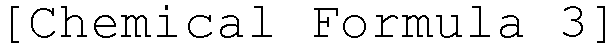 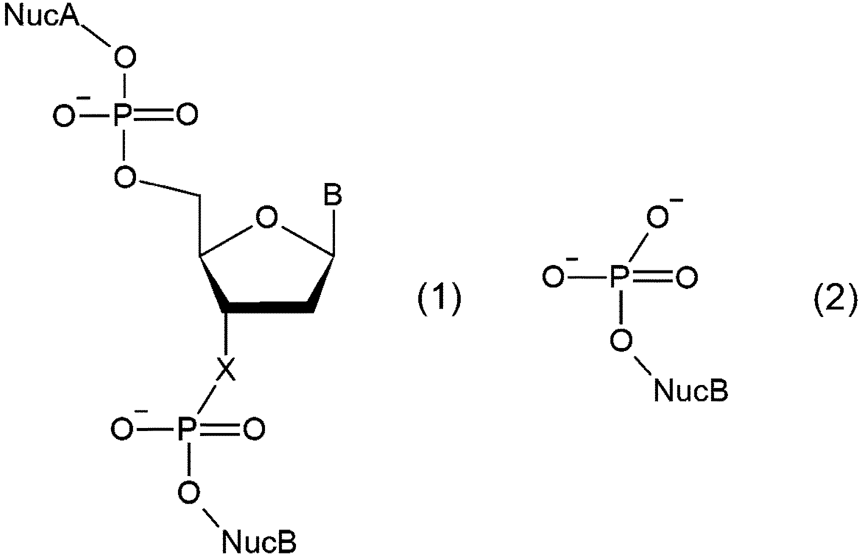 (Here, B represents a base, and X represents sulfur or selenium. NucA is composed of at least one nucleotide and is a part of the nucleic acid to be cleaved, and represents a part on the 5' end side with reference to the X. NucB is composed of at least one nucleotide and is a part of the nucleic acid to be cleaved, and represents a part on the 3' end side with reference to the X.)
[9] The nucleic acid strand cleaving device according to [8], wherein the nucleic acid preparation means synthesizes a part or all of the nucleic acid to be cleaved by a phosphoramidite method using an amidite reagent represented by the following Formula (3) and Formula (4). 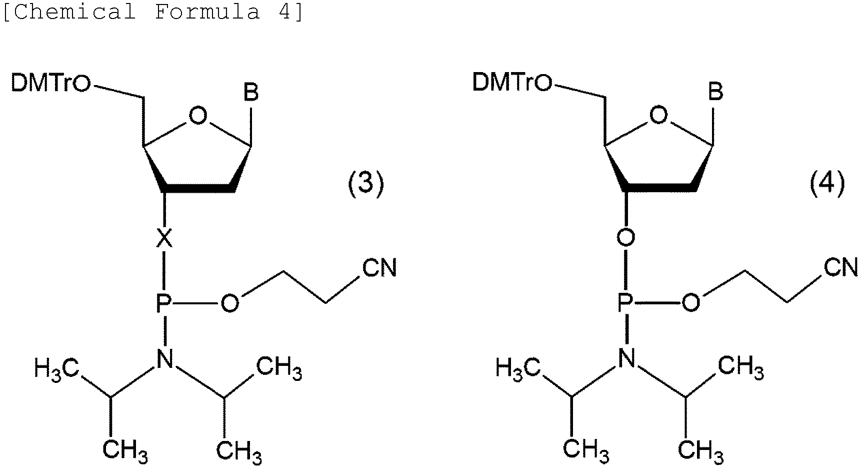 (Here, B represents a base, X represents sulfur or selenium, and DMTr represents a dimethoxytrityl group.)
[10] The nucleic acid strand cleaving method according to [9], in which the nucleic acid preparation step includes synthesizing 5-[3,5-bis (trifluoromethyl)phenyl]-1 H-tetrazole as an activator by a phosphoramidite method.
[11] A method for producing double-stranded DNA for producing double-stranded DNA having a sticky end, the method including:
   a double-stranded DNA preparation step of preparing double-stranded DNA to be cleaved including a sense strand and an antisense strand having a sequence complementary to the sense strand, the double-stranded DNA to be cleaved having a structure represented by the following Formula (1) in at least one of the sense strand and the antisense strand; and
   a sticky end generation step of reacting the double-stranded DNA to be cleaved with a cleaving agent to cleave the sense strand and/or the antisense strand at a portion of X in the Formula (1) to generate double-stranded DNA having a structure represented by the following Formula (2) and having a sticky end, in which
   the cleaving agent is a metal nanoparticle containing an atom selected from the group consisting of silver, mercury, and cadmium. 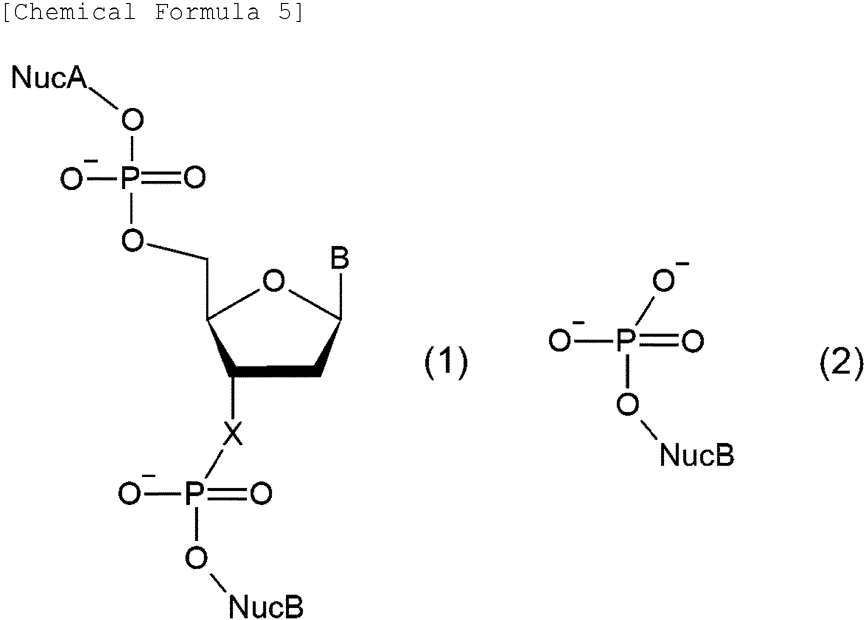 (Here, B represents a base, and X represents sulfur or selenium. NucA is composed of at least one nucleotide and is a part of the nucleic acid to be cleaved, and represents a part on the 5' end side with reference to the X. NucB is composed of at least one nucleotide and is a part of the nucleic acid to be cleaved, and represents a part on the 3' end side with reference to the X.)
[12] The double-stranded DNA production method according to [11], in which the double-stranded DNA to be cleaved has a plurality of structures represented by the Formula (1), and the number of nucleotides between the structures is 10 or less.
[13] A device for producing double-stranded DNA for producing double-stranded DNA having a sticky end, the device comprising:
   a double-stranded DNA preparation means for preparing double-stranded DNA to be cleaved including a sense strand and an antisense strand having a sequence complementary to the sense strand, the double-stranded DNA to be cleaved having a structure represented by the following Formula (1) in at least one of the sense strand and the antisense strand; and
   a sticky end generation means for reacting the double-stranded DNA to be cleaved with a cleaving agent to cleave the sense strand and/or the antisense strand at a portion of X in the Formula (1) to generate double-stranded DNA having a structure represented by the following Formula (2) and having a sticky end,
   the cleaving agent is a metal nanoparticle containing an atom selected from the group consisting of silver, mercury, and cadmium. 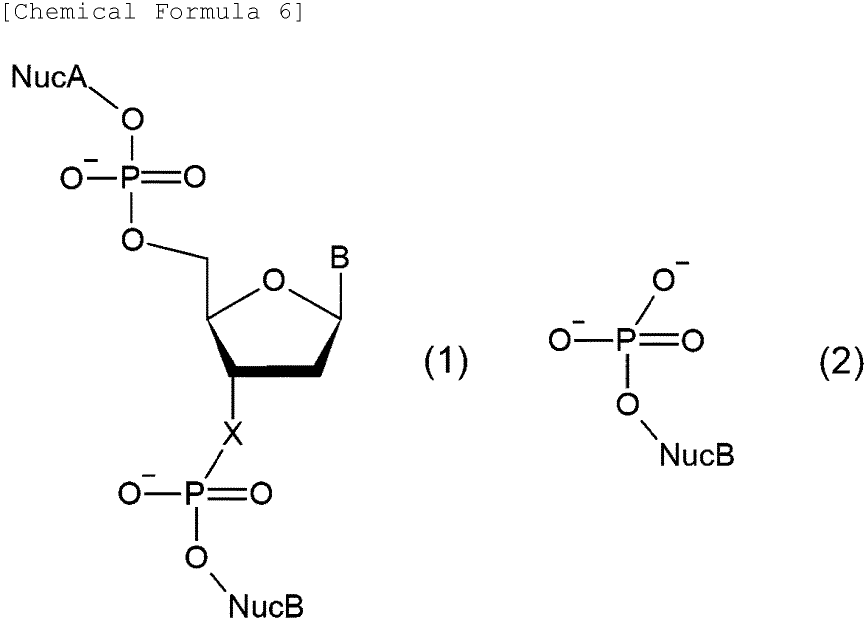 (Here, B represents a base, and X represents sulfur or selenium. NucA is composed of at least one nucleotide and is a part of the nucleic acid to be cleaved, and represents a part on the 5' end side with reference to the X. NucB is composed of at least one nucleotide and is a part of the nucleic acid to be cleaved, and represents a part on the 3' end side with reference to the X.)
[14] The double-stranded DNA production device according to [13], in which the double-stranded DNA to be cleaved has a plurality of structures represented by the Formula (1), and the number of nucleotides between the structures is 10 or less.

### Advantageous Effects of Invention

According to the present invention, it is possible a nucleic acid strand cleaving method and a nucleic acid strand cleaving device which have a high cleavage efficiency and a high recovery rate of a target nucleic acid. In addition, according to the present invention, it is possible to provide a double-stranded DNA production method and a double-stranded DNA production device which have a high sticky end formation efficiency and a high recovery rate of a double-stranded DNA having a target sticky end.

### Brief Description of Drawings

Fig. 1 is a schematic view showing an outline of a nucleic acid strand cleavage reaction using metal nanoparticles and a reaction for preparing double-stranded DNA having a sticky end and ligating the double-stranded DNA.
Fig. 2 is a diagram showing results of synthesis of an oligonucleotide having a 3'-thiophosphate bond using a thiated amidite reagent and analysis by reverse phase HPLC and MALDI-TOF-MS.
Fig. 3 is a view showing an examination of a DNA strand cleavage reaction by silver nanoparticle treatment and the silver nanoparticle size and reaction time/reaction temperature dependency in the result.
Fig. 4 is a view showing a result of improving DNA strand cleavage activity by polymer modification of a silver nanoparticle surface.
Fig. 5 is a diagram showing the results of preparing 3' overhang sticky ends by silver nanoparticle treatment of double-stranded short-strand DNA.
Fig. 6 is a diagram showing the results of PCR study when a thiated oligonucleic acid is introduced into a DNA template.
Fig. 7 is a schematic diagram of DNA amplification by PCR and preparation of a sticky fragment by BsaI or silver nanoparticle treatment.
Fig. 8 is a diagram showing the results of an experiment of linking sticky fragments using T4 DNA ligase.
Fig. 9 is a view showing a result of cleavage of a thiated oligonucleic acid strand by silver nitrate treatment as an existing technique.
Fig. 10 is a view showing a molecular weight analysis result by MALDI-TOF-MS of a cleaved product in the cleavage of a thiated oligonucleic acid strand by silver nitrate treatment as an existing technique.
Fig. 11 is a diagram showing the results of examination of cleavage of a thiated oligonucleotide strand by silver nitrate treatment and removal of silver ions by addition of thiols as existing techniques.
Fig. 12 is a view showing removal (nanoparticle diameter dependency) of silver nanoparticles by centrifugation.
Fig. 13 is a comparison between silver nitrate treatment and silver nanoparticle treatment in cleavage of a thiated oligonucleic acid strand. In the gel image, Lane 1 is a diagram showing the result of electrophoresis of DNA (15 mer) 5'-CACATTAATTGCGTT-FAM-3' having the same base length as the DNA generated after cleavage.
Fig. 14 is a view showing that dispersibility is improved by surface PEG modification of silver nanoparticles.
Fig. 15 is a view showing that dispersibility of silver nanoparticles is improved by surface PEG modification.
Fig. 16 is a view showing a sequence design of a DNA linking reaction using sticky end generation by DNA strand cleavage by silver nanoparticles.
Fig. 17 is a diagram showing the results of examination of coupling condition optimization in dimer model synthesis aiming at improvement of reaction conditions of 3'-thioated amidite.
Fig. 18 is an experimental result showing the influence of reaction time when 0.25 M BTFTH (13 equivalents) was used in dimer model synthesis.
Fig. 19 is a view showing an estimation mechanism of a side reaction in a coupling reaction revealed in dimer model synthesis.
Fig. 20 shows the results of applying the reaction conditions optimized in dimer synthesis to oligo DNA synthesis and improving the conditions.
Fig. 21 is a diagram showing synthesis results of oligo DNAs (sequence: 5'-TAA Ts CATTAATTGCGTT-FAM-3') synthesized and studied by adopting optimized coupling conditions.
Fig. 22 is a view showing the synthesis result of 51 base-long oligo DNA (5'-ATGAAACGCCGAGTTsAACGCCATCAAAAATsAATTCGCGTCTGGCCTTCCTsG-3') into which 3'thiophosphate bonds are introduced at three locations.
Fig. 23 is a view showing a sequence of a PCR primer DNA into which 3'thiophosphate bonds are introduced at four locations and two locations and a synthesis result thereof.
Fig. 24 is a diagram showing the synthesis results of 3'thiophosphorylated oligo-DNA (5'-TAACAs CACATTAATTGCGTT-FAM-3') synthesized using a 3'-thiolated amidite adenosine derivative.
Fig. 25 is a view showing a result of studying preparation of a 3'-overhang sticky end by cleavage of 3'-thiophosphate binding DNA containing an adenosine analog by silver nanoparticles.
Fig. 26 is a view showing the experimental design and sequence of sticky end preparation and linking when a primer into which a plurality of modifications has been introduced is used.
Fig. 27 is a schematic view showing sticky end preparation using a plurality of modified introduction primers and a linking reaction, and a view showing agarose electrophoresis results of PCR products.
Fig. 28 shows a result of comparing the linking efficiency with the linking between 34 base-long sticky end DNAs obtained by cleaving PCR amplification products at a plurality of locations.

### Description of Embodiments

### 1. Nucleic acid strand cleavage method

Hereinafter, a nucleic acid strand cleaving method of the present invention will be described. The nucleic acid strand cleaving method of the present invention includes a nucleic acid preparation step of preparing a nucleic acid to be cleaved having a structure represented by the following Formula (1), and a cleaving step of reacting the nucleic acid to be cleaved with a cleaving agent to cleave the nucleic acid to be cleaved at the part X of the Formula (1) to generate a nucleic acid having a structure represented by the following Formula (2). 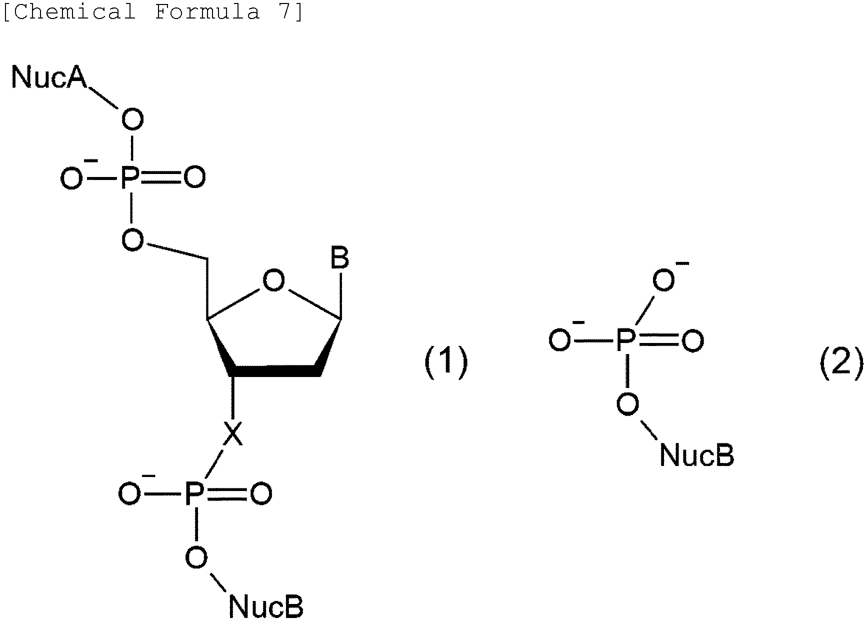 (Here, B represents a base, and X represents sulfur or selenium. NucA is composed of at least one nucleotide and is a part of the nucleic acid to be cleaved, and represents a part on the 5' end side with reference to the X. NucB is composed of at least one nucleotide and is a part of the nucleic acid to be cleaved, and represents a part on the 3' end side with reference to the X.)

Here, specific examples of the base B include adenine, guanine, cytosine, thymine, uracil, N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, and 5,6-dihydrouracil.

The cleaving agent is metal nanoparticles containing an atom selected from the group consisting of silver, mercury, and cadmium. As the cleaving agent, silver is particularly preferable from the viewpoint of low toxicity and low cost. When the cleaving agent is silver nanoparticles, X in Formula (1) of the nucleic acid to be cleaved is preferably sulfur.

The average particle size of the metal nanoparticles is preferably 100 nm or less, more preferably 50 nm or less, and particularly preferably 20 nm or less from the viewpoint of high cleavage efficiency or the like for the nucleic acid to be cleaved. The lower limit of the average particle size of the metal nanoparticles is not particularly limited, but is, for example, 0.1 nm or more, preferably 0.5 nm or more, and more preferably 1 nm or more.

From the viewpoint of improving the cleavage activity for the nucleic acid to be cleaved, the metal nanoparticles are preferably surface-treated with polyethylene glycol to bind polyethylene glycol to the surface. By binding polyethylene glycol to the surface of the metal nanoparticles, oxidation of the surface of the metal nanoparticles is prevented, and dispersibility of the metal nanoparticles in an aqueous solution is improved. Therefore, as compared with the metal nanoparticles that are not surface-treated with polyethylene glycol, the metal nanoparticles surface-treated with polyethylene glycol have improved cleavage activity for the nucleic acid to be cleaved. Polyethylene glycol can be bound to the metal nanoparticles using a thiol-modified end. The average molecular weight of polyethylene glycol is preferably within the range of 1,000 to 10,000.

### (1) Nucleic acid preparation step

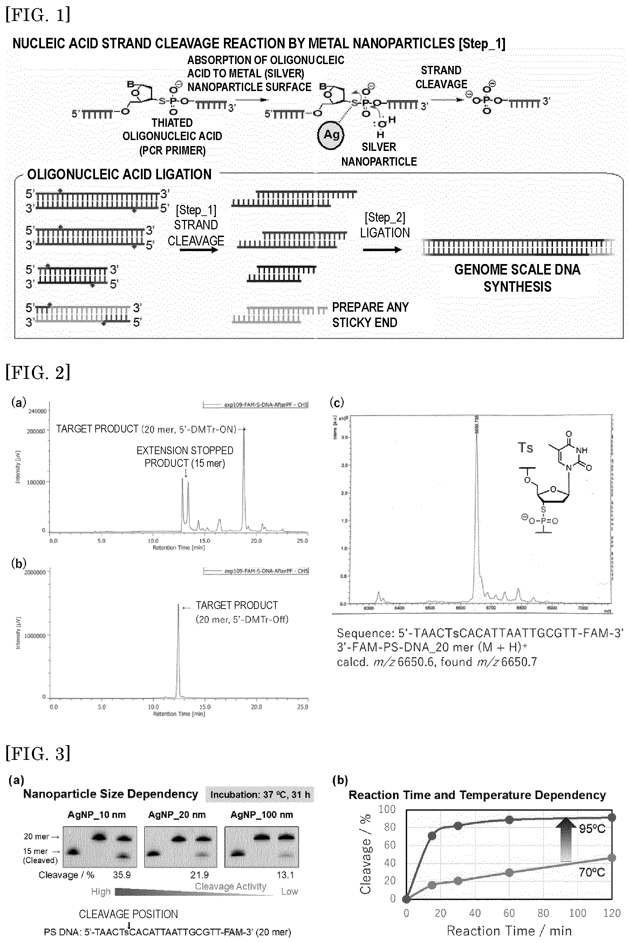

Next, each step of the present invention will be described with reference to Fig. 1. The upper part of Fig. 1 ("Nucleic acid strand cleavage reaction by metal nanoparticles [Step_1]") schematically shows the nucleic acid strand cleaving method of the present invention. In Fig. 1, a case where X in Formula (1) is sulfur, the cleaving agent is silver nanoparticles, and a nucleic acid to be cleaved (thiated oligonucleic acid) is used as a PCR primer is described, but the present invention is not limited thereto. For example, when X is selenium or when the cleaving agent is mercury or cadmium, the nucleic acid to be cleaved can be cleaved by the same mechanism.

First, the nucleic acid preparation step will be described. The nucleic acid preparation step is a step of preparing a nucleic acid to be cleaved having the structure represented by Formula (1). In this step, a part or all of the nucleic acid to be cleaved can be synthesized by a phosphoramidite method using amidite reagents represented by the following Formula (3) and Formula (4). 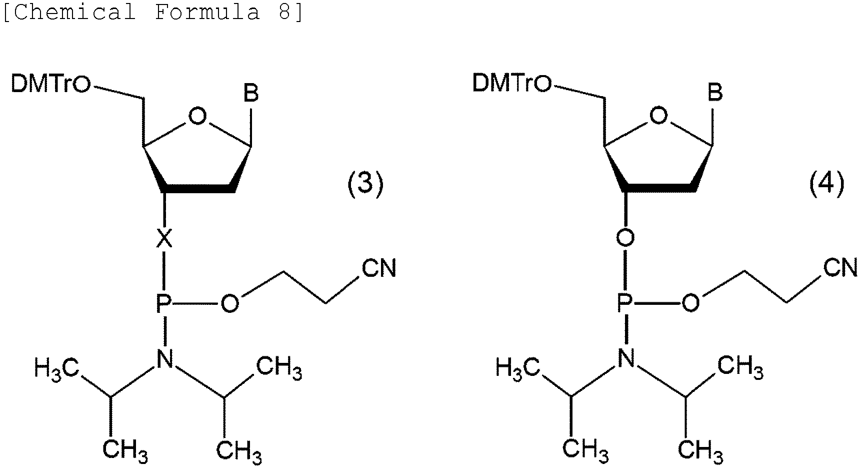 (Here, B represents a base, X represents sulfur or selenium, and DMTr represents a dimethoxytrityl group.)

The amidite reagent represented by Formula (3) (thiated amidite reagent or selenated amidite reagent) can be synthesized by the following method. First, a nucleoside derivative in which an amino group of a base is protected by an amide bond and 5' carbon is protected with a dimethoxytrityl group (DMTr group) or a tertbutyldimethylsilyl group (TBDMS group) is prepared. Next, triphenylphosphine (PPh₃), diisopropyl azodicarboxylate (DIAD), or the like is reacted with the nucleoside derivative to stereoscopically invert the 3' hydroxyl group, and thiobenzoic acid or selenobenzoic acid is reacted with the 3' hydroxyl group. Subsequently, by alkali hydrolysis of benzoic acid, nucleosides in which the oxygen of the 3' hydroxyl group of the nucleoside derivative is substituted with sulfur or selenium are obtained. Finally, amidites such as 2-cyanoethyl-N,N-diisopropyl chlorophosphoramidite are reacted to obtain a target amidite reagent.

The nucleic acid to be cleaved can be synthesized using a known automatic nucleic acid synthesizing device using a thiated or selenated amidite reagent represented by Formula (3) and a normal (that is, thiation or selenation is not performed) amidite reagent represented by Formula (4). By synthesizing a nucleic acid of a desired sequence with the amidite reagent represented by Formula (4) and using the amidite reagent represented by Formula (3) at a desired position, a nucleic acid to be cleaved in which a thiated or selenated nucleotide is disposed at a desired position can be synthesized.

When the nucleic acid to be cleaved is synthesized, it is preferable to add an activator. Examples of the activator include 1H-tetrazole (1H-tet), 5-benzylthio-1H-tetrazole (BTT), 4,5-dicyanoimidazole (DCI), and 5-[3,5-bis(trifluoromethyl)phenyl]-1H-tetrazole (BTFTH). Among them, BTFTH is most preferable from the viewpoint of reducing the generation amount of by-products (dithiolate forms) and improving the yield of the target nucleic acid to be cleaved. Furthermore, when BTFTH is used as an activator, the coupling time is preferably within the range of 60 to 120 seconds, and it is preferable to perform the coupling reaction a plurality of times (for example, 2 to 7 times) at this coupling time from the viewpoint of improving the yield.

The entire length of the nucleic acid to be cleaved can be synthesized using the above-described automatic nucleic acid synthesizing device, but only a part of the nucleic acid to be cleaved is synthesized by the automatic nucleic acid synthesizing device, and the entire length of the nucleic acid to be cleaved can be synthesized by performing a plurality of cycles of polymerase chain reaction (PCR) using a template DNA having a sequence complementary to the nucleic acid to be cleaved as a primer. That is, a nucleic acid to be cleaved is generated by extending a primer along the template DNA by PCR. The number of nucleotides constituting the primer can be appropriately set according to the purpose, and can be, for example, 5 mer or more and 50 mer or less.

As described later, a vector or the like can be constructed by forming a sticky end in the nucleic acid to be cleaved of the present invention and ligating the nucleic acid to be cleaved to a DNA strand having another sticky end. When the length of the single-stranded DNA on the protruding side of the sticky end is long, for example, 20 bases or more, it is preferable to introduce the structure represented by the above Formula (1) at a plurality of locations on the complementary strand side. In this case, as shown in Examples described later, from the viewpoint of ligation efficiency, the number of nucleotides nt (number of bases: b) between the structures of Formula (1) is preferably 15 or less, and more preferably 10 or less. When the number of nucleotides is 15 or less, dissociation of the DNA fragment after the strand cleavage by the metal nanoparticles is accelerated, and the ligation efficiency tends to be high.

### (2) Cleaving step

Next, the cleaving step will be described. In the cleaving step, the nucleic acid to be cleaved is reacted with a cleaving agent to cleave the nucleic acid to be cleaved at the part X in Formula (1) to generate a nucleic acid having a structure represented by the following Formula (2). In Fig. 1, a nucleic acid to be cleaved (thiolated oligonucleic acid) is cleaved using silver nanoparticles, and after the cleavage, the oligonucleic acid on the 5' end side of the part X is excised, and the cleavage site becomes a phosphate group.

The cleaving agent is preferably used in the state of a dispersion in which metal nanoparticles are dispersed. The cleavage reaction is preferably performed at 70°C or higher, more preferably 90°C or higher, and particularly preferably 95°C or higher from the viewpoint of high cleavage activity. The upper limit of the temperature of the cleavage reaction is not particularly limited, and is preferably 100°C or lower. In addition, the reaction time of the cleavage reaction can be appropriately set according to the reaction temperature, and the reaction time can be set shorter as the reaction temperature is higher. The reaction time is preferably in the range of 5 minutes or more and 120 minutes or less.

### 2. Nucleic acid strand cleaving device

Next, the nucleic acid strand cleaving device will be described. The nucleic acid strand cleaving device of the present invention is a device for carrying out the above-described nucleic acid strand cleaving method. The nucleic acid strand cleaving device includes: nucleic acid preparation means that prepares a nucleic acid to be cleaved having a structure represented by the following Formula (1); and cleaving means that reacts the nucleic acid to be cleaved with a cleaving agent to cleave the nucleic acid to be cleaved at the part X of the Formula (1) to generate a nucleic acid having a structure represented by the following Formula (2).

Examples of the nucleic acid preparation means include amidite reagents represented by the following Formula (3) and Formula (4), an automatic nucleic acid synthesizing device, and various reagents used for nucleic acid synthesis. In addition, when a nucleic acid synthesized by an automatic nucleic acid synthesizing device is used as a primer, a template DNA, a PCR device, a reagent used for PCR, and the like are also included in the nucleic acid preparation means.

Examples of the cleaving means include the nucleic acid to be cleaved synthesized by the nucleic acid preparation means, the above-described cleaving agent, various devices (incubator and the like) for performing a cleavage reaction of the nucleic acid to be cleaved by the cleaving agent, and various reagents used for the reaction.

### 3. Method for producing double-stranded DNA having sticky end

Next, a double-stranded DNA production method having a sticky end will be described. In the production method of the present invention, a sticky end is formed on one end side or both end sides of double-stranded DNA. First, a double-stranded DNA preparation step is performed. In this step, a double-stranded DNA to be cleaved including a sense strand and an antisense strand is prepared. The antisense strand has a sequence complementary to the sense strand. At least one of the sense strand and the antisense strand has a structure represented by Formula (1). In the lower part of Fig. 1 (a part surrounded by a square), a case where both the sense strand and the antisense strand have the structure of Formula (1) is shown. As described above, by introducing the structure of Formula (1) into both strands, DNA with sticky ends at both ends can be generated in the sticky end generation step to be described later.

Next, a sticky end generation step is performed ("[Step_1] Strand cleavage" in Fig. 1). In this step, the double-stranded DNA to be cleaved is reacted with the cleaving agent to cleave the sense strand and/or the antisense strand at the part X in Formula (1). As a result, double-stranded DNA having the structure represented by Formula (2) and having a sticky end is generated. The cleaving conditions and the like are as described in the above "(2) Cleaving step".

### 3. Device for producing double-stranded DNA having sticky end

Next, a device for producing double-stranded DNA having a sticky end will be described. The device of the present invention is a device for carrying out the above-described double-stranded DNA production method having a sticky end. Specifically, double-stranded DNA preparation means for preparing double-stranded DNA to be cleaved, and sticky end generation means for reacting the double-stranded DNA to be cleaved with a cleaving agent to generate double-stranded DNA having a structure represented by Formula (2) and having a sticky end are included.

Examples of the double-stranded DNA preparing means include the amidite reagent represented by Formula (3) and Formula (4), the automatic nucleic acid synthesizing device, the template DNA, the PCR device, and various reagents, which are described in the above "nucleic acid preparation means".

Examples of the sticky end generation means include the nucleic acid to be cleaved synthesized by the nucleic acid preparation means, the cleaving agent, various devices such as an incubator, various reagents, and the like, which are described in the above "cleaving means".

In the present invention, since metal nanoparticles are used as a cleaving agent without using a metal ion conventionally used, the recovery rate of DNA as a cleaved product is further increased while retaining a high cleaving activity equivalent to that of a metal ion, and a sticky end can be efficiently formed. Furthermore, DNAs having such sticky ends can be freely linked to each other. For example, a sticky end common to both the target DNA and the vector is formed with a cleaving agent, and these are ligated to prepare a recombinant DNA, which can be used for cloning, library creation, mass expression system construction, and the like. Further, by linking a plurality of genome sequences having sticky ends, genome build-up reaction can be performed in vitro (refer to "Step_2" at the lower part of Fig. 1). Alternatively, by introducing double-stranded DNA in a blunt end state into a cell and performing deprotection treatment in the cell, genome build-up reaction can also be performed in the cell.

### Examples

The present invention will now be specifically described with reference to Examples, but these will not limit the object of the present invention. In Examples below, notation of "%" is on the mass basis (mass percent) unless otherwise specifically noted.

### 1. Improved synthesis of thiated amidite reagent and introduction into oligonucleic acid

### (1) Synthesis of thiated amidite reagent (thymidine derivative: T, cytidine derivative: C)

The synthesis scheme of the thiated amidite reagent (thymidine derivative: T, cytidine derivative: C) is shown below.

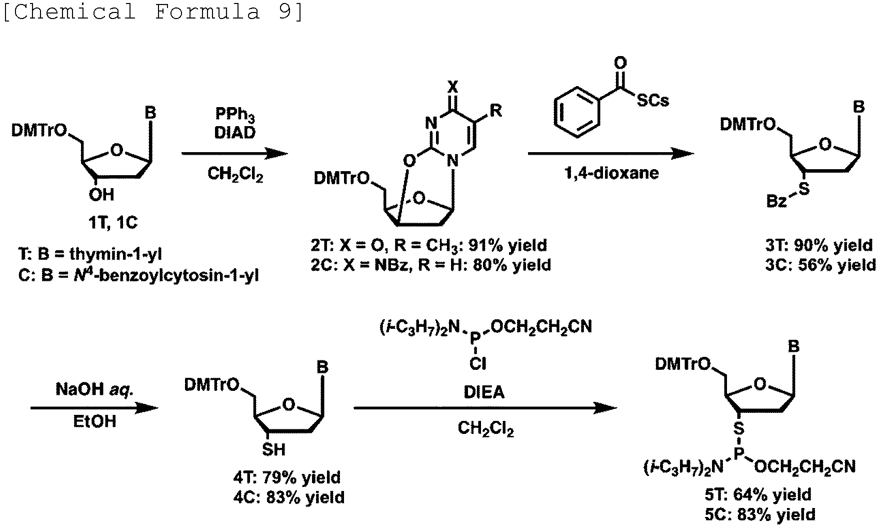

### (a) Compound 2T

Triphenylphosphine (0.72 g, 2.8 mmol) was added to 5'-O-(4,4'-dimethoxytrityl)thymidine(Compound 1T) (1.0 g, 1.8 mmol), and then dissolved in dichloromethane (6.0 mL). In an ice bath, diisopropyl azodicarboxylate (0.54 mL, 2.8 mmol) was slowly added dropwise thereto. After stirring at room temperature for 3 hours, the reaction solution was concentrated with a rotary evaporator. Purification was performed by column chromatography (neutral flash silica, developing solvent: methanol/ethyl acetate mixed solvent containing 1% triethylamine = 0/1 → 1/4), and 0.89 g (1.7 mmol) of Compound 2T was obtained as a white solid (yield: 94%).

¹H NMR(400 MHz, DMSO-d₆) δ 7.59(d, J = 1.3 Hz, 1H), 7.37 - 7.30(m, 2H), 7.30 - 7.11(m, 7H), 6.85 - 6.76(m, 4H), 5.85(d, J = 3.7 Hz, 1H), 5.27(q, J = 2.2 Hz, 1H), 4.37(ddd, J = 7.8, 4.8, 2.5 Hz, 1H), 3.68(d, J = 2.5 Hz, 6H), 3.13 - 2.98(m, 2H), 2.54(dd, J = 12.8, 1.5 Hz, 1H), 2.44 - 2.37(m, 1H), 1.73(d, J = 1.1 Hz, 3H) ppm. The spectral data showed good agreement with literature values.

### (b) Compound 3T

Compound 2T (1.0 g, 1.9 mmol) and cesium thiobenzoate (1.8 g, 7.0 mmol) were suspended in 1,4 dioxane (40 mL) and stirred at 120°C overnight. Further, cesium thiobenzoate (1.8 g, 7.0 mmol) was added thereto, and the mixture was stirred at 130°C overnight. The reaction solution was cooled to room temperature, then diluted with ethyl acetate, and washed twice with a saturated aqueous sodium bicarbonate solution. The organic phase was dried over anhydrous sodium sulfate and then concentrated by a rotary evaporator. Purification was performed by column chromatography (neutral flash silica, developing solvent: methanol/dichloromethane mixed solvent containing 1% triethylamine = 0/1 → 1:60), and 1.1 g (1.7 mmol) of Compound 3T was obtained as a brown solid (yield: 90%).

¹H NMR(600 MHz, CDCl₃)δ 7.93 - 7.86(m, 2H), 7.71(d, J = 1.4 Hz, 1H), 7.61 - 7.56(m, 1H), 7.47 - 7.43(m, 4H), 7.34 - 7.31(m, 4H), 7.26(dd, J = 8.4, 7.0 Hz, 2H), 7.22 - 7.17 (m, 1H), 6.82 - 6.77(m, 4H), 6.30(dd, J = 6.6, 5.2 Hz, 1H), 4.49(q, J = 7.4 Hz, 1H), 4.14(dt, J = 7.2, 2.7 Hz, 1H), 3.73(d, J = 4.4 Hz, 6H), 3.52(dd, J = 10.8, 2.4 Hz, 1H), 3.45(dd, J = 10.8, 3.1 Hz, 1H), 2.75(ddd, J = 13.8, 8.3, 5.2 Hz, 1H), 2.53 - 2.48(m, 1H), 1.48(d, J = 1.3 Hz, 3H) ppm. The spectral data showed good agreement with literature values.

### (c) Compound 4T

Compound 3T (840 mg, 1.26 mmol) was dissolved in a mixed solution of ethanol (30 mL)-10 M aqueous sodium hydroxide solution (4 mL) that had been subjected to a deoxygenation treatment by argon bubbling for 30 minutes, and then the solution was stirred at room temperature for 2 hours under argon bubbling. The reaction solution was cooled on an ice bath and the reaction was stopped by slow dropwise addition of 1 M aqueous hydrochloric acid (40 mL). The generated precipitate was recovered by suction filtration and washed three times with water on the filter paper. The obtained solid was dissolved in dichloromethane and then dried over anhydrous sodium sulfate, and then the solvent was distilled off with a rotary evaporator. Purification was performed by column chromatography (neutral flash silica, developing solvent: hexane/ethyl acetate mixed solvent containing 1% triethylamine = 1/1 → 1/3), and 560 mg (0.995 mmol) of Compound 5T was obtained as a brown solid (yield: 79%).

¹H NMR (600 MHz, CDCl₃) δ 7.68 (d, J = 1.4 Hz, 1H), 7.41(dd, J = 7.5, 1.7 Hz, 2H), 7.34 - 7.20(m, 7H), 6.83(dd, J = 8.8, 2.0 Hz, 4H), 6.14(dd, J = 7.1, 3.0 Hz, 1H), 4.10(q, J = 7.1 Hz, 1H), 3.85(dt, J = 8.8, 2.7 Hz, 1H), 3.77(s, 6H), 3.63 - 3.56(m, 2H), 3.39(dd, J = 11.0, 2.9 Hz, 1H), 2.59(ddd, J = 13.9, 7.6, 2.9 Hz, 1H), 2.35(ddd, J = 13.9, 10.2, 7.1 Hz, 1H), 1.55(d, J = 6.9 Hz, 1H), 1.48(d, J = 1.2 Hz, 3H) ppm. The spectral data showed good agreement with literature values.

### (d) Compound 5T

Compound 4T (360 mg, 0.640 mmol) was dissolved in dichloromethane (5.00 ml), and then N,N-diisopropylethylamine (331 µL, 1.92 mmol) was added. Subsequently, 2-cyanoethyl-N,N-diisopropylchlorophosphoramidite (185 µL, 0.830 mmol) was added thereto, and then the mixture was stirred at room temperature for 2 hours. The reaction solution was diluted with dichloromethane and washed with saturated aqueous sodium bicarbonate solution. The organic phase was dried with anhydrous sodium sulfate, and then the solvent was distilled off with a rotary evaporator. Purification was performed by column chromatography (neutral flash silica, developing solvent: hexane/ethyl acetate mixed solvent containing 1% triethylamine = 2/1 → 1/1.5), and 312 mg (0.410 mmol) of Compound 5T was obtained as a white solid (yield: 64%).

¹H NMR(400 MHz, CD₃CN)δ 9.41 (s, 1H), 7.55-7.40(m,3H),7.38-7.24(m, 6H),7.24-7.14(m,1H),6.83(dd,J=8.7,5.5 Hz,4H),6.18-5.99(m,1H),4.10-3.93(m,2H),3.86-3.75(m,1H),3.73(s, 6H),3.71-3.44(m,4H),3.44-3.28(m,1H),2.66-2.53(m,2H),2.53-2.39(m,1H),1.95(s,1H),1.55-1.42(m,3H),1.31-1.05(m,9H),1.03(s,1H),1.01(s,1H) ppm.

³¹P NMR(162 MHz, CD₃CN)δ 161.96, 158.44ppm. The spectrum data showed good agreement with literature values.

### (e) Compound 2C

N4-benzoyl-5'-O-(4,4'-dimethoxytrityl)-2'-deoxycytidine (Compound 1C) (1.00 g, 1.57 mmol) was azeotroped with toluene (20.0 mL) three times. Triphenylphosphine (0.456 g, 1.74 mmol) was added thereto, and then the mixture was dissolved in dichloromethane (6.00 mL). In an ice bath, diisopropyl azodicarboxylate (0.456 mL, 1.74 mmol) was slowly added dropwise thereto. Thereafter, the mixture was stirred at room temperature for 16 hours. The reaction solution was concentrated by a rotary evaporator, purification was performed by column chromatography (neutral flash silica, developing solvent: methanol/ethyl acetate mixed solvent containing 1% triethylamine = 0/1 → 1/4), and 0.780 g (1.26 mmol) of Compound 2C was obtained as a white solid (yield: 78%).

¹H-NMR(600 MHz, DMSO-d₆) δ 7.81(d, J = 7.1 Hz, 2H), 7.69(d, J = 7.5 Hz, 1H), 7.51(t, J = 7.3 Hz, 1H), 7.18 - 7.39(m, 12H), 6.84(dd, J = 9.0, 3.6 Hz, 4H), 6.42(d, J = 7.5 Hz, 1H), 5.97(d, J = 4.1 Hz, 1H), 5.33(s, 1H), 4.40(s, 1H), 3.70(d, J = 2.4 Hz, 7H), 3.04 - 3.13 (m, 2H), 2.63(d, J = 11.9 Hz, 1H) ppm. The spectrum data showed good agreement with literature values.

### (f) Compound 3C

Compound 2C (2.8 g, 4.8 mmol) and cesium thiobenzoate (6.0 g, 24 mmol) were dissolved in 1,4-dioxane (60 mL), and the mixture was stirred at room temperature for 5 hours. Thereafter, the reaction solution was diluted with ethyl acetate (100 mL), and washed twice with a saturated aqueous sodium bicarbonate solution (100 mL) and twice with saturated saline. The organic phase was dried with anhydrous sodium sulfate, and then the solvent was distilled off with a rotary evaporator. Purification was performed by column chromatography (neutral flash silica, developing solvent: ethyl acetate/hexane = 1/1 → 7/1), and 2.0 g (2.7 mmol) of Compound 3C was obtained as a brown solid (yield: 56%) .

¹H-NMR(600 MHz, DMSO-d₆)δ 11.29(s, 1H), 8.55(d, J = 7.5 Hz, 1H), 8.00(d, J = 7.5 Hz, 2H), 7.88(d, J = 7.1 Hz, 2H), 7.50 - 7.73(m, 4H), 7.38(d, J = 7.5 Hz, 2H), 7.14 - 7.27(m, 9H), 6.82(q, J = 4.5 Hz, 5H), 6.12(d, J = 9.2 Hz, 1H), 4.40(t, J = 9.0 Hz, 1H), 4.23(s, 1H), 3.69(d, J = 5.4 Hz, 1H), 3.66(s, 6H), 2.65(s, 2H), 2.36(s, 1H) ppm. The spectrum data showed good agreement with literature values.

### (g) Compound 4C, Compound 5C

Compound 3C (1.64 g, 2.2 mmol) was dissolved in a methanol (15 mL)-THF (21 mL)-0.5 M aqueous sodium hydroxide solution (22 mL, 11 mmol) mixed solution that had been subjected to a deoxygenation treatment by argon bubbling for 30 minutes, and then the solution was stirred at -10°C for 30 minutes under argon bubbling. To the reaction solution, a 1 M aqueous potassium dihydrogen phosphate solution (46.6 mL, 46.6 mmol) was slowly added dropwise to stop the reaction. After dilution with ethyl acetate (100 mL), it was washed twice with water (100 mL). The organic phase was dried with anhydrous sodium sulfate, and then the solvent was distilled off with a rotary evaporator. Purification was performed by column chromatography (neutral flash silica, developing solvent: methanol/dichloromethane mixed solvent containing 1% triethylamine = 1/50 → 1/10), and then 1.18 g of Compound 4C was obtained as a white solid. 0.94 g (1.43 mmol) of the obtained Compound 4C was weighed and dissolved in dichloromethane (15.2 mL), and then N,N-diisopropylethylamine (0.35 mL, 1.57 mmol) and 2-cyanoethyl-N,N-diisopropylchlorophosphoramidite (0.35 mL, 1.57 mmol) were added, and the mixture was stirred at room temperature for 2 hours. The reaction solution was diluted with dichloromethane (20 mL) and washed with saturated aqueous sodium bicarbonate solution (20 mL). The organic phase was dried with anhydrous sodium sulfate, and then the solvent was distilled off with a rotary evaporator. Purification was performed by column chromatography (neutral flash silica, developing solvent: acetonitrile/dichloromethane mixed solvent containing 1% triethylamine = 3/7), and then 1.0 g (1.2 mmol) of Compound 5C was obtained as a white solid (2-step total yield: 68%).

¹H-NMR(400 MHz, CDCl₃)δ 9.19(s, 1H), 8.78(s, 1H), 8.30(d, J = 19.5 Hz, 1H), 8.02 - 8.05(m, 2H), 7.51 - 7.63(m, 3H), 7.37 - 7.42(m, 2H), 7.27 - 7.31(m, 5H), 7.18 - 7.25(m, 3H), 6.77 - 6.81(m, 4H), 6.42(dd, J = 7.0, 2.5 Hz, 1H), 4.21 - 4.25(m, 1H), 3.90(t, J = 6.2 Hz, 1H), 3.79 - 3.85(m, 1H), 3.77(d, J = 4.3 Hz, 6H), 3.57 - 3.67(m, 5H), 3.41 - 3.45(m, 1H), 3.07 - 3.14(m, 1H), 2.60(t, J = 6.2 Hz, 1H), 2.44(t, J = 6.3 Hz, 2H), 1.18 - 1.22(m, 6H), 1.13 - 1.17(m, 6H) ppm.

³¹P-NMR(162 MHz, CDCl₃)δ 165.57, 161.68 ppm. The spectrum data showed good agreement with literature values.

### (2) Synthesis of thiated amidite reagent (adenosine derivative: A)

The synthesis scheme of the thiated amidite reagent (adenosine derivative: A) is shown below.

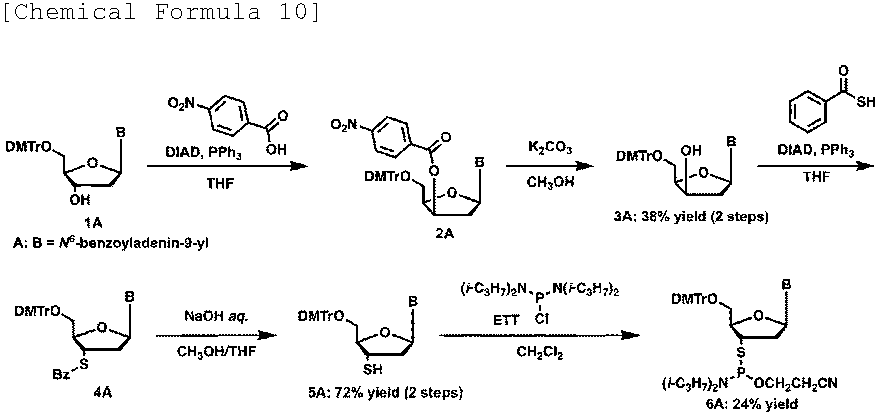

### (a) Compound 2A, Compound 3A

N6-benzoyl-5'-O-(4,4'-dimethoxytrityl)-2'-deoxyadenosine (Compound 1A) (10.0 g, 15.2 mmol), 4-nitrobenzoic acid (5.08 g, 30.4 mmol), and triphenylphosphine (16.0 g, 61.0 mmol) were dissolved in THF (800 mL) and diisopropyl azodicarboxylate (11.9 mL, 61.0 mmol) was slowly added dropwise with stirring at 0°C. After stirring at 0°C for 2 hours, the reaction solution was concentrated by a rotary evaporator, purification was performed by column chromatography (neutral flash silica, developing solvent: ethyl acetate/hexane = 7/3), and 18.5 g of Compound 2A was obtained as a white solid. 10.0 g (12.6 mmol) of the obtained Compound 2A was weighed, and potassium carbonate (1.71 g, 49.6 mmol) was added thereto, then the mixture was dissolved in methanol (125 mL). After stirring in an ice bath for 2 hours, the reaction solution was concentrated. The residue was dissolved in ethyl acetate (75 mL) and washed twice with water (75 mL). The organic phase was dried with anhydrous sodium sulfate, and then the solvent was distilled off with a rotary evaporator. Purification was performed by column chromatography (neutral flash silica, developing solvent: ethyl acetate/hexane = 1/1), and 1.40 g (2.10 mmol) of Compound 3A was obtained as a white solid (2-step total yield: 38%) .

¹H-NMR(400 MHz, DMSO-d₆) δ 11.18(s, 1H), 8.75(s, 1H), 8.47(s, 1H), 8.03(d, J = 7.6 Hz, 2H), 7.53 - 7.66(m, 3H), 7.17 - 7.39(m, 9H), 6.75 - 6.83(m, 4H), 6.50(d, J = 7.4 Hz, 1H), 5.48(d, J = 4.3 Hz, 1H), 4.31(d, J = 44.2 Hz, 2H), 3.66 - 3.74(m, 6H), 3.37(d, J = 8.1 Hz, 1H), 3.20(d, J = 10.3 Hz, 1H), 2.78 (s, 1H), 2.38(d, J = 14.6 Hz, 1H) ppm. The spectrum data showed good agreement with literature values.

### (b) Compound 4A, Compound 5A, Compound 6A

Triphenylphosphine (2.4 g, 9.0 mmol) was dissolved in THF (53 mL), and diisopropyl azodicarboxylate (1.75 mL, 9.0 mmol) was slowly added dropwise while stirring in an ice bath. The mixture was stirred in an ice bath for 30 minutes, then thiobenzoic acid (1.08 mL, 9.0 mmol) was added thereto, and the mixture was further stirred in an ice bath for 30 minutes. Subsequently, Compound 3A (2.0 g, 3.0 mmol) was added, and the mixture was stirred in an ice bath for 16 hours. The reaction solution was concentrated by a rotary evaporator, the residue is purified by column chromatography (neutral flash silica, developing solvent: ethyl acetate/hexane = 1/1 → 2/1), and 2.3 g of Compound 4A was obtained as a white solid. 2.3 g (3.0 mmol) of the obtained Compound 4A was weighed and dissolved in a methanol (42 mL)-THF (28 mL)-0.5 M aqueous sodium hydroxide solution (18 mL, 9.0 mmol) mixed solution subjected to a deoxygenation treatment by argon bubbling for 30 minutes, and then the solution was stirred at -10°C for 30 minutes under argon bubbling. To the reaction solution, a 1 M aqueous potassium dihydrogen phosphate solution (38 mL, 19 mmol) was slowly added dropwise to stop the reaction. The generated precipitate was recovered by suction filtration and washed three times with water (200 mL) on the filter paper. The obtained solid was purified by column chromatography (neutral flash silica, developing solvent: methanol/dichloromethane mixed solvent containing 1% triethylamine = 1/50 → 1/20), and then 1.62 g of Compound 5A was obtained as a white solid. 1.5 g (2.3 mmol) of the obtained Compound 5A was weighed, and 5-(ethylthio)-1H-tetrazole (0.30 g, 2.3 mmol) was added, then the mixture was dissolved in dichloromethane (10 mL). Subsequently, 2-cyanoethyl N,N,N',N'-tetraisopropyl phosphorodiamidite (1.1 mL, 3.5 mmol) was added thereto, and the mixture was stirred at room temperature for 1 hour. The reaction solution was diluted with dichloromethane (50 mL), and then washed with a saturated aqueous sodium bicarbonate solution (50 mL). The organic phase was dried with anhydrous sodium sulfate, and then the solvent was distilled off with a rotary evaporator. Purification was performed by column chromatography (neutral flash silica, developing solvent: ethyl acetate/hexane = 2/1→8/1), and 0.46 g (0.54 mmol) of Compound 6A was obtained as a white solid (3-step total yield: 17%).

¹H-NMR(400 MHz, CDCl₃)δ 9.19(s, 1H), 8.78(s, 1H), 8.30(d, J = 19.5 Hz, 1H), 8.02 - 8.05(m, 2H), 7.51 - 7.63(m, 3H), 7.37 - 7.42(m, 2H), 7.27 - 7.31(m, 5H), 7.18 - 7.25(m, 3H), 6.77 - 6.81(m, 4H), 6.42(dd, J = 7.0, 2.5 Hz, 1H), 4.21 - 4.25(m, 1H), 3.90(t, J = 6.2 Hz, 1H), 3.79 - 3.85(m, 1H), 3.76 - 3.77(m, 7H), 3.57 - 3.67(m, 5H), 3.41 - 3.45(m, 1H), 3.07 - 3.14(m, 1H), 2.60(t, J = 6.2 Hz, 1H), 1.18 - 1.22(m, 6H), 1.13 - 1.17(m, 6H) ppm.

³¹P-NMR(162 MHz, CDCl₃)δ 165.22 ppm. The spectrum data showed good agreement with literature values.

### (3) Synthesis of thiated amidite reagent (guanosine derivative: G)

The synthesis scheme of the thiated amidite reagent (guanosine derivative: G) is shown below.

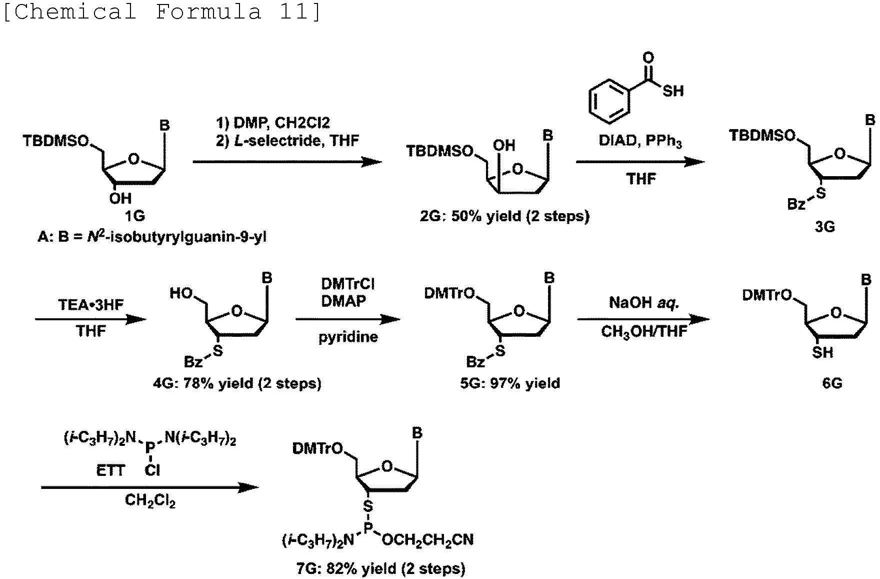

### (a) Compound 2G

Compound 1G (7.3 g, 16 mmol) was dissolved in dichloromethane (370 mL), and Dess-Martin periodinane (34 g, 80 mmol) and sodium hydrogen carbonate (21 g) were added. The mixture was stirred in an ice bath for 2 hours, followed by 3 hours at room temperature. After completion of the reaction, a saturated aqueous sodium bicarbonate solution (370 ml) and sodium thiosulfate (370 mL) were added, and the mixture was extracted twice with dichloromethane (730 mL). The organic phase was dried with anhydrous sodium sulfate, and then the solvent was distilled off with a rotary evaporator. The resulting 2'-keto form was dissolved in tetrahydrofuran (260 mL) and stirred at -78°C for 30 minutes. Subsequently, a 1M L-selectride/tetrahydrofuran solution (40 mL, 40 mmol) was added dropwise, and the mixture was stirred at -78°C for 16 hours. The reaction was ended by adding a saturated aqueous ammonium chloride solution (50 mL) to the reaction solution, and the mixture was extracted three times with ethyl acetate. The organic phase was dried with anhydrous sodium sulfate, and then the solvent was distilled off with a rotary evaporator. Purification was performed by column chromatography (neutral flash silica, developing solvent: methanol/dichloromethane = 1/15), and 3.6 g (8.0 mmol) of Compound 2G was obtained as a white solid (yield: 50%).

¹H-NMR(400 MHz, DMSO-d₆)δ 12.06(s, 1H), 11.70(s, 1H), 8.12 - 8.19(m, 1H), 6.12(dd, J = 8.2, 1.9 Hz, 1H), 5.35 - 5.39(m, 1H), 4.29 - 4.37(m, 1H), 3.92 - 4.11(m, 2H), 3.73 - 3.79(m, 1H), 2.65 - 2.81(m, 2H), 2.21 - 2.32(m, 1H), 1.08 - 1.15(m, 6H), 0.83 - 0.86(m, 9H), 0.16 - 0.14 (m, 6H) ppm. The spectrum data showed good agreement with literature values.

### (b) Compound 3G, Compound 4G

Triphenylphosphine (11 g, 40 mmol) was dissolved in tetrahydrofuran (140 mL), and diisopropyl azodicarboxylate (7.8 mL, 40 mmol) was slowly added dropwise thereto while stirring in an ice bath. The mixture was stirred in an ice bath for 30 minutes, thiobenzoic acid (4.7 mL, 40 mmol) was added thereto, and the mixture was further stirred for 30 minutes at 0°C. Then, Compound 2G (3.0 g, 6.7 mmol) was added, and the mixture was stirred in an ice bath for 3 hours and further stirred at room temperature for 16 hours. The reaction solution was concentrated by a rotary evaporator, then purification was performed by column chromatography (neutral flash silica, developing solvent: ethyl acetate/hexane = 1/1), and 25 g of Compound 3G was obtained as a white solid. The obtained compound 3G (25 g, 6.7 mol) was dissolved in tetrahydrofuran (100 mL), and then triethylamine hydrogen trifluoride (16 ml, 100 mmol) was added, and the mixture was stirred at room temperature for 16 hours. The reaction solution was concentrated by a rotary evaporator and then purified by column chromatography (neutral flash silica, developing solvent: methanol/dichloromethane = 1/15). Furthermore, recrystallization was performed with dichloromethane, and 2.4 g (5.2 mmol) of Compound 4G was obtained as a white solid (2 steps total yield 78%).

¹H-NMR(600 MHz, DMSO-d₆)δ 12.09(s, 1H), 11.69 (s, 1H), 8.34(d, J = 15.6 Hz, 1H), 7.93(dd, J = 8.3, 0.8 Hz, 2H), 7.67 - 7.74(m, 1H), 7.54 - 7.63(m, 2H), 6.24(t, J = 5.9 Hz, 1H), 5.20(t, J = 5.4 Hz, 1H), 4.34(q, J = 7.1 Hz, 1H), 4.09 - 4.14(m, 1H), 3.60 - 3.74(m, 2H), 3.01 - 3.05(m, 1H), 2.72 - 2.79(m, 1H), 2.58 - 2.63(m, 1H), 1.11(dd, J = 7.0, 2.2 Hz, 6H) ppm. The spectrum data showed good agreement with literature values.

### (c) Compound 5G

Compound 4G (1.5 g, 3.3 mmol) and 4,4'-dimethoxy trityl chloride (2.2 g, 6.6 mmol) were dissolved in pyridine (30 mL), and the mixture was stirred at room temperature for 16 hours. The reaction solution was concentrated by a rotary evaporator, and then the residue was dissolved in dichloromethane (50 mL) and washed with a saturated aqueous sodium bicarbonate solution (50 mL). The organic phase was dried with anhydrous sodium sulfate, and then the solvent was distilled off with a rotary evaporator. Purification was performed by column chromatography (neutral flash silica, developing solvent: methanol/dichloromethane = 1/20), and then 2.41 g (3.2 mmol) of Compound 5G was obtained as a white solid (yield 97%) .

¹H-NMR(600 MHz, DMSO-d₆) δ 12.11(s, 1H), 11.70(d, J = 17.3 Hz, 1H), 8.17 - 8.26(m, 1H), 7.89(dd, J = 8.3, 1.2 Hz, 2H), 7.52 - 7.79(m, 3H), 7.14 - 7.40(m, 9H), 6.73 - 6.82(m, 4H), 6.30(q, J = 3.5 Hz, 1H), 4.45 - 4.57 (m, 1H), 4.20 - 4.23(m, 1H), 3.61 - 3.79(m, 6H), 3.22 - 3.31(m, 2H), 3.17(dq, J = 13.7, 3.9 Hz, 1H), 2.61 - 2.82(m, 2H), 1.13(dd, J = 6.8, 1.4 Hz, 6H) ppm. The spectrum data showed good agreement with literature values.

### (d) Compound 6G, Compound 7G

Compound 5G (2.0 g, 2.6 mmol) was dissolved in a methanol/tetrahydrofuran mixed solvent (3/2, 90 mL), and the mixture was stirred in an ice bath for 30 minutes. Subsequently, a 0.5 M aqueous sodium hydroxide solution (18 mL, 9.0 mmol) was added, and then the mixture was treated at 0°C for 2 hours and at room temperature for 30 minutes under a deoxidizing condition by argon bubbling. To the reaction solution, a 1 M aqueous potassium dihydrogen phosphate solution (33 mL, 33 mmol) was slowly added dropwise to stop the reaction. Subsequently, the reaction solution was diluted with ethyl acetate (300 mL), and washed with a saturated aqueous sodium bicarbonate solution (300 mL) and saturated saline (300 ml). The organic phase was dried with anhydrous sodium sulfate, and then the solvent was distilled off with a rotary evaporator to obtain 1.7 g of Compound 6G as a white solid. 5-(Ethylthio)-1H-tetrazole (0.34 g, 2.6 mmol) was added to the obtained Compound 6G (1.7 g, 2.6 mmol), and dissolved in dichloromethane (40 mL). Subsequently, 2-cyanoethyl N,N,N',N'-tetraisopropyl phosphorodiamidite (1.3 mL, 4.0 mmol) was added thereto, and the mixture was stirred at room temperature for 1 hour. The reaction solution was diluted with dichloromethane (300 mL) and washed twice with saturated aqueous sodium bicarbonate solution (300 mL). The organic phase was dried with anhydrous sodium sulfate, and then the solvent was distilled off with a rotary evaporator. Purification was performed by column chromatography (neutral flash silica, developing solvent: acetonitrile/dichloromethane mixed solvent containing 1% triethylamine = 1/4), and 1.2 g (1.4 mmol) of Compound 7G was obtained as a brown solid (yield: 53%).

¹H-NMR (600 MHz, CDCl₃) δ 12.00(d, J = 10.2 Hz, 1H), 8.45(d, J = 34.0 Hz, 1H), 7.83 - 7.87(m, 1H), 7.41 - 7.43(m, 2H), 7.29 - 7.31(m, 4H), 7.17 - 7.24(m, 3H), 6.73 - 6.82(m, 4H), 6.14 - 6.17(m, 1H), 4.19 - 4.25(m, 1H), 3.87 - 3.55(11H), 3.54 - 3.27(2H), 3.00 - 3.12(m, 1H), 2.32 - 2.68(m, 4H), 1.09 - 1.21(m, 18H) ppm.

³¹P-NMR(243 MHz, CDCl₃)δ 160.81, 159.97 ppm. The spectrum data showed good agreement with literature values.

### (4) Synthesis of oligonucleotide having 3'-thiophosphate bond using thiated amidite reagent (Compound 5T) and analysis by reverse phase HPLC and MALDI-TOF-MS

### (a) DNA synthesis

Oligodeoxyribonucleotide 5'-TAACTsCACATTAATTGCGTT-FAM-3' was synthesized by an automatic nucleic acid synthesizer NR-2A 7MX (manufactured by Nippon Techno Service Co., Ltd.) according to a conventional method, using Compound 5T and a commercially available phosphoramidite reagent (ChemGenes). Ts represents a 3'thiated thymidine base. Fluorescein (FAM) was introduced into the 3' end. The various amidite reagents were 70 mM acetonitrile solutions, and only Compound 5T was used as a 150 mM acetonitrile solution. 0.3 M BTT was used as an activator and 0.05 M iodine (pyridine-/water; v/v: 9/1) solution was used as an oxidizing agent. In addition, a design was made in which FAM is introduced to the 3' end by using 6-FAM-glycerol support 500 Å (manufactured by ChemGenes) as a solid phase carrier. The synthesis was set to Final DMTr-ON and taken out while leaving the DMTr group on the 5'-end hydroxyl group. After completion of the synthesis, concentrated aqueous ammonia (500 µL) and a 40% aqueous methylamine solution (500 µL) were added to the solid phase carrier, and the solid phase carrier was cut out and deprotected by treatment at 65°C for 1 hour. The supernatant was filtered using a Millex LH filter (0.45 µm, manufactured by Merck Corporation), and then the filtrate was dried under reduced pressure by a centrifugal evaporator. The obtained oligonucleotide was redissolved in super deionized water, and the concentration was calculated by measuring the absorption at 260 nm. MALDI-TOF molecular weight measurement of the oligonucleotide was performed using 3-hydroxypicolinic acid as a matrix and using a linear positive mode of UltrafleXtreme (manufactured by Bruker).

### (b) Analysis results

Fig. 2 is a diagram showing results of synthesis an oligonucleotide having a 3'-thiophosphate bond using a thiated amidite reagent (compound 5T) and analysis by reverse phase HPLC and MALDI-TOF-MS.
(a) of Fig. 2 shows the results of synthesizing a 20 base-long oligodeoxyribonucleotide 5'-TAACTsCACATTAATTGCGTT-FAM-3' having a 3'-thiophosphate bond between the 4th base and the 5th base from the 5' side using Compound 5T and analyzing it by reverse phase HPLC. (a, b) HPLC analysis conditions are as follows.
   <Use system>
      · Column: YMC Hydrosphere C 18 (column size: 250 x 10.0 mm)
      · Eluent A: 50 mM triethylammonium acetate containing 5% acetonitrile (pH 7.0)
      · Eluent B: Acetonitrile
   <Gradient condition>
      · Flow rate: 3 mL/min
      · Column temperature: 50°C
      · Detection wavelength: 260 nm
   As a result of reverse phase HPLC, the main peak observed around the retention time of 18.8 minutes is 20 mer target DNA, and a DMTr group remains as a 5'-hydroxyl group protecting group. On the other hand, a product of which synthesis had been stopped at the introduction stage of Compound 5T was also partially observed around a retention time of 12.7 to 13.5 minutes, but was easily separable from an oligodeoxyribonucleotide (DNA) of a 20 mer target product. A peak around the retention time of 18.8 minutes was fractionated to obtain a 5'-DMTr protected product of the target DNA.
(b) of Fig. 2 shows the result of analysis of the obtained DNA by reverse phase HPLC in which the DMTr group was deprotected by adding a 10% aqueous acetic acid solution to the 5'-DMTr protected product fractionated and purified by reverse phase HPLC and treating the 5'-DMTr protected product at room temperature for 1 hour. As a result, one peak was observed around the retention time of 12.3 minutes, and it was confirmed that the target DNA was obtained with a high purity of 99% or more.
(c) of Fig. 2 shows the MALDI-TOF-MS molecular weight analysis result of the DNA obtained above. As a result, a peak at m/z 6650.7 derived from the target product was observed.

### 2. DNA strand cleavage reaction by metal nanoparticles of thiated oligonucleic acid

### (1) Silver nanoparticle size of DNA strand cleavage reaction by silver nanoparticle treatment

### (a) Silver nanoparticle treatment

10 µL of 3 µM PS-modified DNA (5'-TAACTsCACATTAATTGCGTT-FAM-3') was weighed, 50 µL of a silver nanoparticle dispersion (10 nm, 0.02 mg/mL) was added, and the mixture was incubated at 37°C for 31 hours. 20 µL of the reaction solution was weighed, and 20 µL of 2x loading buffer was added thereto. After heat treatment at 95°C for 5 minutes, analysis was performed by 15% denaturing acrylamide electrophoresis (containing 7.5 M urea, 10 x 12 cm, 30 mA, 20 min, 6 µL application). A gel electrophoresis image was acquired by FAM-derived fluorescence detection using a gel image analyzer (manufactured by BioRad Laboratories, Inc.).

### (b) Reaction time and reaction temperature dependency of DNA strand cleavage reaction by silver nanoparticle treatment

10 µL of 3 µM PS-modified DNA (5'-TAACTsCACATTAATTGCGTT-FAM-3') was weighed, 50 µL of a silver nanoparticle dispersion (10 nm, 0.02 mg/mL) was added, and the mixture was incubated at 70°C or 95°C for a predetermined time. 30 µL of the reaction solution was weighed, and 30 µL of 2x loading buffer was added thereto. After heat treatment at 95°C for 5 minutes, analysis was performed by 15% denaturing acrylamide electrophoresis (containing 7.5 M urea, 10 x 12 cm, 30 mA, 20 min, 6 µL application). A gel electrophoresis image was acquired by FAM-derived fluorescence detection using a gel image analyzer (manufactured by BioRad Laboratories, Inc.).

### (c) Results

Fig. 3 is a view showing an examination of a DNA strand cleavage reaction by silver nanoparticle treatment and the silver nanoparticle size and reaction time/reaction temperature dependency in the results.
(a) of Fig. 3 shows the silver nanoparticle size dependency of DNA strand cleavage by silver nanoparticles in the results. Using 10 nm, 20 nm and 100 nm silver nanoparticles, the generation of 15 mer DNA 5'-pCACATTAATTGCGTT-FAM-3' by strand cleavage of 20 mer DNA 5'-TAACTsCACATTAATTGCGTT-FAM-3' was analyzed by denaturing gel electrophoresis. Ts represents a 3'thiated thymidine base. In addition, FAM is introduced as a fluorescent dye at the 3' end. It is designed such that after cleavage, phosphoric acid (p) remains at the 5' end of the 15 mer DNA. The cleavage reaction was performed at 37°C for 31 hours. The band intensity analysis of the gel by FAM-derived fluorescence detection revealed that the cleavage efficiency was 35.9%, 21.9%, and 13.1% when the silver nanoparticles of 10 nm, 20 nm, and 100 nm were used, respectively, and the cleavage efficiency decreased as the nanoparticle diameter increased.
(b) of Fig. 3 shows the reaction time and reaction temperature dependency of DNA strand cleavage by silver nanoparticles. A strand cleavage reaction of 20 mer DNA was performed at 70°C or 95°C using 10 nm silver nanoparticles. Reactions were performed for 15, 30, 60, and 120 minutes, and each reaction solution was analyzed by denaturing gel electrophoresis. Cleavage efficiency at each temperature and time was calculated from band intensity analysis of the gel by FAM-derived fluorescence detection. The reaction time was plotted on the horizontal axis, and the cleavage efficiency was plotted on the vertical axis. As a result, it was revealed that the strand cleavage efficiency was improved in a reaction time-dependent manner, and after 2 hours from the start of the reaction, approximately 50% strand cleavage was observed at 70°C and 90% or more at 95°C, and the cleavage activity was improved with an increase in temperature. From this, it was found that a commercially available nanoparticle requires a high-temperature condition in order to induce highly efficient DNA strand cleavage.

### (2) DNA strand cleavage using surface PEG-modified silver nanoparticles

### (a) Polymer modification of silver nanoparticle surface

9 µL of 3.4 µM PS-modified DNA (5'-TAACTsCACATTAATTGCGTT-FAM-3') was weighed, 51 µL of the surface PEG-modified silver nanoparticle dispersion (10 nm) was added thereto, and the mixture was incubated at a predetermined time and temperature. A surface PEG-modified silver nanoparticle dispersion was prepared by adding 23.9 g/L of an aqueous solution of end thiolated PEG(O-[2-(3-mercaptopropionylamino)ethyl]-O'-methylpolyethylene glycol, average molecular weight 5,000, manufactured by Sigma-Aldrich Co. LLC.) (1 µL) to 50 µL of a commercially available silver nanoparticle dispersion (manufactured by Sigma-Aldrich Co. LLC., 10 nm, 0.02 mg/mL). 20 µL of the reaction solution was weighed, and 20 µL of 2x loading buffer was added thereto. After heat treatment at 95°C for 5 minutes, analysis was performed by 15% denaturing acrylamide electrophoresis (containing 7.5 M urea, 10 x 12 cm, 30 mA, 20 min, 6 µL application). A gel electrophoresis image was acquired by FAM-derived fluorescence detection using a gel image analyzer (manufactured by BioRad Laboratories, Inc.).

### (b) Results

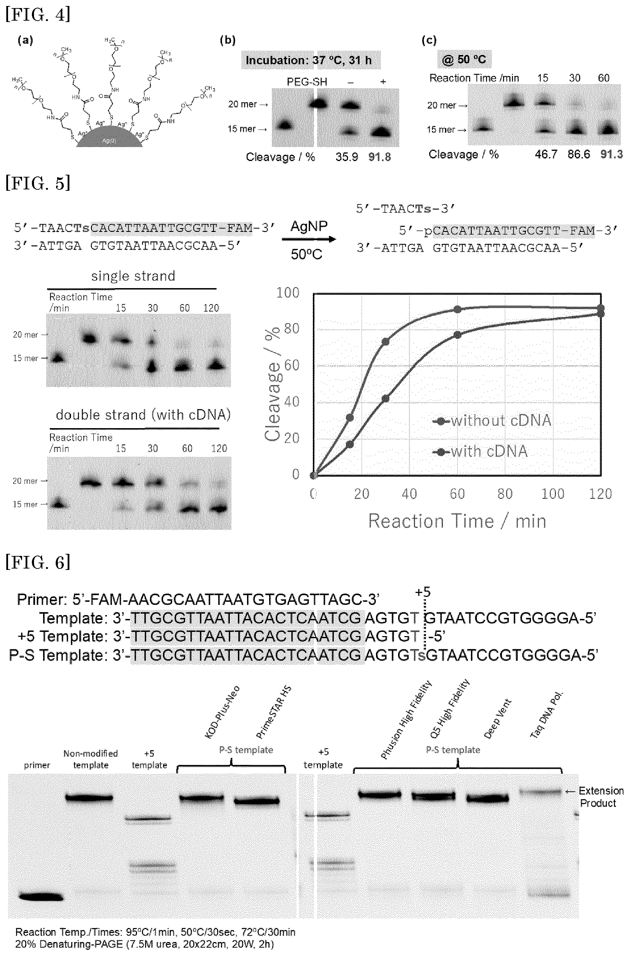

Fig. 4 is a view showing a result of improving DNA strand cleavage activity by polymer modification of a silver nanoparticle surface.
(a) of Fig. 4 is a schematic view showing a silver nanoparticle surface subjected to polymer modification. Modification with the end thiol-modified polyethylene glycol having an average molecular weight of 5,000 was performed. Surface-modified nanoparticles can be easily prepared by mixing any amount of polymer with a commercially available silver nanoparticle dispersion. It is also possible to control the amount of modification by adjusting the amount of polymer to be added.
(b) of Fig. 4 shows the result of comparison of DNA strand cleavage activity in the presence or absence of surface modification of silver nanoparticles of 1 nm. In Fig. 4, PEG-SH represents polyethylene glycol having an end thiol. The reaction was performed at 37°C for 31 hours, the reaction solution was analyzed by denaturing gel electrophoresis, the band intensity of the gel was analyzed by FAM-derived fluorescence detection, and the cleavage efficiency was calculated. As a result, the cleavage efficiency was 35.9% in the case without PEG-SH modification, whereas the cleavage activity was 91.8% in the case of PEG-modified silver nanoparticles, and the cleavage activity was significantly improved by surface PEG modification. This cleavage activity improving effect is described by surface oxidation prevention of silver nanoparticles by PEG modification and improvement of dispersibility in an aqueous solution.
(c) of Fig. 4 shows the reaction time dependency of DNA strand cleavages using 1 nm surface PEG-modified silver nanoparticles in the results. Reactions were performed at 50°C for 15, 30, and 60 minutes, and each reaction solution was analyzed by denaturing gel electrophoresis. Cleavage efficiency at each reaction time was calculated from band intensity analysis of the gel by FAM-derived fluorescence detection. As a result, the strand cleavage efficiency was improved in a reaction time-dependent manner, and cleavage of 90% or more was observed after 60 minutes from the start of the reaction. From this result, PEG modification of the surface with commercially available silver nanoparticles enabled highly efficient DNA strand cleavage under relatively mild temperature conditions.

### (3) Preparation of 3' overhang sticky ends by silver nanoparticle treatment of double-stranded short-strand DNA

### (a) Preparation of 3' overhang sticky end

PS-modified DNA (5'-TAACTsCACATTAATTGCGTT-FAM-3') and complementary strand DNA (5'-AACGCAATTAATGTGAGTTA-3') were mixed to be 3 µM each, treated at 95°C for 3 minutes, and then annealed by cooling on an ice bath for 10 minutes or more. 9 µL of the solution after annealing was weighed, and 51 µL of a surface PEG-modified silver nanoparticle dispersion (10 nm) was added, and the mixture was incubated at 50°C for a predetermined time. The surface PEG-modified silver nanoparticle dispersion was prepared by adding 1 µL of an end thiol PEG aqueous solution (23.9 g/L) to 50 µL of a commercially available silver nanoparticle dispersion (manufactured by Sigma-Aldrich Co. LLC., 10 nm, 0.02 mg/mL). 30 µL of the reaction solution was weighed, and 30 µL of 2x loading buffer was added thereto. After heat treatment at 95°C for 5 minutes, analysis was performed by 15% denaturing acrylamide electrophoresis (containing 7.5 M urea, 10 x 12 cm, 30 mA, 20 min, 6 µL application). A gel electrophoresis image was acquired by FAM-derived fluorescence detection using a gel image analyzer (manufactured by BioRad Laboratories, Inc.).

### (b) Results

Fig. 5 shows the results of preparing 3' overhang sticky ends by silver nanoparticle treatment of double-stranded short-strand DNA. Cleavage of 20 mer double DNA with 3'-SP bonds was performed using 1 nm surface PEG-modified silver nanoparticles. The SP bond is positioned between the 5th base and the 6th base from the 5' end, and was designed such that a sticky end protruding by 5 bases can be prepared when cleavage occurs. Reactions were performed at 50°C for 15, 30, and 60 minutes, and each reaction solution was analyzed by denaturing gel electrophoresis. Cleavage efficiency at each reaction time was calculated from band intensity analysis of the gel by FAM-derived fluorescence detection. The cleavage efficiency was compared between the presence and absence of the complementary strand, and the cleavage efficiency at each reaction time was plotted on the vertical axis, and the reaction time was plotted on the horizontal axis. As a result, in the case of the presence of the complementary strand, the cleavage activity was slightly reduced as compared with the case of the absence of the complementary strand, but the cleavage activity was almost the same 2 hours after the start of the reaction, and 90% or more of the DNA was cleaved. From this, it was revealed that DNA strand cleavage by surface PEG-modified silver nanoparticles can be applied to preparation of sticky end DNA.

### 3. Application to oligonucleic acid ligation using thiated oligonucleic acid as primer for polymerase chain extension reaction (PCR)

### (1) Experiment

### (a) PCR study using thiated DNA as template

110 µM thiated template DNA (0.37 µL) and 15 µL primer DNA (1.34 µL) were mixed, and according to the manufacturer's recommended conditions for various polymerases, a dNTP mixture, various polymerases (KOD-Pkus-Neo, PrimeSTAR HS, Phusion High Fidelity, Q5 High Fidelity, Deep Vent, Taq DNA polymerase) and a polymerase attachment buffer were added to make the total liquid volume 20 µL. Subsequently, denaturation (95°C, 1 min)-annealing (50°C, 30 seconds)-strand extension (72°C, 30 min) was performed, and the reaction solution was diluted with 2x loading buffer (20 µL). Analysis was performed by 20% denaturing gel electrophoresis (containing 7.5 M urea, 20 x 22 cm, 20 W, 2 h). The gel was stained by shaking with 1 x SYBR Green II solution for 30 minutes, and a gel electrophoresis image was obtained with a gel image analyzer (manufactured by BioRad Laboratories, Inc.).

### (b) PCR with thiated DNA primers

20 µM thiated forward primer (1.25 µL), 20 µM thiated reverse primer (1.25 µL), 10 ng/µL template DNA (2.5 µL), 2 mM dNTP mixture (5 µL), 25 mM magnesium sulfate (3 µL), 10 x PCR buffer for KOD-Plus-Neo (5 µL), and super deionized water (31 µL) were mixed, 1 U/µL KOD-Plus-Neo (1 µL) was added, and then the mixture was treated at 95°C for 2 minutes using a thermal cycler (manufactured by BioRad Laboratories, Inc.). Subsequently, DNA amplification was performed by performing 30 cycles of denaturation (95°C, 15 seconds)-annealing (55°C, 15 seconds)-strand extension (68°C, 30 seconds). The PCR product was purified using a wizard column (manufactured by Promega Corporation) according to the manufacturer's recommended protocol to obtain thiated DNA.

### (c) Preparation of sticky fragments by metal nanoparticle treatment

7.5 nM PCR product thiated DNA (225 fmol/sample) was dissolved in super deionized water to prepare 4.5 µL of an aqueous solution. PEG-modified silver nanoparticles were prepared by mixing 10 nm silver nanoparticles (manufactured by Sigma-Aldrich Co. LLC., 200 µg/mL citric acid buffer suspension, 25 µL) with end thiol-modified PEG (manufactured by Sigma-Aldrich Co. LLC., 23.9 g/L aqueous solution, 0.5 µL). The thionized DNA aqueous solution (4.5 µL) of the prepared PCR product and the PEG-modified silver nanoparticle dispersion (25.5 µL) were mixed, and then the mixture was treated at 50°C for 2 to 4 hours to cleave the SP bond, thereby preparing a sticky end.

### (d) Ligation by DNA ligase and gel analysis

7.5 nM silver nanoparticle-treated product DNA_1 (3.6 µL), 7.5 nM silver nanoparticle-treated product DNA_2 (3.6 µL), 10 x T4 DNA Ligase Reaction Buffer (manufactured by New England BioLabs, 0.90 µL), and super deionized water (0.45 µL) were mixed, then T4 DNA ligase (manufactured by New England BioLabs, 2000 U/µL, 0.45 µL) was added, and the mixture was incubated at 25°C for 3 hours. 1 µL of 10 x loading buffer was added to the reaction solution (9 µL) and analyzed by 1% agarose gel electrophoresis (electrophoretic buffer: 1 x TBE, 100 V, 30 min). The gel was stained by shaking with 10,000 x SYBR Green I solution for 30 minutes, and a gel electrophoresis image was obtained with a gel image analyzer (manufactured by BioRad Laboratories, Inc.).

### (2) Results

Fig. 6 shows the result of PCR study when a thiated oligonucleic acid was introduced into a DNA template. A 22 mer 5'-FAMylated DNA primer 5'-FAM-AACGCAATTAATGTGAGTTAGC-3' was annealed to template DNA 5'-AGGGGTGCCTAATGTsGTGAGCTAACTCACATTAATTGCGTT-3' having a 3'-SP bond introduced between the 14th base and the 15th base from the 5' end, and a polymerase extension reaction was performed by various polymerases (KOD-Pkus-Neo, PrimeSTAR HS, Phusion High Fidelity, Q5 High Fidelity, Deep Vent, Taq DNA polymerase). When the extension stops at the 3'-SP modification site, a product is obtained by extending the 22 mer primer by 4 bases or 5 bases. On the other hand, when the 3'-SP modification site extended without any problem, a full-length 41 mer DNA is generated. The reaction solution was analyzed by 20% denaturing gel electrophoresis (containing 7.5 M urea, 20 x 22 cm, 20 W, 2 h), and bands were visualized with a gel image analyzer (manufactured by BioRad Laboratories, Inc.) using fluorescence detection derived from 5'-FAM. As a result, even when any polymerase was used, a band derived from DNA having a full length of 41 mer was observed, and no extension stop product at the 3'-SP modification site was observed. This revealed that the DNA into which the 3'-SP modification was introduced can function as a primer.

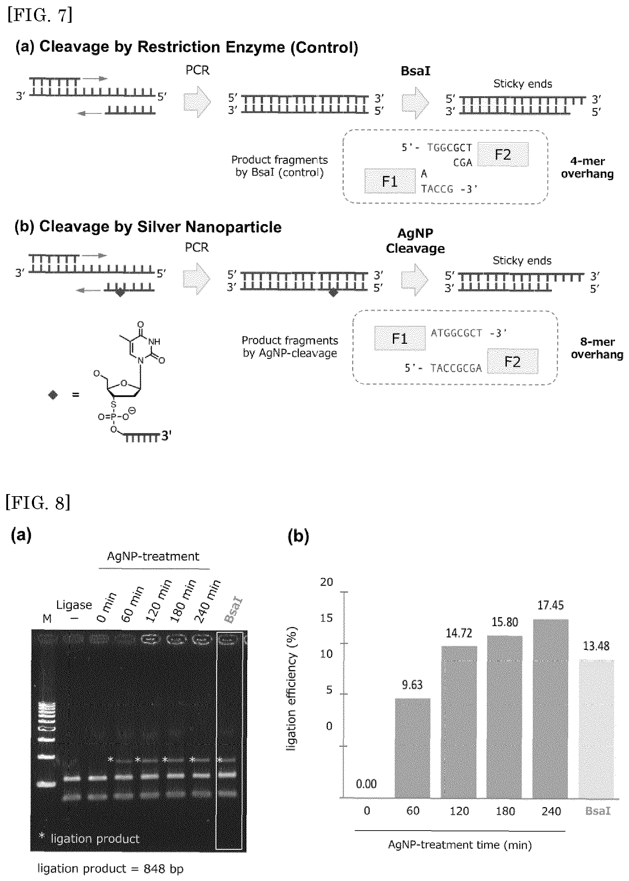

Fig. 7 is a schematic diagram of DNA amplification by PCR and preparation of a sticky fragment by BsaI or silver nanoparticle treatment. (a) of Fig. 7 shows the preparation result of 4-base overhang sticky ends using the restriction enzyme BsaI. (b) of Fig. 7 shows the preparation result of 8-base overhang sticky ends utilizing 3'-SP bond cleavage by silver nanoparticle treatment. As the silver nanoparticles, those having a size of 10 nm and subjected to surface PEG modification were used.

Fig. 8 shows the results of an experiment of linking sticky fragments using T4 DNA ligase.
(a) of Fig. 8 shows the results of linkage of two sticky fragment DNAs prepared by silver nanoparticle-treatment or restriction enzyme BsaI treatment as a control experiment with T4 DNA ligase and analysis by 1% agarose gel electrophoresis (electrophoretic buffer: 1 x TBE, 100 V, 30 min). The reaction was performed at 25°C for 3 hours, the PCR product DNA was treated with silver nanoparticles for 60, 120, 180, 240 minutes, and the cleavage reaction product at each reaction time was used to compare the linking efficiency by T4 DNA ligase. From the results of the gel, generation of an 838 bp linked product was confirmed under all linking reaction conditions.
(b) of Fig. 8 shows a graph showing the linking efficiency of the product and the product by the BsaI treatment at each silver nanoparticle treatment time. As the silver nanoparticle treatment time was increased, the amount of the linked product increased. It was shown that, when the DNA fragments prepared by the silver nanoparticle treatment for 240 minutes were linked, the linking efficiency was higher than that of linking of the DNA fragments prepared by the BsaI treatment.

### 4. Comparison between silver nitrate treatment (existing technique) and silver nanoparticle treatment (technique of the present invention) in cleavage of thiated oligonucleic acid strand

### (1) Thioligonucleic acid strand cleavage by silver nitrate treatment

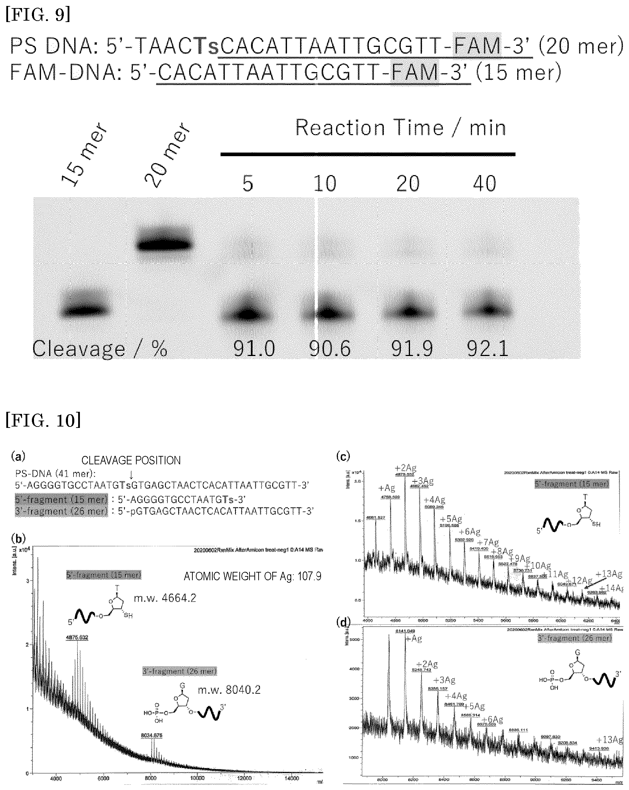

30 µM PS-modified DNA 20 mer (5'-TAACTsCACATTAATTGCGTT-FAM-3') was used. In addition, Ts represents a 3' thiated thymidine base. In addition, FAM is introduced as a fluorescent dye at the 3' end. 1 µL of the PS-modified DNA was weighed and mixed with a 50 mM silver nitrate aqueous solution (29 µL), and then incubated at room temperature for 5 to 40 minutes to perform a cleavage reaction. When the predetermined time was reached, 5 µL of each of the reaction solutions was weighed, and a 60 mM aqueous ethanethiol solution (5 µL) was added to stop the reaction. At this time, generation of a white precipitate derived from silver ions was confirmed. 2x loading buffer (10 µL) was added to this mixture (10 µL). After heat treatment at 95°C for 5 minutes, analysis was performed by 15% denaturing acrylamide electrophoresis (containing 7.5 M urea, 10 x 12 cm, 30 mA, 20 min, 6 µL application). A gel electrophoresis image was acquired by FAM-derived fluorescence detection using a gel image analyzer (manufactured by BioRad Laboratories, Inc.). The results are shown in Fig. 9.

Fig. 9 is a view showing a result of cleavage of a thiated oligonucleic acid strand by silver nitrate treatment as an existing technique. Generation of 15 mer PS-DNA 5'-pCACATTAATTGCGTT-FAM-3', which is a strand cleaved product by silver nitrate treatment of 20 mer PS-DNA 5'-TAACTsCACATTAATTGCGTT-FAM-3' was analyzed by denaturing gel electrophoresis. It is designed such that after cleavage, phosphoric acid (p) remains at the 5' end of the 15 mer DNA. Band intensity analysis of the gel by FAM-derived fluorescence detection showed that 90% or more of DNA was cleaved at 5 minutes after the start of the reaction.

### (2) Molecular weight analysis by MALDI-TOF-MS of a cleaved product in the cleavage of a thiated oligonucleic acid strand by silver nitrate treatment

3 µL of 110 µM PS-modified DNA 41 mer (5'-AGGGGTGCCTAATGTsGTGAGCTAACTCACATTAATTGCGTT-3') was weighed and mixed with 50 mM silver nitrate aqueous solution (67 µL), and then incubated at room temperature for 22 hours. The reaction solution was subjected to ultrafiltration centrifugal filter (Amicon Ultra 3K, manufactured by Merck Ltd.) according to the manufacturer's recommended protocol, and silver nitrate was removed by cutting off molecules having a molecular weight of less than 3 **K.** By this operation, silver ions and the like contained in the reaction system can be roughly removed. The solution remaining after ultrafiltration was recovered and subjected to MALDI-TOF-MS molecular weight analysis. MALDI-TOF-MS molecular weight analysis was performed using 3-hydroxypicolinic acid as a matrix in a linear negative mode of UltraFleXtreme (manufactured by Bruker). The results are shown in Fig. 10.

Fig. 10 is a view showing a molecular weight analysis result by MALDI-TOF-MS of a cleaved product in the cleavage of a thiated oligonucleic acid strand by silver nitrate treatment as an existing technique.
(a) of Fig. 10 shows the sequence of cleavage target DNA (41 mer) 5'-AGGGGTGCCTAATGTsGTGAGCTAACTCACATTAATTGCGTT-3' and the cleaved product DNA fragment thereof. It is designed that cleavage is performed at the 3'-SP binding site indicated by the arrow in Fig. 10, and the 5'-fragment (15 mer) side becomes 3'-SH (5'-AGGGGTGCCTAATGTs-3'), and the 3'-fragment (26 mer) becomes a 5'-phosphate form (5'-p GTGAGCTAACTCACATTAATTGCGTT-3', p represents a phosphate group).
(b) of Fig. 10 is a view showing MALDI-TOF-MS molecular weight analysis of a cleavage reaction solution by silver nitrate treatment. Peaks corresponding to the molecular weights of 4664.2 and 8040.2 of 5'-fragment (15 mer) and 3'-fragment (26 mer) generated by cleavage were observed, and thus it is considered that a target DNA fragment was generated.
(c) of Fig. 10 is an enlarged view of a MALDI-TOF-MS spectral peak region derived from a cleaved product 5'-fragment (15 mer) by silver nitrate treatment.
(d) of Fig. 10 is an enlarged view of a MALDI-TOF-MS spectral peak region derived from a cleaved product 3'-fragment (26 mer) by silver nitrate treatment.

From Figs. (c) and (d), it was shown that the cleaved product was present in a form in which a plurality of silver ions were added to the phosphate binding site. On the MALDI-TOF-MS spectrum, the silver ion addition number was detected as a peak having a clustered molecular weight distribution different from each other. From these results, it was shown that it was difficult to remove excessively added silver ions by a simple ultrafiltration treatment in the cleavage of the thiated oligonucleic acid strand by the silver nitrate treatment. Silver ions are strongly bound to chloride ions (Cl⁻) contained in a buffer used for physiological conditions and biochemical experiments, and poorly watersoluble AgCl is formed and precipitates (the water solubility of AgCl was 192 µg/100 m: J. Phys. Chem. C., 2015, 119, 20632-20641). Therefore, a cleavage system using silver ions in which silver ions remain in the solution even when the DNA cleavage reaction occurs with high efficiency is difficult to apply to the ligation reaction after the DNA cleavage reaction.

### (3) Comparison between thioated oligonucleic acid strand cleavage by silver nitrate treatment and recovery amount of cleaved product DNA after silver ion removal by addition of thiols

It has been reported that silver ions can be removed as a precipitate by high interaction with thiols. Therefore, the reaction was stopped by adding dithiothreitol (DTT) or ethanethiol (EtSH) to the cleavage reaction solution, and a precipitate derived from generated silver was removed by centrifugation. The supernatant was recovered and analyzed by denaturing gel electrophoresis.

10 µL (1.10 nmol) of 110 µM PS-modified DNA 41 mer (5'-AGGGGTGCCTAATGTsGTGAGCTAACTCACATTAATTGCGTT-3') was weighed and mixed with a 50 mM silver nitrate aqueous solution (90 µL), and then incubated at room temperature for 1 day to perform a cleavage reaction. 20 µL (220 pmol) of the reaction solution was weighed, and the reaction was stopped by adding a 60 mM dithiothreitol (DTT) aqueous solution (20 µL) or a 60 mM ethanethiol (EtSH) aqueous solution (20 µL). At this time, generation of a white precipitate derived from silver ions was confirmed. This mixture was diluted with super deionized water (160 µL) and then incubated at room temperature for 20 minutes. Subsequently, a precipitate derived from silver ions was removed by centrifugation (15,000 rpm, 15 min). The supernatant was recovered and concentrated using an ultrafiltration centrifugal filter (Amicon Ultra 3K, manufactured by Merck Ltd.) according to the manufacturer's recommended protocol.

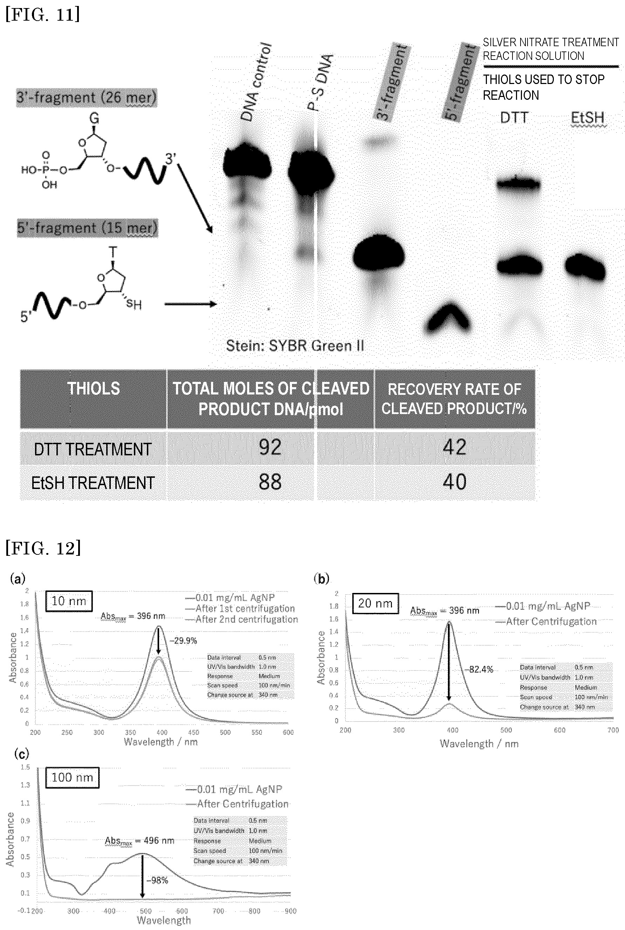

The amount of the product recovered was calculated by measuring the absorbance of the concentrated sample solution at 260 nm derived from the nucleic acid using NanoDrop to quantify the cleaved product. In calculating the amount of the cleaved product, DNA was quantified according to the Lambert-Beer law using ε₂₆₀ = 396, 900 L·mol⁻¹·cm⁻¹, which is the sum of the molar absorbance coefficients at 260 nm of both cleaved products, or ε₂₆₀ = 250,000 L·mol⁻¹·cm⁻¹, which is the molar absorbance coefficient at 260 nm of the cleaved product on the 3' side (5'-pGTGAGCTAACTCACATTAATTGCGTT-3'). 5 µL of the concentrated sample solution was weighed and mixed with 2x loading buffer (5 µL). After treatment at 95°C for 10 minutes, analysis was performed by 15% denaturing acrylamide electrophoresis (containing 7.5 M urea, 10 x 12 cm, 30 mA, 20 min, 6 µL application). The gel after electrophoresis was taken out, immersed in a 1 x SYBR Green II aqueous solution, and shaken for 20 minutes to stain DNA bands present in the gel. A gel electrophoresis image was acquired using a gel image analyzer (manufactured by BioRad Laboratories, Inc.) by fluorescence detection derived from SYBR Green II bound to DNA. The results are shown in Fig. 11.

Fig. 11 show the results of examination of cleavage of a thiated oligonucleotide strand by silver nitrate treatment and removal of silver ions by addition of thiols as existing techniques. It is designed that a 41 mer 5'-AGGGGTGCCTAATGTsGTGAGCTAACTCACATTAATTGCGTT-3' is used as the cleavage target DNA, and as shown in Fig. 11, cleavage is performed at the 3'-SP binding site, and 5'-fragment (15 mer) 5'-AGGGGTGCCTAATGTs-3') and 3'-fragment (26 mer) 5'-pGTGAGCTAACTCACATTAATTGCGTT-3' are generated.

For DNA staining by gel analysis, SYBR Green II was used to visualize bands on the gel by fluorescence detection from SYBR Green II bound to DNA. As a result, in a system using DTT to stop the reaction, a band derived from dimerization of a thiol site of 5'-fragment by a disulfide bond together with cleaved products 5'-fragment and 3'-fragment was observed. The band derived from dimerization has almost the same mobility as that of the 41 mer 5'-AGGGGTGCCTAATGTsGTGAGCTAACTCACATTAATTGCGTT-3', which is a cleavage target DNA of the raw material, and it was difficult to discriminate the band, but after cutting out the gel band and extracting DNA from the gel piece, MALDI-TOF-MS molecular weight analysis of the extract revealed that a dimerized form of 5'-fragment was obtained.

In the system in which EtSH was used to stop the reaction, the band derived from the 41 mer 5'-AGGGGTGCCTAATGTsGTGAGCTAACTCACATTAATTGCGTT-3', which is a cleavage target DNA, completely disappeared, and only the cleaved product 3'-fragment (26 mer) 5'-pGTGAGCTAACTCACATTAATTGCGTT-3' was observed on the gel. This is believed to be because 5'-fragment (15 mer) 5'-AGGGGTGCCTAATGTs-3', a cleaved product on the 5' side, strongly bound to EtSH and formed a precipitate with silver ions, and thus after silver ion precipitation removal, only 3'-fragment (26 mer) 5'-pGTGAGCTAACTCACATTAATTGCGTT-3', a cleaved product on the 3' side, was recovered as a supernatant.

Furthermore, the results of quantifying the amount of DNA contained in the supernatant after removal of silver ion precipitation by addition of thiols by absorbance measurement at 260 nm derived from DNA are shown in the table in Fig. 11. The total amount of DNA per reaction solution was 220 pmol, but the amount of DNA recovered as a cleaved product was 92 pmol in the case of the DTT-added system and 88 pmol in the case of the EtSH-added system, and the recovery rate was approximately 40% in both systems. From this result, it was shown that in the silver ion precipitation removal method using thiols, approximately 60% of the cleaved product DNA was coprecipitated together with silver ions, and the yield of the cleaved product was reduced. From this, it was revealed that the method for cleaving a thiated oligonucleic acid strand by silver nitrate, which is an existing technique, is difficult to put into practical use.

### (4) Demonstration of removal of silver nanoparticles by centrifugation

It is known that silver nanoparticles exhibit absorption ranging over a range of 350 to 700 nm depending on a particle diameter due to a surface plasmon resonance effect. Therefore, by acquiring the absorption spectrum of the dispersion, the amount of nanoparticles present in the dispersion can be estimated from the absorbance.

700 µL of a dispersion (manufactured by Sigma-Aldrich Co. LLC., 0.02 mg/mL sodium citrate aqueous solution) of silver nanoparticles having particle diameters of 10, 20, and 100 nm was weighed and diluted with super deionized water (700 µL), and then an absorption spectrum was measured using a quartz cell (optical path length: 1 cm, optical path width: 1 cm). The device used for the measurement was an ultraviolet-visible spectrophotometer V-650 manufactured by JASCO Corporation. As measurement conditions, data interval was set to 0.5 nm, bandwidth was set to 1.0 nm, response was set to medium, and scanning speed was set to 100 nm/min. After measuring the absorption spectrum, the silver nanoparticle dispersion diluted with super deionized water (700 µL) was transferred to an Eppendorf tube, and centrifuged at 15,000 rpm for 1 hour to precipitate nanoparticles. The supernatant was recovered and transferred again to a quartz cell (optical path length: 1 cm, optical path width: 1 cm), and then the absorption spectrum was measured. The results are shown in Fig. 12.

Fig. 12 is a view showing removal (nanoparticle size dependency) of silver nanoparticles by centrifugation. (a) of Fig. 12 shows the results when silver nanoparticles having a particle diameter of 10 nm were used. (b) of Fig. 12 shows the results when silver nanoparticles having a particle diameter of 20 nm were used. (c) of Fig. 12 shows the results when silver nanoparticles having a particle diameter of 100 nm were used. The blue spectrum in Fig. 12 is from a 0.01 mg/mL silver nanoparticle dispersion. The orange spectrum in Fig. 12 is the result of measurement in which nanoparticles are precipitated by centrifuging the nanoparticle dispersion at 15,000 rpm for 1 hour and the obtained supernatant is recovered.

As a result, 29.9% of the silver nanoparticle dispersion having a particle diameter of 10 nm, 82.4% of the silver nanoparticle dispersion having a particle diameter of 20 nm, and almost 100% of the silver nanoparticle dispersion having a particle diameter of 100 nm, and a decrease in absorbance around 400 to 500 nm was observed by centrifugation. This result means that nanoparticles floating in the dispersion liquid were precipitated by centrifugation operation. Therefore, it was found that silver nanoparticles having a particle diameter of 10 to 100 nm can be removed from the reaction solution as a precipitate by centrifugation. Furthermore, it was shown that the larger the silver nanoparticles size, the easier it is to remove the silver nanoparticles from the reaction system by centrifugation, and nanoparticles having a particle diameter of 20 nm or more can be removed by centrifugation.

### (5) Comparison between silver nitrate treatment and silver nanoparticle treatment in cleavage of thiated oligonucleic acid strand (calculation of recovery amount of oligonucleic acid)

### (a) Silver nitrate treatment

2 µL of 122 µM PS-modified DNA 20 mer (5'-TAACTsCACATTAATTGCGTT-FAM-3') was weighed and mixed with 50 mM silver nitrate aqueous solution (38 µL), and then incubated at room temperature for 1.5 hours. When the predetermined time was reached, the reaction was stopped by adding a 60 mM DTT aqueous solution (40 µL) to the reaction solution (40 µL). At this time, generation of a white precipitate derived from silver ions was confirmed. This mixture (80 µL) was diluted with super deionized water (20 µL) and well mixed, and then centrifuged at 15,000 rpm for 1 hour to remove the precipitate. The supernatant was recovered and concentrated using an ultrafiltration centrifugal filter (Amicon Ultra 3K, manufactured by Merck Ltd.) according to the manufacturer's recommended protocol. The amount of the product recovered was calculated by measuring the absorbance of the concentrated sample solution at 260 nm derived from the nucleic acid using NanoDrop to quantify the cleaved product. The concentrated sample solution was diluted with super deionized water to prepare a 0.50 µM aqueous solution. 5 µL of 0.50 µM cleaved product solution was weighed and mixed with 2x loading buffer (5 µL). After treatment at 95°C for 5 minutes, analysis was performed by 15% denaturing acrylamide electrophoresis (containing 7.5 M urea, 10 x 12 cm, 30 mA, 20 min, 5 µL application). A gel electrophoresis image was acquired by FAM-derived fluorescence detection using a gel image analyzer (manufactured by BioRad Laboratories, Inc.).

### (b) Silver nanoparticle treatment

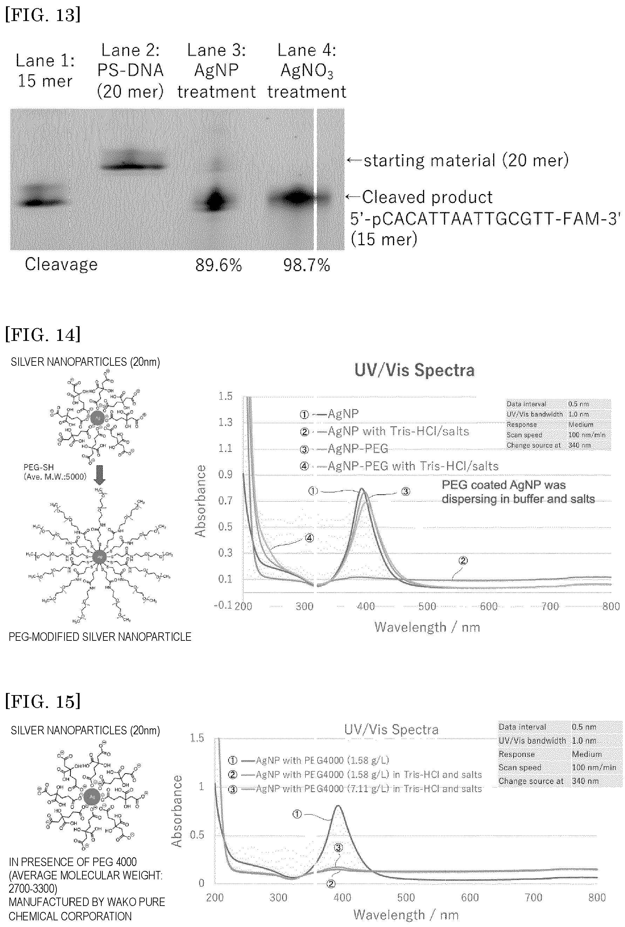

2 µL of 122 µM PS-modified DNA 20 mer (5'-TAACTsCACATTAATTGCGTT-FAM-3') was weighed and mixed with a dispersion of silver nanoparticles having a particle diameter of 100 nm (manufactured by Sigma-Aldrich Co. LLC., 0.02 mg/mL sodium citrate aqueous solution) (98 µL), and then incubated at 90°C for 25 hours. The reaction solution was centrifuged at 15,000 rpm for 1 h to remove silver nanoparticles as precipitates. The supernatant was recovered and concentrated using an ultrafiltration centrifugal filter (Amicon Ultra 3K, manufactured by Merck Ltd.) according to the manufacturer's recommended protocol. The amount of the product recovered was calculated by measuring the absorbance of the concentrated sample solution at 260 nm derived from the nucleic acid using NanoDrop to quantify the cleaved product. The concentrated sample solution was diluted with super deionized water to prepare a 0.50 µM aqueous solution. 5 µL of 0.50 µM cleaved product solution was weighed and mixed with 2x loading buffer (5 µL). After treatment at 95°C for 5 minutes, analysis was performed by 15% denaturing acrylamide electrophoresis (containing 7.5 M urea, 10 x 12 cm, 30 mA, 20 min, 5 µL application). A gel electrophoresis image was acquired by FAM-derived fluorescence detection using a gel image analyzer (manufactured by BioRad Laboratories, Inc.). The results are shown in Fig. 13.

Fig. 13 is a comparison between silver nitrate treatment and silver nanoparticle treatment in cleavage of a thiated oligonucleic acid strand. In the gel image, Lane 1 is a result of electrophoresis of DNA (15 mer) 5'-CACATTAATTGCGTT-FAM-3' having the same base length as the DNA generated after cleavage. Lane 2 is the result of electrophoresis of cleavage target DNA (20 mer) 5'-TAACTsCACATTAATTGCGTT-FAM-3'. Ts in the sequence represents a 3'thiated thymidine base. In addition, FAM is introduced as a fluorescent dye at the 3' end. Lane 3 is the result of electrophoresis of a reaction product when cleavage of a thioated oligonucleic acid strand was performed using silver nanoparticles having a particle diameter of 100 nm. The reaction was performed by adding silver nanoparticles and then incubating at 90°C for 25 hours. The silver nanoparticles in the reaction solution were removed as precipitates by centrifugation at 15,000 rpm for 1 hour, and then subjected to electrophoresis. As a result, it was shown that almost all the cleavage target DNA (20 mer) disappeared and was converted into cleaved product DNA (15 mer) (89.6%). Lane 4 is the result of electrophoresis of the reaction product when the thiated oligonucleic acid strand was cleaved using silver nitrate. The reaction was performed by adding silver nitrate and then incubating at room temperature for 1.5 hours. Silver ions in the reaction solution were removed as a precipitate by adding DTT, and then electrophoresis was performed. As a result, it was shown that the cleavage target DNA (20 mer) was cleaved and converted into the cleaved product DNA (15 mer) (98.7%).

**[Table 1]**

| **Cleaving agent** | **Moles of cleaved product DNA** | **Recovery rate of cl eaved product** |
|---|---|---|
| Silver nitrate (Ag⁺) | 35.1 pmol | 14.4% |
| Silver nanoparticle (100 nm) | 240 pmol | 98.4% |

The above is a table showing a comparison of the recovery amount of the cleaved product DNA in the silver nitrate treatment and the silver nanoparticle treatment. In the case of silver nitrate treatment, the amount of DNA contained in the supernatant after removal of silver ion precipitation by addition of DTT was quantified by absorbance measurement at 260 nm derived from DNA. In the case of silver nanoparticle treatment, silver nanoparticles were removed as precipitates by centrifugation, and then the amount of DNA contained in the supernatant was quantified by measuring the absorbance at 260 nm derived from DNA. As a result, while the total amount of DNA per reaction solution was 244 pmol, in the case of the silver nitrate treatment, the amount of DNA recovered from the cleaved product was 35.1 pmol, and the recovery rate was only 14.4%, and it is considered that most of the cleaved product was coprecipitated when silver ion precipitation was removed by addition of DTT. On the other hand, the amount was 240 pmol in the case of the silver nanoparticle treatment, and the cleaved product DNA was almost quantitatively recovered. From this result, it is difficult to put the method for cleaving a thiated oligonucleic acid strand using silver nitrate, which is an existing technique, into practical use because the yield is greatly reduced, but the method for cleaving a thiated oligonucleic acid strand using silver nanoparticles is a practical technique capable of recovering a cleaved product with high efficiency.

### (6) Evaluation of dispersibility accompanying surface PEG modification of silver nanoparticles

Surface modification of silver nanoparticles can be easily prepared by mixing end thiolated polyethylene glycol (PEG-SH) with a commercially available silver nanoparticle dispersion (particle diameter 20 nm with citric acid as antioxidant). PEG-SH having an average molecular weight of 5,000 was used. Dispersibility of the nanoparticles was evaluated by measuring an absorption spectrum based on the surface plasmon resonance effect of the nanoparticle dispersion.

The absorption spectrum of the silver nanoparticle dispersion without surface modification was measured using a quartz cell (optical path length: 1 cm, optical path width: 1 cm) after 350 µL of a dispersion (manufactured by Sigma-Aldrich Co. LLC., 0.02 mg/mL citrate aqueous solution) of silver nanoparticles having a particle diameter of 20 nm was weighed and diluted with super deionized water (1050 µL). The absorption spectrum of the silver nanoparticle dispersion without surface modification in the presence of buffer salts was measured using a quartz cell (optical path length: 1 cm, optical path width: 1 cm) by measuring 350 µL of a dispersion (manufactured by Sigma-Aldrich Co. LLC., 0.02 mg/mL sodium citrate aqueous solution) of silver nanoparticles having a particle diameter of 20 nm, diluting the dispersion with super deionized water (630 µL), and then adding 100 mM Tris-HCl buffer (pH 8.3) (140 µL), 500 mM potassium chloride aqueous solution (140 µL), and 15 mM magnesium chloride aqueous solution (140 µL) to prepare a sample.

The absorption spectrum of the dispersion of surface PEG-modified silver nanoparticles was measured using a quartz cell (optical path length: 1 cm, optical path width: 1 cm) by measuring 350 µL of a dispersion (manufactured by Sigma-Aldrich Co. LLC., 0.02 mg/mL sodium citrate aqueous solution) of silver nanoparticles having a particle diameter of 20 nm, adding 23.9 g/L of an end thiolated PEG(O-[2-(3-mercaptopropionylamino)ethyl]-O'-methylpolyethylene glycol, average molecular weight 5,000, manufactured by Sigma-Aldrich Co. LLC.) aqueous solution (140 µL), then diluting the dispersion with super deionized water (910 µL). The absorption spectrum of the surface PEG-modified silver nanoparticle dispersion in the presence of buffer salts was measured using a quartz cell (optical path length: 1 cm, optical path width: 1 cm) by measuring 350 µL of a dispersion (manufactured by Sigma-Aldrich Co. LLC., 0.02 mg/mL sodium citrate aqueous solution) of silver nanoparticles having a particle diameter of 20 nm, adding 23.9 g/L of an end thiolated PEG(O-[2-(3-mercaptopropionylamino) ethyl]-O'-methylpolyethylene glycol, average molecular weight 5,000, manufactured by Sigma-Aldrich Co. LLC.) aqueous solution (140 µL), then diluting the dispersion with super deionized water (490 µL), and then adding 100 mM Tris-HCl buffer (pH 8.3) (140 µL), 500 mM potassium chloride aqueous solution (140 µL), and 15 mM magnesium chloride aqueous solution (140 µL) to prepare a sample. The device used for the measurement was an ultraviolet-visible spectrophotometer V-650 manufactured by JASCO Corporation. As measurement conditions, data interval was set to 0.5 nm, bandwidth was set to 1.0 nm, response was set to medium, and scanning speed was set to 100 nm/min. The results are shown in Fig. 14.

Fig. 14 is a view showing that dispersibility is improved by surface PEG modification of silver nanoparticles. The blue spectrum in Fig. 14 is an absorption spectrum derived from a commercially available silver nanoparticle dispersion, and a maximum absorption was observed at 396 nm. When a tris-hydrochloric acid buffer (pH 8.3), which is often used for biochemical experiments, particularly for ligation with DNA ligase, and potassium chloride and magnesium chloride aqueous solutions as salts were added to this nanoparticle dispersion, absorption around 396 nm disappeared as shown in a red spectrum. This result indicates that the surfaces of the silver nanoparticles were coated with AgCl by adding a buffer or a salt, and the nanoparticles were bound to each other by van der Waal's interaction and precipitated as an aggregate. On the other hand, the surface PEG-modified silver nanoparticles also had absorption characteristics equivalent to those of commercially available silver nanoparticles as shown in a gray spectrum, but the surface PEG-modified silver nanoparticles showed strong absorption around 396 nm even when a buffer or a salt was added as shown in an orange spectrum. From this, it was found that in the surface PEG-modified silver nanoparticles, the surface of the nanoparticles was protected by the excluded volume effect of PEG, and high dispersibility was exhibited even in the presence of a buffer or a salt.

### (7) Evaluation of dispersibility of silver nanoparticles in presence of PEG 4000

The absorption spectrum of the silver nanoparticle dispersion in the presence of PEG 4000 (polyethylene glycol 4,000, average molecular weight 2700 to 3300, manufactured by FUJIFILM Wako Pure Chemical Corporation) was measured using a quartz cell (optical path length: 1 cm, optical path width: 1 cm) by measuring 350 µL of a dispersion of silver nanoparticles having a particle diameter of 20 nm (manufactured by Sigma-Aldrich Co. LLC., 0.02 mg/mL sodium citrate aqueous solution), diluting the dispersion with super deionized water (910 µL), and then adding 15.8 g/L of an aqueous PEG 4000 solution (140 µL) to prepare a sample. The absorption spectrum in the presence of buffer and salts was measured using a quartz cell (optical path length: 1 cm, optical path width: 1 cm) by measuring 350 µL of a dispersion (manufactured by Sigma-Aldrich Co. LLC., 0.02 mg/mL sodium citrate aqueous solution) of silver nanoparticles having a particle diameter of 20 nm, diluting the dispersion with super deionized water (490 µL), and then adding 15.8 g/L of an aqueous PEG 4000 solution (140 µL), 100 mM Tris-HCl buffer (pH 8.3) (140 µL), 500 mM potassium chloride solution (140 µL), and 15 mM magnesium chloride solution (140 µL) to prepare a sample. The device used for the measurement was an ultraviolet-visible spectrophotometer V-650 manufactured by JASCO Corporation. As measurement conditions, data interval was set to 0.5 nm, bandwidth was set to 1.0 nm, response was set to medium, and scanning speed was set to 100 nm/min. The results are shown in Fig. 15.

Fig. 15 is a diagram for proving that thiol groups of PEG for surface modification are important for interaction with silver nanoparticles, and dispersibility of silver nanoparticles is improved by surface PEG modification. In this experiment, as a comparison, the behavior of silver nanoparticles (particle diameter: 20 nm) in a dispersion was analyzed by absorption spectrum measurement in the presence of thiol-unmodified PEG 4000 (average molecular weight: 2700 to 3300, manufactured by FUJIFILM Wako Pure Chemical Corporation) that is considered not to directly interact with the silver nanoparticles. The blue spectrum in Fig. 15 indicates the absorption behavior of a dispersion obtained by adding 1.58 g/L of PEG 4000 to the silver nanoparticle dispersion. As with the commercially available silver nanoparticle dispersion itself, a spectrum showing maximum absorption at 396 nm was observed. However, when a tris hydrochloric acid buffer (pH 8.3) and an aqueous solution of potassium chloride and magnesium chloride as salts were added to this dispersion, absorption around 396 nm almost disappeared as shown in gray and red spectra, and therefore it was shown that in PEG 4000 in which surface modification of silver nanoparticles does not occur, a protective effect on the surfaces of silver nanoparticles was not obtained, and dispersibility improvement cannot be expected. From this, it is considered that surface modification with thiolated PEG is essential for improving the activity of silver nanoparticles and maintaining the activity.

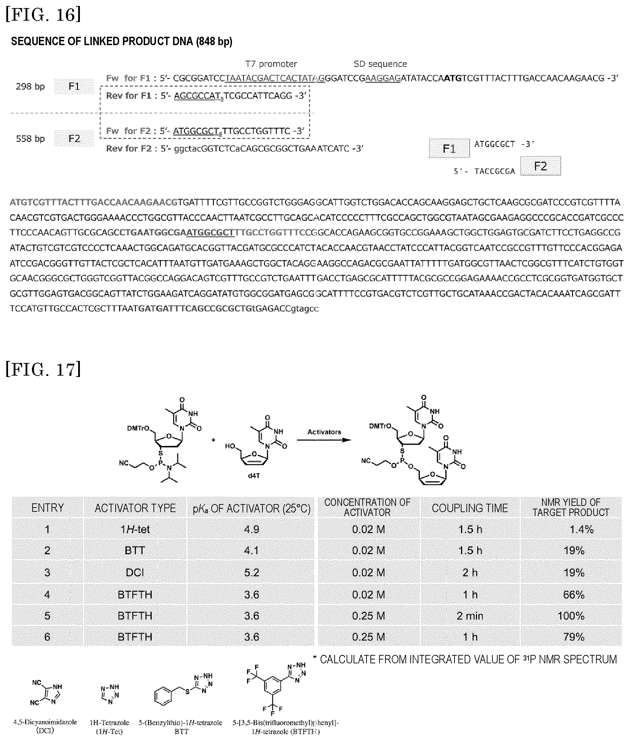

Fig. 16 is a view showing a sequence design of a DNA linking reaction using sticky end generation by DNA strand cleavage by silver nanoparticles. It is designed that the overall length of the linked product DNA is 848 base pairs, and by using two kinds of DNA primers Rev for F1:5'-AGCGCCATsTCGCCATTCAGG-3' and Fw for F1:5'-ATGGCGCTsTTGCCTGGTTTC-3', DNA fragments of 298 base pairs and 558 base pairs, respectively, are amplified by PCR, and the obtained PCR amplification product is treated with silver nanoparticles, and accordingly, a sticky end in which 8 bases protrude from the 5'-side can be prepared. The red underlined portion (ATGGCGCT) in the linked product sequence corresponds to the sequence of the 8-base protrusion part. DNA of 298 base pairs and 558 base pairs after PCR amplification is treated with silver nanoparticles, and then ligated with T4 DNA ligase, and accordingly, a linked product DNA having a total length of 848 base pairs can be synthesized. The two DNA primers Rev for F1:5'-AGCGCCATsTCGCCATTCAGG-3' and Fw for F1:5'-ATGGCGCTsTTGCCTGGTTTC-3' were synthesized by an automated nucleic acid synthesizer.

### (8) Coupling condition optimization in dimer model synthesis aimed at improvement of reaction conditions of 3'-thioated amidite.

Reaction tracking experiment: Compound 5T (100 mg, 0.13 mmol, 1.0 eq.), 3,5-Bis[3,5-bis(trifluoromethyl)phenyl]-1H-1,2,4-triazole(533 mg, 1.69 mmol, 13 eq.), and d4T (88 mg, 0.39 mmol, 3.0 eq.) were dissolved in acetonitrile (6.76 ml), and the mixture was stirred at normal temperature for 2 to 120 minutes. After reaction initiation and 2, 5, 10, 30, 45, 60, and 120 minutes, 650 µL of the reaction solution was sampled and transferred to an NMR sample tube, and phosphorus NMR was measured.

### Synthesis and isolation of dimer model compound

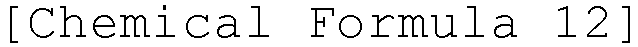

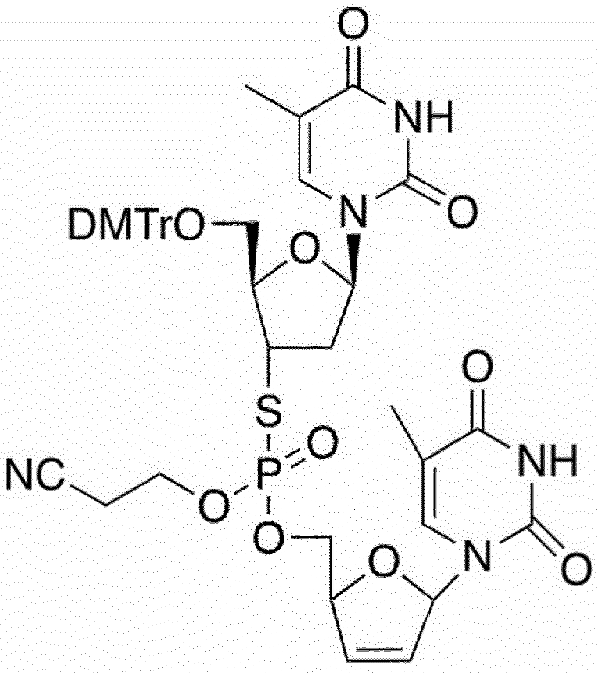

Compound 5T (100 mg, 0.13 mmol, 1.0 eq.), 3,5-Bis[3,5-bis(trifluoromethyl)phenyl]-1H-1, 2, 4-triazole(82 mg, 0.26 mmol, 2.0 eq.), and d4T(88 mg, 0.39 mmol, 3.0 eq.) were dissolved in acetonitrile (13 ml), and the solution was stirred at normal temperature for 1 hour, then tetrabutylammonium periodic acid (74 mg, 0.17 mmol, 1.3 eq.) was added, and the mixture was further stirred at normal temperature for 7 minutes. A saturated aqueous sodium thiosulfate solution (10 ml) was added, and the mixture was extracted twice with dichloromethane (20 ml). The reaction solution was concentrated by a rotary evaporator and purified by chromatography (neutral flash silica, methanol/dichloromethane = 1/7, 1% triethylamine) to obtain 50 mg (0.065 mmol, 50%) of a white solid.

¹H-NMR(600 MHz, CHLOROFORM-D)δ 9.15 - 9.29(m, 1H), 9.01(s, 0H), 6.98 - 7.65(m, 15H), 6.75 - 6.85(m, 4H), 6.19 - 6.31(m, 2H), 5.90 - 5.93(m, 1H), 4.95(d, J = 40.5 Hz, 1H), 4.01 - 4.36(m, 6H), 3.67 - 3.91(m, 6H), 3.48 - 3.66(m, 1H), 3.38 - 3.46(m, 1H), 2.55 - 2.80(m, 4H), 1.87 - 2.00(m, 3H), 1.78(d, J = 32.0 Hz, 3H), 1.34 - 1.56(m, 3H), 1.15 - 1.33(m, 1H) ppm.

³¹P-NMR(243 MHz, CHLOROFORM-D)27.509, 26.278 ppm.

### Synthesis and isolation of dimer model compound

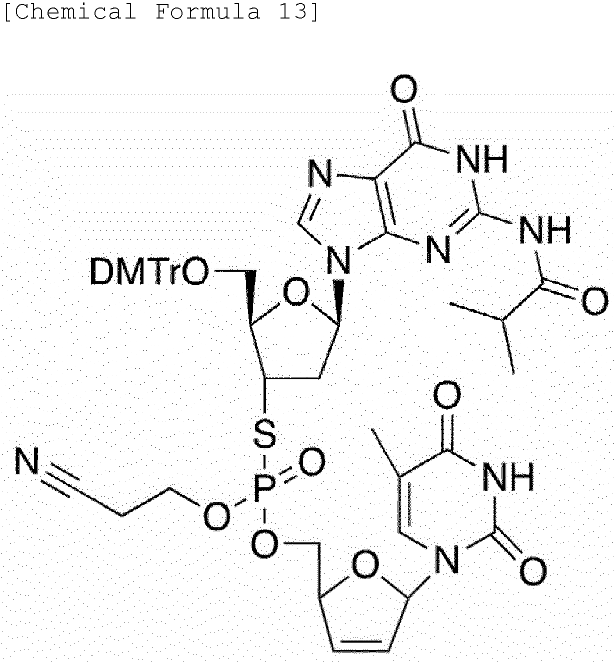

Compound 7G (30 mg, 0.035 mmol, 1.0 eq.), 3,5-Bis[3,5-bis (trifluoromethyl)phenyl]-1 H-1,2,4-triazole(14 mg, 0.50 mmol, 13 eq.), and d4T(24 mg, 0.12 mmol, 3.0 eq.) were dissolved in acetonitrile (2.0 ml), and the solution was stirred at normal temperature for 1 hour, then 0.01 MI₂(4.0 ml, 0.040 mmol, 1.1 eq.) was added, and further stirred at normal temperature for 7 minutes. A saturated aqueous sodium thiosulfate solution (10 ml) was added, and the mixture was extracted twice with dichloromethane (20 ml). The reaction solution was concentrated by a rotary evaporator and purified by chromatography (neutral flash silica, methanol/dichloromethane = 1/7, 1% triethylamine) to obtain 31 mg (0.032 mmol, 89%) of a white solid.

¹H-NMR(600 MHz, CHLOROFORM-D)δ 11.92(d, J = 23.1 Hz, 1H), 10.30 - 10.13(1H), 8.60 - 8.68(m, 2H), 7.60 - 7.72(m, 2H), 7.11 - 7.30(m, 15H), 6.92 - 6.98(m, 1H), 6.67 - 6.83(m, 4H), 6.31(dtd, J = 58.3, 3.8, 2.0 Hz, 1H), 5.94 - 6.04(m, 2H), 5.04 - 5.11(m, 1H), 4.80 - 4.93(m, 1H), 4.31 - 4.43(m, 2H), 4.19 - 4.24(m, 1H), 3.97 - 4.12(m, 2H), 3.75(d, J = 5.4 Hz, 6H), 3.26 - 3.40(m, 3H), 2.79 - 2.87(m, 1H), 2.49 - 2.68(m, 3H), 1.87(t, J = 1.5 Hz, 3H), 1.14 - 1.25(m, 7H) ppm.

³¹P-NMR(243 MHz, CHLOROFORM-D)29.983, 28.438 ppm.

As shown in Fig. 2(a), in the synthesis of a 19 base DNA (sequence: 5'-TAACTsCACATTAATTGCGTT-FAM-3') in which a 3'-thiation modification was introduced at the 4th base from the 5' end, an extension stop product having a length of 15 bases (approximately 50% from the HPLC peak area) was observed. The extension stop product appeared as two kinds of peaks in the vicinity of 13 to 14 minutes on the HPLC chart, and they were a mixture of 15 base-long 5' phosphorylated products generated by strand cleavage under iodine-pyridine oxidation conditions after uncoupling failed to react with 3'-thiated amidite and the 3'-thiated amidite. The generation of such by-products is considered to be a cause of a decrease in yield in oligomer synthesis. Therefore, an improvement condition of oligomer synthesis was searched by optimizing the reaction conditions in the dimer model synthesis.

Fig. 17 shows the results of examining the concentrations and coupling times of various activators and activators in the dimer model synthesis, and analyzing the generation yield of dimers of the target product. Examples of the activator include 1H-tetrazole (1H-tet), 5-benzylthio-1H-tetrazole (BTT), 4,5-dicyanoimidazole (DCI), and 5-[3,5-bis(trifluoromethyl)phenyl]-1H-tetrazole (BTFTH). The yield of the target product was calculated by comparing the integral ratio of the signal derived from 3'-thioamidite of the raw material (around 160 ppm), the signal derived from the target product (190 ppm), and the signal derived from the byproduct (dithiolate form: around 140 ppm) by phosphorus NMR analysis. As a result, while the yield of the target product was 1.4% when 1H-tet was used (entry 1), the yield was improved to 19% by using the BTT having a higher acidity (entry 2). When DCI, an activator with higher nucleophilicity, was used, no change in BTT and yield was observed (entry 3). Furthermore, use of BTFTH with high acidity improved the yield to 66% (entry 4). BTFTH was considered to be an optimal activator, and when the concentration of BTFTH was increased from 0.02 M to 0.25 M, the reaction proceeded almost quantitatively at a reaction time of 2 minutes (entry 5). However, when the reaction time was extended to 1 hour under the condition in which BTFTH was used at 0.25 M, the yield of the target product decreased, and generation of a dithiolate form as a by-product was confirmed (entry 6). From this result, it was shown that it is important to terminate the reaction in a short time using a high concentration of BTFTH in order to maximize the yield of the target product, and by increasing the reaction time, the target product is further reacted to be converted into a by-product, and the yield is reduced. After the coupling reaction of the 3'-thioamidite, oxidation of the phosphorus atom from trivalent to pentavalent was performed by adding 0.01 M iodine/pyridine/aqueous solution or tetrabutylammonium periodic acid as an oxidizing agent, and a dimer model having a 3' thiophosphoric acid bond was synthesized.

### (9) Influence of reaction time when 0.25 M BTFTH (13 eq) was used in dimer model synthesis

Reaction tracking experiment Compound 5T (100 mg, 0.13 mmol, 1.0 eq.), 3,5-Bis[3,5-bis(trifluoromethyl)phenyl]-1H-1,2,4-triazole(533 mg, 1.69 mmol, 13 eq.), and d4T (88 mg, 0.39 mmol, 3.0 eq.) were dissolved in acetonitrile (6.76 ml), and the mixture was stirred at normal temperature for 2 to 120 minutes. After reaction initiation and 2, 5, 10, 30, 45, 60, and 120 minutes, 650 µL of the reaction solution was sampled and transferred to an NMR sample tube, and phosphorus NMR was measured.

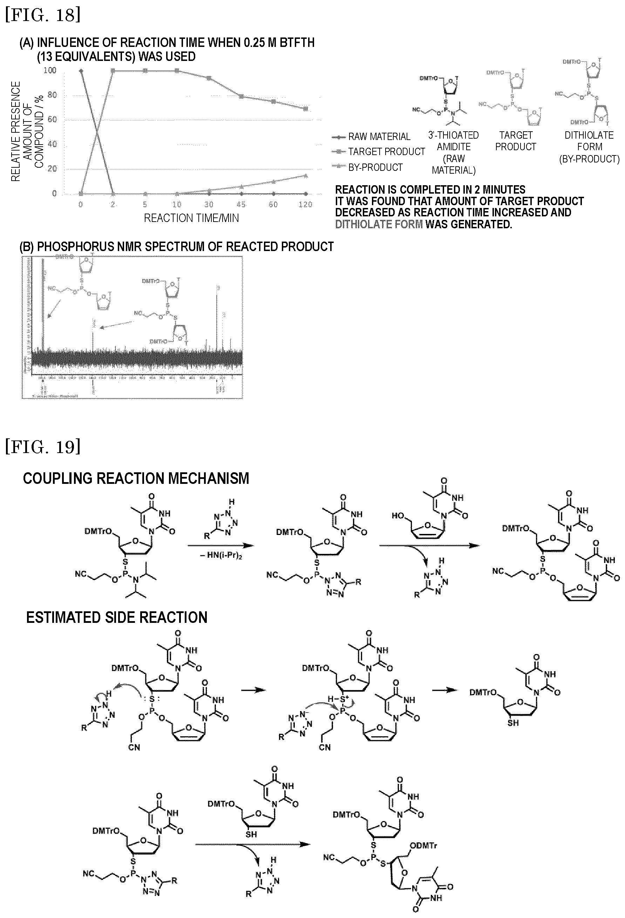

Fig. 18 is an experimental result showing the influence of the reaction time when 0.25 M BTFTH (13 equivalents) was used in dimer model synthesis. Fig. 18 (A) shows a result of plotting the relative abundance of the compound and the reaction time, and it was found that the desired reaction was completed in 2 minutes from the start of the reaction, and the target product was decomposed and converted into a dithiolate form by further extending the reaction time. Fig. 18(B) is a phosphorus NMR spectrum of the reaction product (2 hours after the start of the reaction). A signal of a target product was observed at around 190 ppm, and a signal derived from a dithiolate form as a by-product was observed at around 140 ppm.

Fig. 19 shows the mechanism of the side reactions estimated. After a dimer as a target product is formed, the dimer is further activated by an activator, and a dithiolate form is generated after strand cleavage.

### (10) Application to oligo DNA synthesis with reference to reaction conditions optimized in dimer synthesis

Oligodeoxyribonucleotide 5'-TAA Ts CATTAATTGCGTT-FAM-3' was synthesized by an automatic nucleic acid synthesizer NR-2 A7MX (manufactured by Nippon Techno Service Co., Ltd.) according to a conventional method, using Compound 5T and a commercially available phosphoramidite reagent (ChemGenes). Ts represents a 3'thiated thymidine base. Fluorescein (FAM) was introduced into the 3' end. The various amidite reagents were 50 mM acetonitrile solutions, and only Compound 5T was used as a 150 mM acetonitrile solution. BTFTH was used as an activator and 0.05 M iodine (pyridine-/water; v/v: 9/1) solution was used as an oxidizing agent. As the examination of the coupling conditions, 2 to 5 couplings were examined for 20 to 450 seconds using 0.25 M or 1.0 M BTFTH. In addition, a design was made in which FAM is introduced to the 3' end by using 6-FAM-glycerol support 500 Å (manufactured by ChemGenes) as a solid phase carrier. The synthesis was set to Final DMTr-ON and taken out while leaving the DMTr group on the 5'-end hydroxyl group. After completion of the synthesis, concentrated aqueous ammonia (500 µL) and a 40% aqueous methylamine solution (500 µL) were added to the solid phase carrier, and the solid phase carrier was cut out and deprotected by treatment at 65°C for 1 hour. The supernatant was filtered using a Millex LH filter (0.45 µm, manufactured by Merck Corporation), and then the filtrate was dried under reduced pressure by a centrifugal evaporator. The obtained oligonucleotide was redissolved in super deionized water, and the concentration was calculated by measuring the absorption at 260 nm. The product was analyzed by reverse phase HPLC to calculate the peak area ratio between the peak (retention time: 17.2 minutes) derived from the target product in a state where the DMTr group remained as the 5'-hydroxyl group protecting group by analyzing the product by reverse-phase HPLC and the peak (retention time: around 8.0 to 12.0 minutes) derived from the product of which synthesis was stopped and the product that was cleaved at the introduction stage of the 3' thioamidite 5T. HPLC analysis conditions are as follows.

Column: Hydrosphere C18 (250 x 10.0 mml.D., S-5 µm, 12 nm, HS12S05-2510WT, Ser. No. 131EA90023), eluent A: 50 mM triethylammonium acetate containing 5% acetonitrile (pH 7.0), eluent B: acetonitrile, gradient conditions: flow rate: 3 mL/min, column temperature: 50°C, detection wavelength: 260 nm.

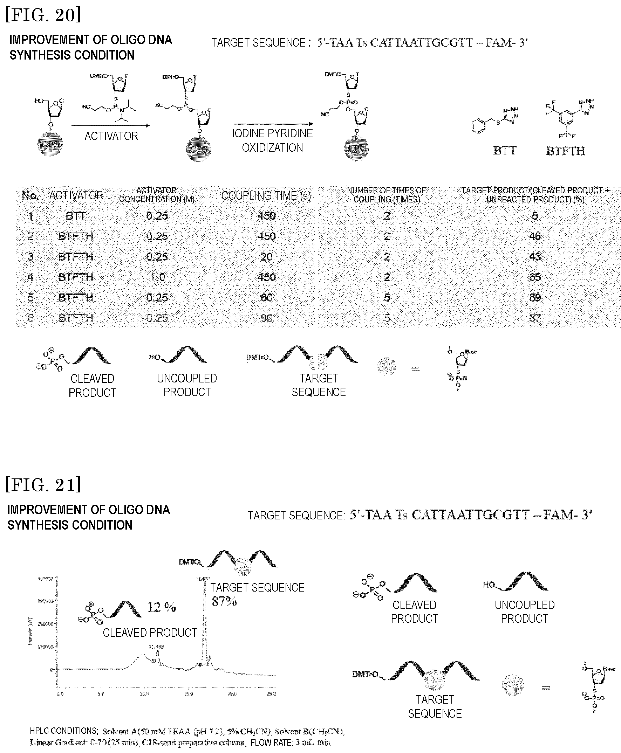

Fig. 20 shows the results of applying the reaction conditions optimized in dimer synthesis to oligo DNA synthesis and improving the conditions. As an activator, BTFTH that was found to be most excellent in dimer synthesis was used, and the concentration of the activator, the coupling time, and the number of couplings were examined to calculate the production ratio of the resulting 17 base-long oligo DNA (sequence: 5'-TAA Ts CATTAATTGCGTT-FAM-3') on the reverse phase HPLC peak. In the table of FIG. 20, the peak ratio between the target product and the strand cleave + unreacted product (13 base length: sequence: 5'-CATTAATTGCGTT-FAM-3') is shown. As the oxidizing agent, the most common iodine-pyridine solution was used. When coupling was performed twice in 450 seconds using 0.25 M BTT as a control for condition study, the target product ratio was 5% (entry 1). When coupling was performed twice in 450 seconds using 0.25 M BTFTH, the target product ratio was 46% (entry 2), and when the coupling time was reduced to 20 seconds, the target product ratio was almost unchanged to 43% (entry 3). Next, when the BTFTH concentration was increased to 1.0 M and coupling was examined twice in 450 seconds, the target product ratio was improved by approximately 20% (entry 4), and when the coupling time was changed to 60 seconds and the number of couplings was changed to 5 times, the target product ratio was slightly improved to 69% (entry 5). Subsequently, when coupling was performed 5 times in 90 seconds using 0.25 M BTFTH, the target product ratio was improved to 87% (entry 6). From these results, it was found that in the solid phase synthesis of the oligo DNA, the target product generation ratio can be maximized by repeating the coupling cycle a plurality of times in a short coupling time.

### (11) Synthesis of oligo DNAs (sequence: 5'-TAA Ts CATTAATTGCGTT-FAM-3') synthesized and studied by adopting optimized coupling conditions.

Oligodeoxyribonucleotide 5'-TAA Ts CATTAATTGCGTT-FAM-3' was synthesized by an automatic nucleic acid synthesizer NR-2 A7MX (manufactured by Nippon Techno Service Co., Ltd.) according to a conventional method, using Compound 5T and a commercially available phosphoramidite reagent (ChemGenes). Ts represents a 3'thiated thymidine base. Fluorescein (FAM) was introduced into the 3' end. The various amidite reagents were 50 mM acetonitrile solutions, and only Compound 5T was used as a 150 mM acetonitrile solution. 0.25 M BTFTH (5 couplings in 90 s) as an activator, 0.05 M iodine (pyridine-/water; v/v: 9/1) solution was used as an oxidizing agent. In addition, a design was made in which FAM is introduced to the 3' end by using 6-FAM-glycerol support 500 Å (manufactured by ChemGenes) as a solid phase carrier. The synthesis was set to Final DMTr-ON and taken out while leaving the DMTr group on the 5'-end hydroxyl group. After completion of the synthesis, concentrated aqueous ammonia (500 µL) and a 40% aqueous methylamine solution (500 µL) were added to the solid phase carrier, and the solid phase carrier was cut out and deprotected by treatment at 65°C for 1 hour. The supernatant was filtered using a Millex LH filter (0.45 µm, manufactured by Merck Corporation), and then the filtrate was dried under reduced pressure by a centrifugal evaporator. The obtained oligonucleotide was redissolved in super deionized water, and the concentration was calculated by measuring the absorption at 260 nm. The 5'-DMTr protected DNA was fractionated by reverse phase HPLC, then a 10% acetic acid aqueous solution was added thereto, and the resultant mixture was treated at room temperature for 1 hour to deprotect the DMTr group, thereby obtaining a target oligo DNA. HPLC analysis conditions are as follows.

Column: Hydrosphere C18 (250 x 10.0 mml. D., S-5 µm, 12 nm, HS12 S05-2510WT, Ser. No. 131EA90023), eluent A: 50 mM triethylammonium acetate containing 5% acetonitrile (pH 7.0), eluent B: acetonitrile, gradient conditions: flow rate: 3 mL/min, column temperature: 50°C, detection wavelength: 260 nm.

Fig. 21 shows the synthesis results of the oligo DNA (sequence: 5'-TAA Ts CATTAATTGCGTT-FAM-3') synthesized and studied by adopting the coupling conditions (entry 6) optimized in the experiment of Fig. 20. From the result of reverse phase HPLC analysis of Fig. 21, the target product having a length of 17 bases and containing a 3'-thiophosphate bond was obtained at a ratio of 87% from the peak area ratio compared to the peak derived from the byproduct (strand cleaved product) (retention time: 16.9 minutes). The target product peak having a retention time of 16.9 minutes was fractionated and purified, then a 10% acetic acid aqueous solution was added, and the DMTr group was deprotected by treatment at room temperature for 1 hour to obtain a target DNA (5'-TAA Ts CATTAATTGCGTT-FAM-3).

### (12) Synthesis of 51 base-long oligo DNA (5'-ATGAAACGCCGAGTTsAACGCCATCAAAAATsAATTCGCGTCTGGCCTTCCTsG-3') into which 3'thiophosphate bonds are introduced at 3 locations

Oligodeoxyribonucleotide 5'-ATGAAACGCCGAGTTsAACGCCATCAAAAATsAATTCGCGTCTGGCCTTCCTsG-3' was synthesized by an automatic nucleic acid synthesizer NR-2A 7MX (manufactured by Nippon Techno Service Co., Ltd.) according to a conventional method, using Compound 5T and a commercially available phosphoramidite reagent (ChemGenes). Ts represents a 3'thiated thymidine base. The various amidite reagents were 50 mM acetonitrile solutions, and only Compound 5T was used as a 150 mM acetonitrile solution. 0.25 M BTFTH (5 couplings in 90 s) as an activator, 0.05 M iodine (pyridine-/water; v/v: 9/1) solution was used as an oxidizing agent. The synthesis was set to Final DMTr-ON and taken out while leaving the DMTr group on the 5'-end hydroxyl group. After completion of the synthesis, concentrated aqueous ammonia (500 µL) and a 40% aqueous methylamine solution (500 µL) were added to the solid phase carrier, and the solid phase carrier was cut out and deprotected by treatment at 65°C for 1 hour. The supernatant was filtered using a Millex LH filter (0.45 µm, manufactured by Merck Corporation), and then the filtrate was dried under reduced pressure by a centrifugal evaporator. The obtained oligonucleotide was redissolved in super deionized water, and the concentration was calculated by measuring the absorption at 260 nm. The 5'-DMTr protected DNA was fractionated by reverse phase HPLC, then a 10% acetic acid aqueous solution was added thereto, and the resultant mixture was treated at room temperature for 1 hour to deprotect the DMTr group, thereby obtaining a target oligo DNA. HPLC analysis conditions are as follows.

Column: Hydrosphere C18 (250 x 10.0 mml. D., S-5 µm, 12 nm, HS12S05-2510WT, Ser. No. 131EA90023), eluent A: 50 mM triethylammonium acetate containing 5% acetonitrile (pH 7.0), eluent B: acetonitrile, gradient conditions: flow rate: 3 mL/min, column temperature: 50°C, detection wavelength: 260 nm.

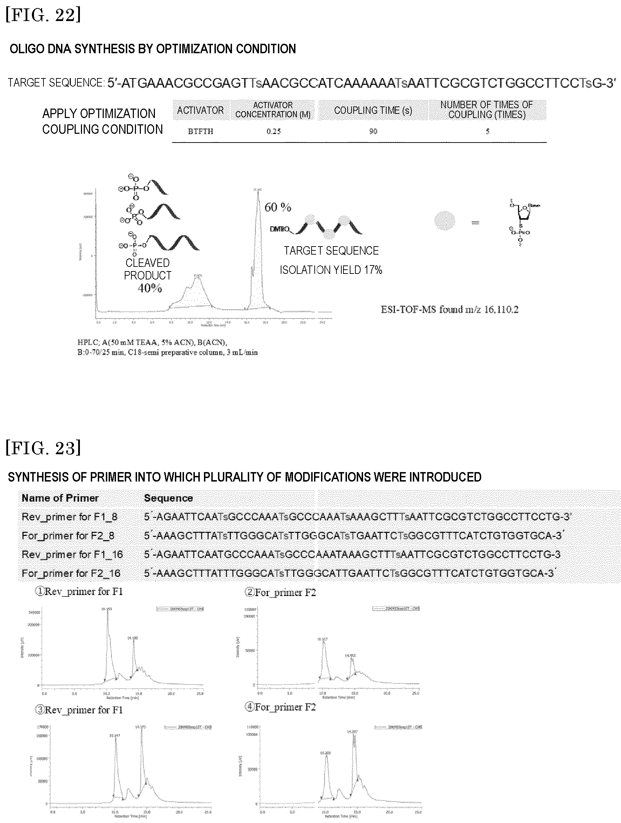

Fig. 22 shows the result of synthesizing 51 base-long oligo DNA (5'-ATGAAACGCCGAGTTsAACGCCATCAAAAATsAATTCGCGTCTGGCCTTCCTsG-3') into which 3'thiophosphate bonds are introduced at three locations, and analyzing reverse phase HPLC. Ts represents a 3'thiated thymidine base. As a result of reverse phase HPLC, the main peak observed around the retention time of 17.2 minutes is 51 mer target DNA, and a DMTr group remains as a 5'-hydroxyl group protecting group. On the other hand, a product of which synthesis was stopped at the introduction stage of Compound 5T and a cleaved product were also partially observed around the retention time of 8.0 to 12.0 minutes, but could be easily separated from the DNA of the target product of 51 mer. A peak around the retention time of 17.2 minutes was fractionated to obtain a 5'-DMTr protected product of the target DNA. ESI-TOF-MS analysis of the 5'-DMTr protected product fractionated and purified by HPLC confirmed that it was a peak at m/z 16, 110.2 derived from the target product. A 10% aqueous acetic acid solution was added, and the mixture was treated at room temperature for 1 hour to deprotect the DMTr group, thereby obtaining a target DNA.

### (13) Sequence of PCR primer DNA having 3'thiophosphate bonds introduced at 4 locations and 2 locations and synthesis thereof

Oligodeoxyribonucleotide was synthesized by an automatic nucleic acid synthesizer NR-2A 7MX (manufactured by Nippon Techno Service Co., Ltd.) according to a conventional method, using Compound 5T and a commercially available phosphoramidite reagent (ChemGenes). Ts represents a 3'thiated thymidine base. The various amidite reagents were 50 mM acetonitrile solutions, and only Compound 5T was used as a 150 mM acetonitrile solution. 0.25 M BTFTH (5 couplings in 90 s) as an activator, 0.05 M iodine (pyridine-/water; v/v: 9/1) solution was used as an oxidizing agent. The synthesis was set to Final DMTr-ON and taken out while leaving the DMTr group on the 5'-end hydroxyl group. After completion of the synthesis, concentrated aqueous ammonia (500 µL) and a 40% aqueous methylamine solution (500 µL) were added to the solid phase carrier, and the solid phase carrier was cut out and deprotected by treatment at 65°C for 1 hour. The supernatant was filtered using a Millex LH filter (0.45 um, manufactured by Merck Corporation), and then the filtrate was dried under reduced pressure by a centrifugal evaporator. The obtained oligonucleotide was redissolved in super deionized water, and the concentration was calculated by measuring the absorption at 260 nm. The 5'-DMTr protected DNA was fractionated by reverse phase HPLC, then a 10% acetic acid aqueous solution was added thereto, and the resultant mixture was treated at room temperature for 1 hour to deprotect the DMTr group, thereby obtaining a target oligo DNA. HPLC analysis conditions are as follows.

Column: Hydrosphere C18 (250 x 10.0 mml. D., S-5 µm, 12 nm, HS12 S05-2510WT, Ser. No. 131EA90023), eluent A: 50 mM triethylammonium acetate containing 5% acetonitrile (pH 7.0), eluent B: acetonitrile, gradient conditions: flow rate: 3 mL/min, column temperature: 50°C, detection wavelength: 260 nm.

Fig. 23 shows sequences of four kinds of PCR primer DNAs in which 3'thiophosphate bonds are introduced at a plurality of positions, and synthesis results thereof. The synthesized primers are Rev_primer for F1_8 and Rev_primer for F2_8, which are cleaved at 4 locations by silver nanoparticle cleavage after PCR amplification to generate a sticky end of 34 bases, and Rev_primer for F1_16 and Rev_primer for F2_16, which are cleaved at 2 locations by silver nanoparticle cleavage after PCR amplification to generate a sticky end of 34 bases. The synthesized four kinds of oligo DNAs were analyzed by reverse phase HPLC. Each peak (around retention time of 15 minutes) of the target length oligo-DNA in a state where the DMTr group remained as the 5'-hydroxyl group protecting group was fractionated and purified, then a 10% acetic acid aqueous solution was added thereto, and the resultant mixture was treated at room temperature for 1 hour to deprotect the DMTr group, thereby obtaining a target DNA.

### (14) Synthesis of 3'thiophosphorylated oligo-DNA (5'-TAACAs CACATTAATTGCGTT-FAM-3') synthesized using a 3'-thiolated amidite adenosine derivative.

Oligodeoxyribonucleotide 5'-TAACAsCACATTAATTGCGTT-FAM-3 was synthesized by an automatic nucleic acid synthesizer NR-2A 7MX (manufactured by Nippon Techno Service Co., Ltd.) according to a conventional method, using Compound 6A and a commercially available phosphoramidite reagent (ChemGenes). As represents a 3'thiated adenosine base. The various amidite reagents were 50 mM acetonitrile solutions, and only Compound 5T was used as a 150 mM acetonitrile solution. 0.25 M BTFTH (2 couplings in 450 s) as an activator, 0.05 M iodine (pyridine-/water; v/v: 9/1) solution was used as an oxidizing agent. The synthesis was set to Final DMTr-ON and taken out while leaving the DMTr group on the 5'-end hydroxyl group. After completion of the synthesis, concentrated aqueous ammonia (500 µL) and a 40% aqueous methylamine solution (500 µL) were added to the solid phase carrier, and the solid phase carrier was cut out and deprotected by treatment at 65°C for 1 hour. The supernatant was filtered using a Millex LH filter (0.45 µm, manufactured by Merck Corporation), and then the filtrate was dried under reduced pressure by a centrifugal evaporator. The obtained oligonucleotide was redissolved in super deionized water, and the concentration was calculated by measuring the absorption at 260 nm. The 5'-DMTr protected DNA was fractionated by reverse phase HPLC, then a 10% acetic acid aqueous solution was added thereto, and the resultant mixture was treated at room temperature for 1 hour to deprotect the DMTr group, thereby obtaining a target oligo DNA. HPLC analysis conditions are as follows.

Column: Hydrosphere C18 (250 x 10.0 mml. D., S-5 µm, 12 nm, HS12S05-2510WT, Ser. No. 131EA90023), eluent A: 50 mM triethylammonium acetate containing 5% acetonitrile (pH 7.0), eluent B: acetonitrile, gradient conditions: flow rate: 3 mL/min, column temperature: 50°C, detection wavelength: 260 nm.

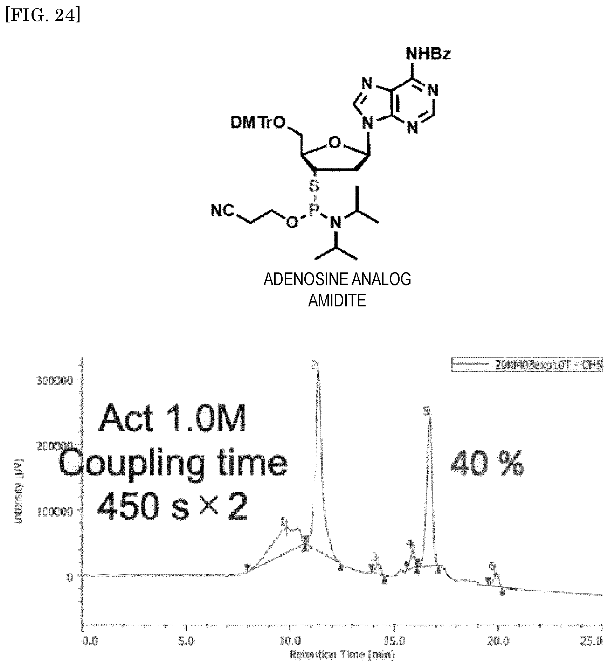

Fig. 24 shows the results of synthesis of 3'-thiophosphorylated oligo-DNA containing an adenosine derivative (sequence: 5'-TAACAs CACATTAATTGCGTT-FAM-3') using a 3'-thiolated amidite adenosine derivative (Compound 6A). Oligo DNA synthesized using BTFTH as an activator was analyzed by reverse phase HPLC. As a result, from the reverse phase HPLC peak area ratio, the ratio of the 5'-DMTr protection object oligo-DNA around the retention time of 16.8 minutes was 40%, and it was clearly separable from the uncoupled product and the strand-cleaved product (around the retention time of 11.5 minutes). After the peak of the target length oligo DNA was fractionated and purified, a 10% acetic acid aqueous solution was added thereto, and the DMTr group was deprotected by treatment at room temperature for 1 hour to obtain a target DNA.

### (15) Preparation of a 3'-overhang sticky end by cleavage of 3'-thiophosphate binding DNA containing an adenosine analog by silver nanoparticles.

PS-modified DNA (5'-TAACAsCACATTAATTGCGTT-FAM-3') and complementary strand DNA (5'-AACGCAATTAATGTGTGTTA-3') were mixed to be 3 µM each, treated at 95°C for 3 minutes, and then annealed by cooling on an ice bath for 10 minutes or more. 9 µL of the solution after annealing was weighed, and 51 µL of a surface PEG-modified silver nanoparticle dispersion (10 nm) was added, and the mixture was incubated at 50°C for a predetermined time. The surface PEG-modified silver nanoparticle dispersion was prepared by adding 1 µL of an end thiol PEG aqueous solution (23.9 g/L) to 50 µL of a commercially available silver nanoparticle dispersion (manufactured by Sigma-Aldrich Co. LLC., 10 nm, 0.02 mg/mL). 30 µL of the reaction solution was weighed, and 30 µL of 2x loading buffer was added thereto. After heat treatment at 95°C for 5 minutes, analysis was performed by 15% denaturing acrylamide electrophoresis (containing 7.5 M urea, 10 x 12 cm, 30 mA, 20 min, 6 µL application). A gel electrophoresis image was acquired by FAM-derived fluorescence detection using a gel image analyzer (manufactured by BioRad Laboratories, Inc.).

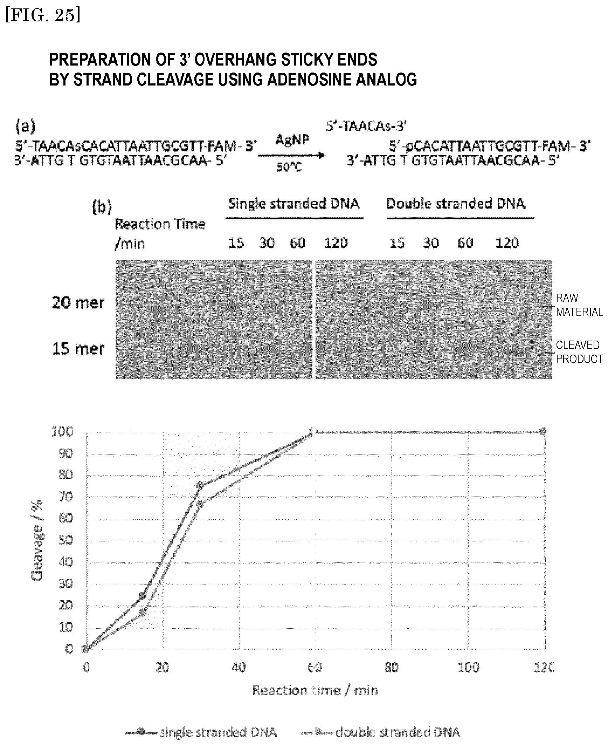

Fig. 25 shows the results of preparing 3' overhang sticky ends by silver nanoparticle treatment of double-stranded short-strand DNA. Here, cleavage of the 3'-thioadenosine analog synthesized in the experiment shown in FIG. 24 was performed using 1 nm surface PEG-modified silver nanoparticles. The SP bond is positioned between the 5th base and the 6th base from the 5' end, and was designed such that a sticky end protruding by 5 bases can be prepared when cleavage occurs. Reactions were performed at 50°C for 15, 30, 60, and 120 minutes, and each reaction solution was analyzed by denaturing gel electrophoresis. Cleavage efficiency at each reaction time was calculated from band intensity analysis of the gel by FAM-derived fluorescence detection. The cleavage efficiency was compared between the presence and absence of the complementary strand, and the cleavage efficiency at each reaction time was plotted on the vertical axis, and the reaction time was plotted on the horizontal axis. As a result, in the case of the presence of the complementary strand, the cleavage activity was slightly reduced as compared with the case of the absence of the complementary strand, but the cleavage activity was almost the same 2 hours after the start of the reaction, and 90% or more of the DNA was cleaved. Furthermore, since this cleavage activity was almost the same as the cleavage experiment result of the thymidine analog shown in Fig. 5, it was shown that the cleavage activity was not affected even when the base species at the modification site was changed.

### (16) Sticky end preparation and linking reaction using plurality of modified introduction primer

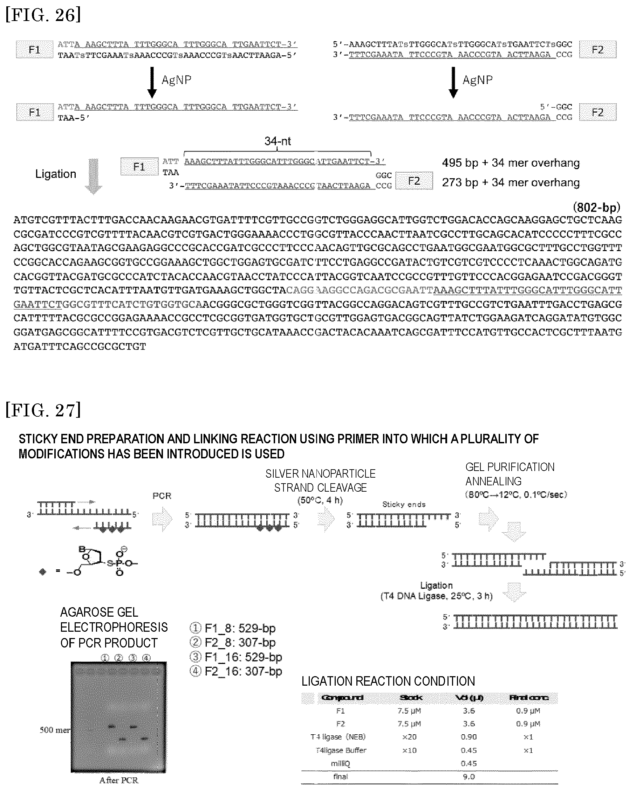

Fig. 26 is a schematic diagram of an experimental system showing sticky end preparation of 34 bases in length by cleaving of silver nanoparticles of a PCR amplification product using the 4 kinds of primer DNAs synthesized in Fig. 23 and the sequence of a ligation product of these cleaved products.

### (a) PCR with four kinds of primers

20 µM thiated forward primer (1.25 µL), 20 µM thiated reverse primer (1.25 µL), 10 ng/µL template DNA (2.5 µL), 2 mM dNTP mixture (5 µL), 25 mM magnesium sulfate (3 µL), 10 x PCR buffer for KOD-Plus-Neo (5 µL), and super deionized water (31 µL) were mixed, 1 U/µL KOD-Plus-Neo (1 µL) was added, and then the mixture was treated at 95°C for 2 minutes using a thermal cycler (manufactured by BioRad Laboratories, Inc.). Subsequently, DNA amplification was performed by performing 30 cycles of denaturation (95°C, 15 seconds)-annealing (55°C, 15 seconds)-strand extension (68°C, 30 seconds). The PCR product was purified using a wizard column (manufactured by Promega Corporation) according to the manufacturer's recommended protocol to obtain thiated DNA.

### (b) Sticky end preparation by cleaving with silver nanoparticles

7.5 nM PCR product thiated DNA (225 fmol/sample) was dissolved in super deionized water to prepare 4.5 µL of an aqueous solution. PEG-modified silver nanoparticles were prepared by mixing 10 nm silver nanoparticles (manufactured by Sigma-Aldrich Co. LLC., 200 µg/mL citric acid buffer suspension, 25 µL) with end thiol-modified PEG (manufactured by Sigma-Aldrich Co. LLC., 23.9 g/L aqueous solution, 0.5 µL). The thionized DNA aqueous solution (4.5 µL) of the prepared PCR product and the PEG-modified silver nanoparticle dispersion (25.5 µL) were mixed, and then the mixture was treated at 50°C for 4 hours to cleave the SP bond, thereby preparing a sticky end.

Fig. 27 shows the flow of an experiment of preparation the 34 sticky end of a PCR amplification product by cleaving the PCR amplification product using the 4 kinds of primer DNAs synthesized in Fig. 23 by treatment with silver nanoparticles, and ligation of these cleaved products, and the results of agarose electrophoresis of the PCR product DNA. It was confirmed that PCR products of 4 desired lengths of 529 bp, 307 bp, 529 bp, and 307 bp were obtained.

### (17) Compare linking efficiency with the linking between 34 base-long sticky end DNAs obtained by cleaving PCR amplification products at a plurality of locations.

7.5 nM silver nanoparticle-treated product DNA_1 (3.6 µL), 7.5 nM silver nanoparticle-treated product DNA_2 (3.6 µL), 10 x T4 DNA Ligase Reaction Buffer (manufactured by New England BioLabs, 0.90 µL), and super deionized water (0.45 µL) were mixed, then T4 DNA ligase (manufactured by New England BioLabs, 2000 U/µL, 0.45 µL) was added, and the mixture was incubated at 25°C for 3 hours. 1 µL of 10 x loading buffer was added to the reaction solution (9 µL) and analyzed by 1% agarose gel electrophoresis (electrophoretic buffer: 1 x TBE, 100 V, 30 min). The gel was stained by shaking with 10,000 x SYBR Green I solution for 30 minutes, and a gel electrophoresis image was obtained with a gel image analyzer (manufactured by BioRad Laboratories, Inc.).

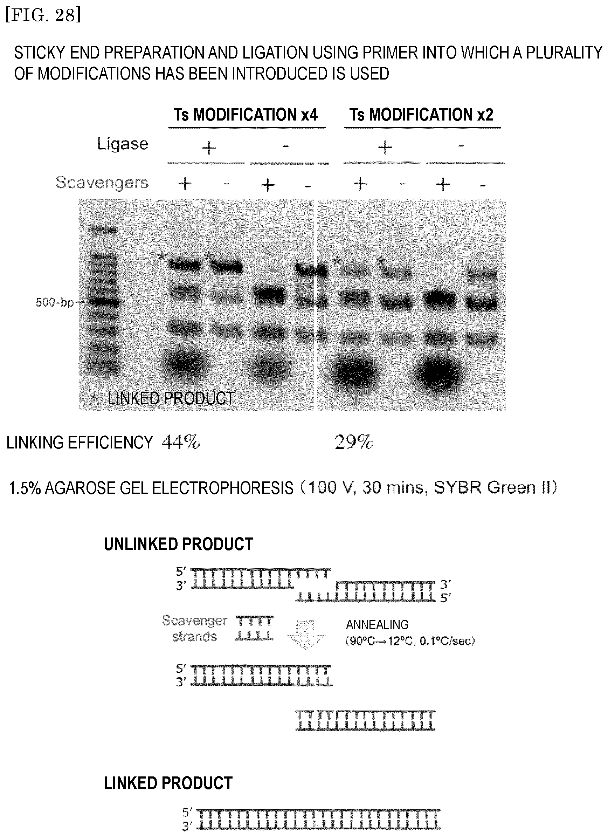

Fig. 28 shows a result of comparing the linking efficiency between 34 base-long sticky end DNAs obtained by cleaving a product PCR-amplified using Rev_primer for F1_8 and Rev_primer for F2_8 with silver nanoparticles and between 34 base-long sticky end DNAs obtained by cleaving a product PCR-amplified using Rev_primer for F1_16 and Rev_primer for F2_16 with silver nanoparticles. The product PCR-amplified using Rev_primer for F1_8 and Rev_primer for F2_8 is designed to have a thiophosphate modification introduced every 8 bases, whereas the product PCR-amplified using Rev_primer for F1_16 and Rev_primer for F2_16 is designed to have a thiophosphate modification introduced every 16 bases. Therefore, by comparing these coupling efficiencies, the influence of the linking efficiency of the cleaved fragment DNA on ligation after cleavage by the silver nanoparticles becomes clear. That is, it is expected that a DNA fragment having 8 bases after cleavage of the silver nanoparticle is dissociated quickly after cleavage and exhibits relatively high ligation efficiency, whereas a DNA fragment having 16 bases after cleavage of the silver nanoparticle is dissociated relatively slowly after cleavage and exhibits low ligation efficiency. As a result of the experiment, in the system in which the DNA fragment is cleaved at two locations (Ts-modified x 2), the coupling efficiency was 29%, and in the system in which the DNA fragment is cleaved at four locations (Ts-modified x 4), the coupling efficiency was 44%, and it was shown that the dissociation of the DNA fragment after cleavage was promoted by cleaving at a plurality of positions, and high coupling efficiency was exhibited. The scavenger strand in the drawing has a function of peeling off a product in which sticky ends form complementary strands but are not linked, and was used to clearly distinguish a linked product from an unlinked product in gel analysis.

## Claims

1. A nucleic acid strand cleaving method comprising:
a nucleic acid preparation step of preparing a nucleic acid to be cleaved having a structure represented by the following Formula (1); and
a cleaving step of reacting the nucleic acid to be cleaved with a cleaving agent to cleave the nucleic acid to be cleaved at the part X of the Formula (1) to generate a nucleic acid having a structure represented by the following Formula (2),
the cleaving agent is a metal nanoparticle containing an atom selected from the group consisting of silver, mercury, and cadmium, 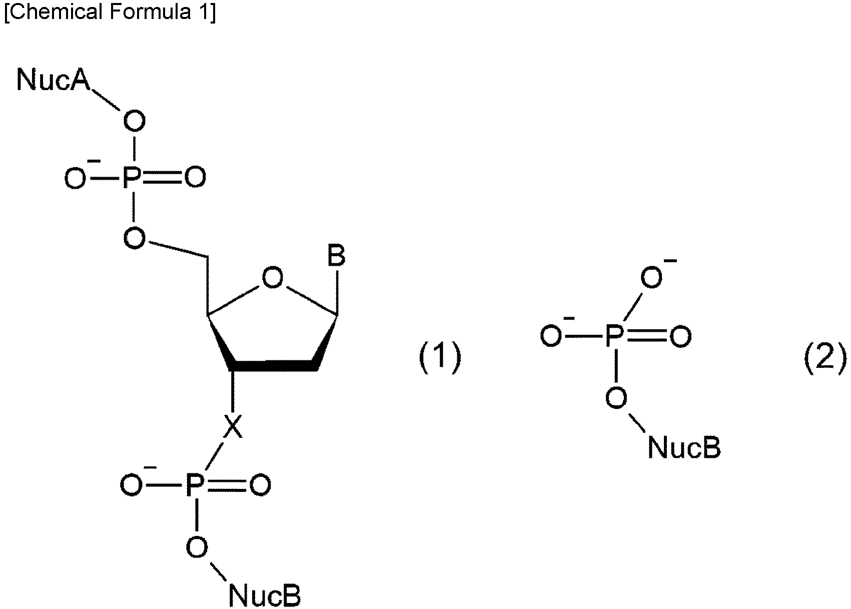
where B represents a base, X represents sulfur or selenium, NucA is composed of at least one nucleotide, is a part of the nucleic acid to be cleaved, and represents a part on the 5' end side with reference to the X, and NucB is composed of at least one nucleotide, is a part of the nucleic acid to be cleaved, and represents a part on the 3' end side with reference to the X.

2. The nucleic acid strand cleaving method according to claim 1, wherein the cleaving agent is silver nanoparticles, and the X is sulfur.

3. The nucleic acid strand cleaving method according to claim 1, wherein the metal nanoparticles have an average particle size within a range of 1 to 20 nm.

4. The nucleic acid strand cleaving method according to claim 1, wherein polyethylene glycol is bound to the surface of the metal nanoparticle.

5. The nucleic acid strand cleaving method according to claim 1, wherein in the nucleic acid preparation step, a part or all of the nucleic acid to be cleaved is synthesized by a phosphoramidite method using an amidite reagent represented by the following Formula (3) and Formula (4), 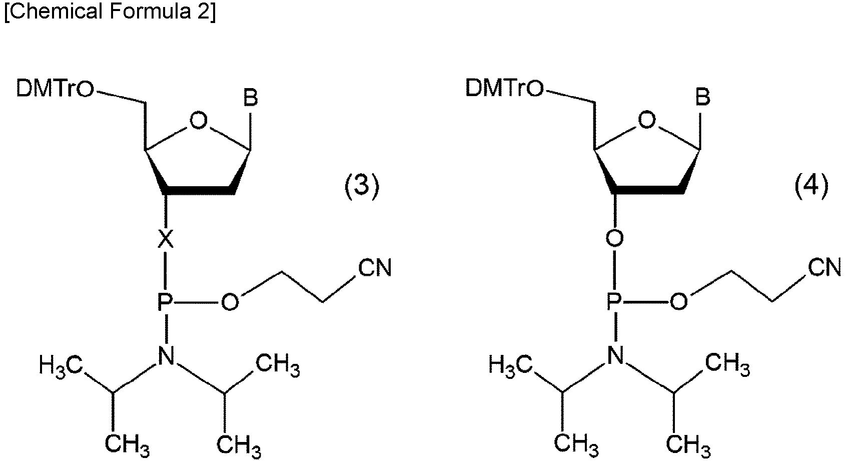 where B represents a base, X represents sulfur or selenium, and DMTr represents a dimethoxytrityl group.

6. The nucleic acid strand cleaving method according to claim 5, wherein the nucleic acid preparation step includes synthesizing 5-[3,5-bis (trifluoromethyl)phenyl]-1 H-tetrazole as an activator by a phosphoramidite method.

7. The nucleic acid strand cleaving method according to claim 5, wherein in the nucleic acid preparation step, a part of the nucleic acid to be cleaved is synthesized by a phosphoramidite method to form a primer, a plurality of cycles of polymerase chain reaction is performed using a template DNA having a sequence complementary to the nucleic acid to be cleaved as a template, and the primer is extended along the template DNA to generate the nucleic acid to be cleaved.

8. A nucleic acid strand cleaving device comprising:
nucleic acid preparation means configured to prepare a nucleic acid to be cleaved having a structure represented by the following Formula (1); and
cleaving means configured to react the nucleic acid to be cleaved with a cleaving agent to cleave the nucleic acid to be cleaved at the part X of the Formula (1) to generate a nucleic acid having a structure represented by the following Formula (2),
wherein the cleaving agent is a metal nanoparticle containing an atom selected from the group consisting of silver, mercury, and cadmium, 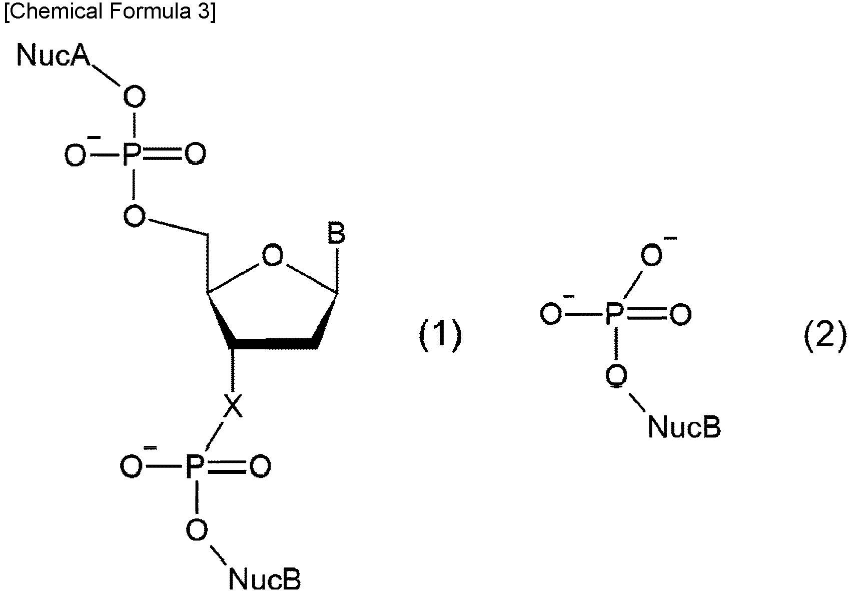
where B represents a base, X represents sulfur or selenium, NucA is composed of at least one nucleotide, is a part of the nucleic acid to be cleaved, and represents a part on the 5' end side with reference to the X, and NucB is composed of at least one nucleotide, is a part of the nucleic acid to be cleaved, and represents a part on the 3' end side with reference to the X.

9. The nucleic acid strand cleaving device according to claim 8, wherein the nucleic acid preparation means is configured to synthesize a part or all of the nucleic acid to be cleaved by a phosphoramidite method using an amidite reagent represented by the following Formula (3) and Formula (4), 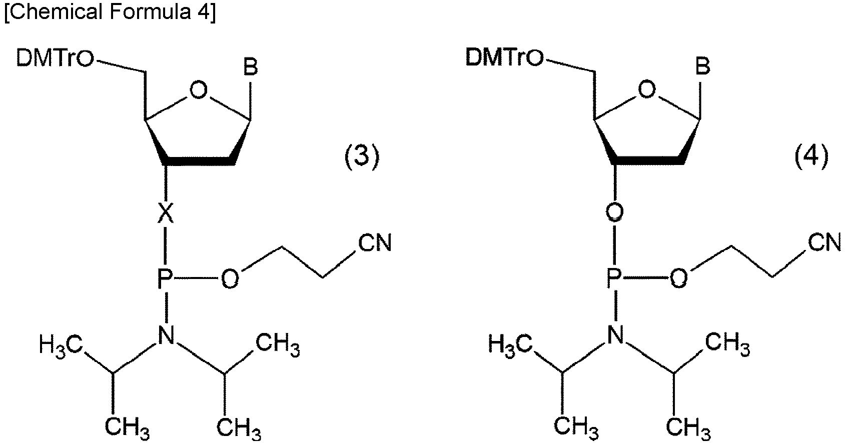 where B represents a base, X represents sulfur or selenium, and DMTr represents a dimethoxytrityl group.

10. The nucleic acid strand cleaving device according to claim 9, wherein the nucleic acid preparation means is configured to synthesize 5-[3,5-bis (trifluoromethyl)phenyl]-1 H-tetrazole as an activator by a phosphoramidite method.

11. A method for producing double-stranded DNA for producing double-stranded DNA having a sticky end, the method including:
a double-stranded DNA preparation step of preparing double-stranded DNA to be cleaved including a sense strand and an antisense strand having a sequence complementary to the sense strand, the double-stranded DNA to be cleaved having a structure represented by the following Formula (1) in at least one of the sense strand and the antisense strand; and
a sticky end generation step of reacting the double-stranded DNA to be cleaved with a cleaving agent to cleave the sense strand and/or the antisense strand at a portion of X in the Formula (1) to generate double-stranded DNA having a structure represented by the following Formula (2) and having a sticky end,
the cleaving agent is a metal nanoparticle containing an atom selected from the group consisting of silver, mercury, and cadmium, 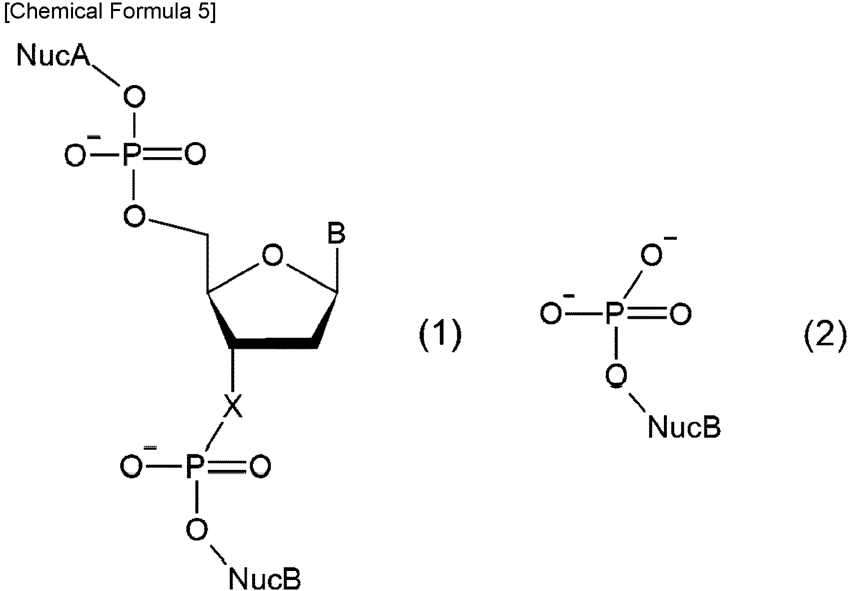
where B represents a base, X represents sulfur or selenium, NucA is composed of at least one nucleotide, is a part of the nucleic acid to be cleaved, and represents a portion on the 5' end side with reference to the X, and NucB is composed of at least one nucleotide, is a part of the nucleic acid to be cleaved, and represents a portion on the 3' end side with reference to the X.

12. The double-stranded DNA production method according to claim 11, wherein the double-stranded DNA to be cleaved has a plurality of structures represented by the Formula (1), and the number of nucleotides between the structures is 10 or less.

13. A double-stranded DNA production device for producing double-stranded DNA having a sticky end, the device including double-stranded DNA preparation means configured to prepare double-stranded DNA to be cleaved including a sense strand and an antisense strand having a sequence complementary to the sense strand, the double-stranded DNA to be cleaved having a structure represented by the following Formula (1) in at least one of the sense strand and the antisense strand, and a sticky end generation means that reacts the double-stranded DNA to be cleaved with a cleaving agent to cleave the sense strand and/or the antisense strand at a portion of X in the Formula (1) to generate double-stranded DNA having a structure represented by the following Formula (2) and having a sticky end, in which
the cleaving agent is a metal nanoparticle containing an atom selected from the group consisting of silver, mercury, and cadmium, 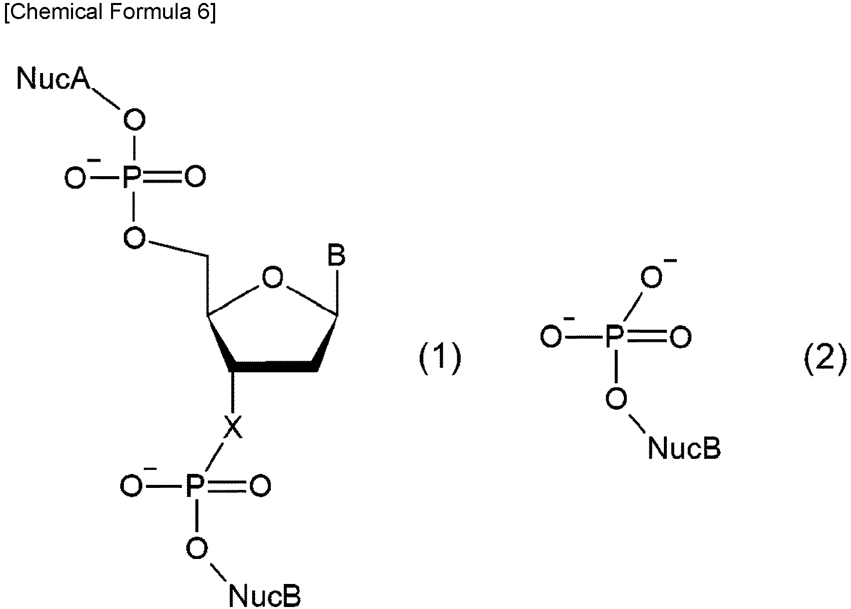 where B represents a base, X represents sulfur or selenium, NucA is composed of at least one nucleotide, is a part of the nucleic acid to be cleaved, and represents a portion on the 5' end side with reference to the X, and NucB is composed of at least one nucleotide, is a part of the nucleic acid to be cleaved, and represents a portion on the 3' end side with reference to the X.

14. The double-stranded DNA production device according to claim 13, wherein the double-stranded DNA to be cleaved has a plurality of structures represented by the Formula (1), and the number of nucleotides between the structures is 10 or less.

## Patentansprüche

1. Nukleinsäurestrangspaltungsverfahren, umfassend:
einen Nukleinsäurepräparationschritt, bei dem eine zu spaltende Nukleinsäure mit einer durch die folgende Formel (1) dargestellten Struktur präpariert wird, und
einen Spaltungsschritt, bei dem die zu spaltende Nukleinsäure mit einem Spaltungsmittel umgesetzt wird, um die zu spaltende Nukleinsäure am Teil X der Formel (1) zu spalten, um eine Nukleinsäure mit einer durch die folgende Formel (2) dargestellten Struktur zu erzeugen,
wobei das Spaltungsmittel ein Metallnanopartikel ist, das ein Atom aus der Gruppe bestehend aus Silber, Quecksilber und Cadmium enthält, 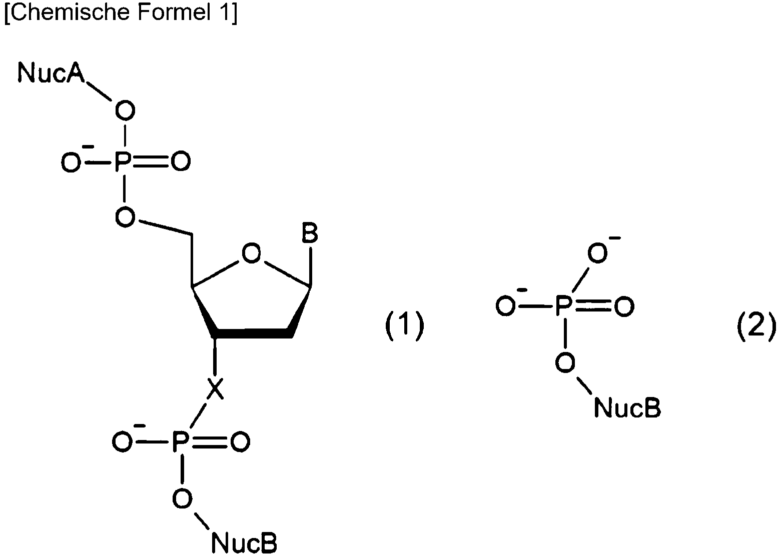
wobei B eine Base ist, X Schwefel oder Selen ist, NucA aus mindestens einem Nukleotid besteht, Teil der zu spaltenden Nukleinsäure ist und ein Teil am 5'-Ende, bezogen auf X, ist und NucB aus mindestens einem Nukleotid besteht, Teil der zu spaltenden Nukleinsäure ist und ein Teil am 3'-Ende, bezogen auf X ist.

2. Nukleinsäurestrangspaltungsverfahren gemäß Anspruch 1, wobei es sich bei dem Spaltungsmittel um Silbernanopartikel handelt und X Schwefel ist.

3. Nukleinsäurestrangspaltungsverfahren gemäß Anspruch 1, wobei die Metallnanopartikel eine mittlere Partikelgröße im Bereich von 1 bis 20 nm aufweisen.

4. Nukleinsäurestrangspaltungsverfahren gemäß Anspruch 1, wobei Polyethylenglykol an die Oberfläche der Metallnanopartikel gebunden ist.

5. Nukleinsäurestrangspaltungsverfahren gemäß Anspruch 1, wobei im Schritt der Nukleinsäurepräparation ein Teil oder die gesamte zu spaltende Nukleinsäure durch ein Phosphoramiditverfahren unter Verwendung eines Amiditreagenzes synthetisiert wird, dargestellt durch die folgenden Formeln (3) und (4): 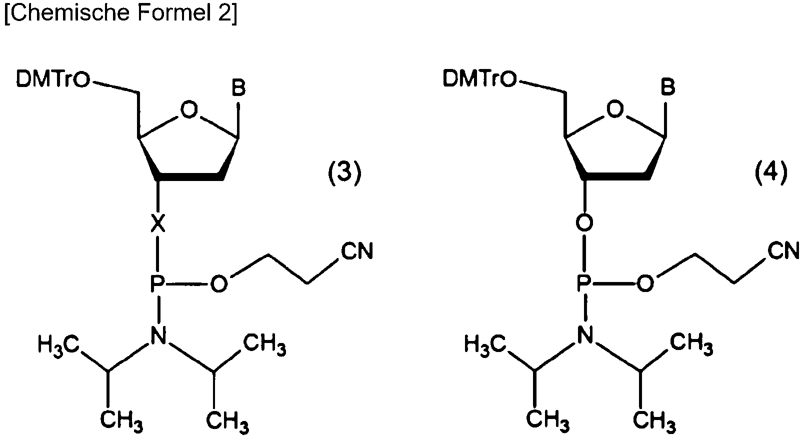 wobei B eine Base ist, X Schwefel oder Selen ist und DMTr eine Dimethoxytritylgruppe ist.

6. Nukleinsäurestrangspaltungsverfahren gemäß Anspruch 5, wobei der Nukleinsäurepräparationsschritt einschließt, 5-[3,5-Bis(trifluormethyl)phenyl]-1H-tetrazol als Aktivator durch ein Phosphoramiditverfahren zu synthetisieren.

7. Nukleinsäurestrangspaltungsverfahren gemäß Anspruch 5, wobei im Nukleinsäurepräparationsschritt ein Teil der zu spaltenden Nukleinsäure durch ein Phosphoramiditverfahren synthetisiert wird, um einen Primer zu bilden, eine Vielzahl von Zyklen einer Polymerasekettenreaktion unter Verwendung einer Matrizen-DNA mit einer zur zu spaltenden Nukleinsäure komplementären Sequenz als Matrize durchgeführt wird und der Primer entlang der Matrizen-DNA verlängert wird, um die zu spaltende Nukleinsäure zu erzeugen.

8. Nukleinsäurestrangspaltungsvorrichtung, umfassend:
Hilfsmittel zur Nukleinsäurepräparation, konfiguriert, um eine zu spaltende Nukleinsäure mit einer durch die folgende Formel (1) dargestellten Struktur zu präparieren, und
Hilfsmittel zur Spaltung, konfiguriert, um die zu spaltende Nukleinsäure mit einem Spaltungsmittel umzusetzen, um die zu spaltende Nukleinsäure am Teil X der Formel (1) zu spalten, um eine Nukleinsäure mit einer durch die folgende Formel (2) dargestellten Struktur zu erzeugen,
wobei das Spaltungsmittel ein Metallnanopartikel ist, das ein Atom enthält, ausgewählt aus der Gruppe bestehend aus Silber, Quecksilber und Cadmium, 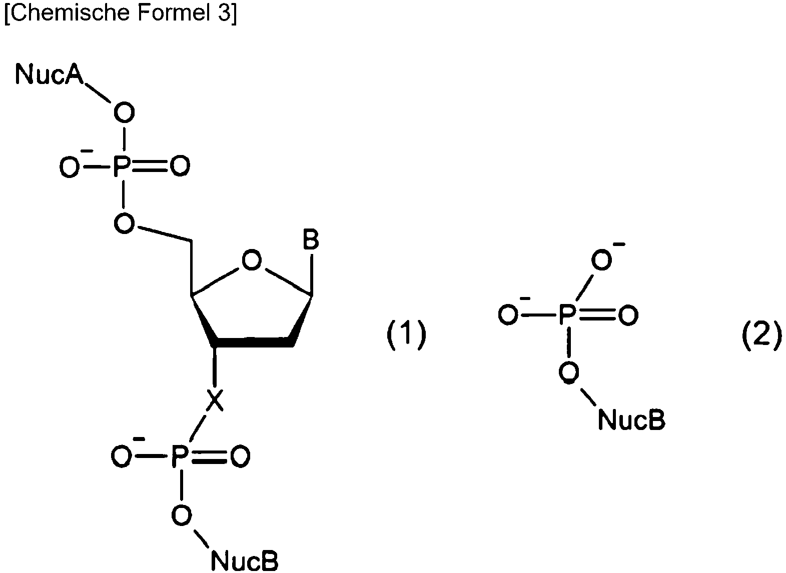
wobei B eine Base ist, X Schwefel oder Selen ist, NucA aus mindestens einem Nukleotid besteht, Teil der zu spaltenden Nukleinsäure ist und ein Teil am 5'-Ende in Bezug auf X ist und NucB aus mindestens einem Nukleotid besteht, Teil der zu spaltenden Nukleinsäure ist und ein Teil am 3'-Ende in Bezug auf X ist.

9. Nukleinsäurestrangspaltungsvorrichtung gemäß Anspruch 8, wobei das Nukleinsäurepräparations-Hilfsmittel konfiguriert ist, um einen Teil oder die gesamte zu spaltende Nukleinsäure durch ein Phosphoramiditverfahren unter Verwendung eines Amiditreagenzes der folgenden Formeln (3) und (4) zu synthetisieren, 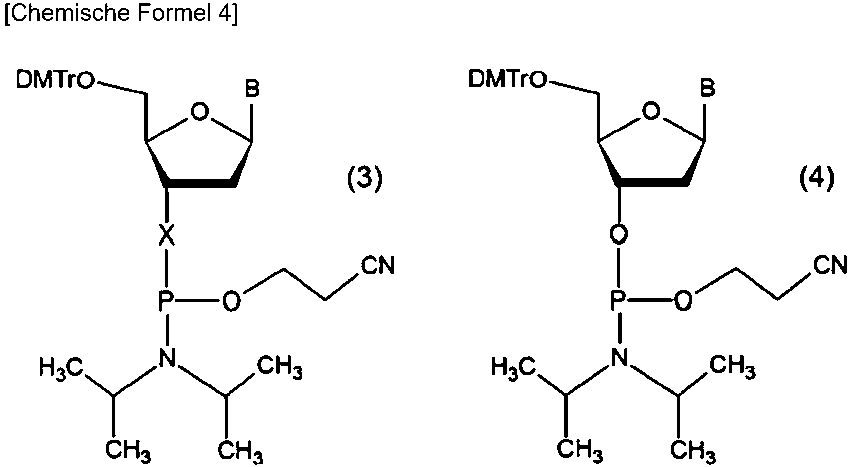 wobei B eine Base ist, X Schwefel oder Selen ist und DMTr eine Dimethoxytritylgruppe ist.

10. Nukleinsäurestrangspaltungsvorrichtung gemäß Anspruch 9, wobei das Nukleinsäurepräparations-Hilfsmittel konfiguriert ist, um 5-[3,5-Bis(trifluormethyl)phenyl]-1H-tetrazol als Aktivator durch ein Phosphoramiditverfahren zu synthetisieren.

11. Verfahren zum Herstellen doppelsträngiger DNA zum Herstellen doppelsträngiger DNA mit einem überlappenden Ende, wobei das Verfahren einschließt:
einen Schritt zur Präparation doppelsträngiger DNA, bei dem doppelsträngige DNA zur Spaltung präpariert wird, die einen Sense-Strang und einen Antisense-Strang mit einer zum Sense-Strang komplementären Sequenz einschließt, wobei die zu spaltende doppelsträngige DNA in mindestens einem aus dem Sense-Strang und dem Antisense-Strang eine Struktur aufweist, dargestellt durch die folgende Formel (1), und
einen Schritt zur Erzeugung eines überlappenden Endes, bei dem die zu spaltende doppelsträngige DNA mit einem Spaltungsmittel gespalten wird, um den Sense-Strang und/oder den Antisense-Strang an einem Abschnitt X in Formel (1) zu spalten, um doppelsträngige DNA mit einer durch die folgende Formel (2) dargestellten Struktur und einem überlappenden Ende zu erzeugen,
wobei das Spaltungsmittel ein Metallnanopartikel ist, das ein Atom, ausgewählt aus der Gruppe bestehend aus Silber, Quecksilber und Cadmium enthält, 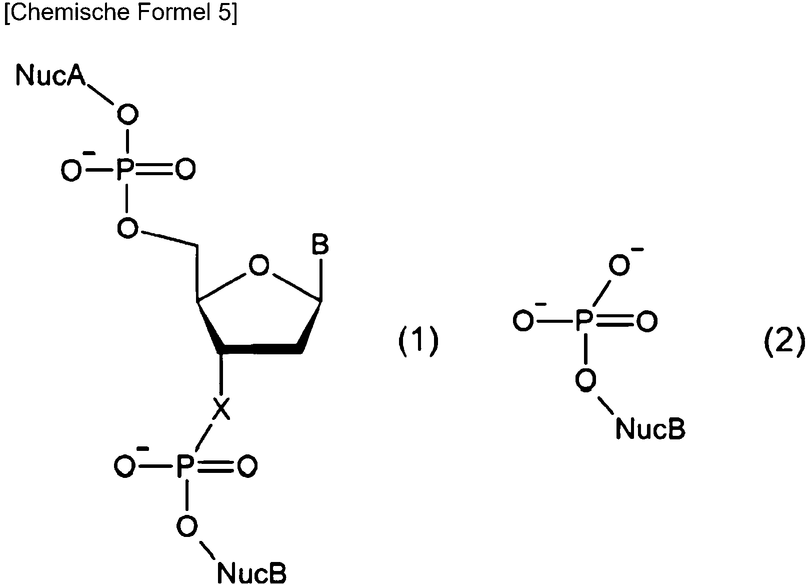
wobei B eine Base ist, X Schwefel oder Selen ist, NucA aus mindestens einem Nukleotid besteht, Teil der zu spaltenden Nukleinsäure ist und ein Abschnitt am 5'-Ende in Bezug auf X ist und NucB aus mindestens einem Nukleotid besteht, Teil der zu spaltenden Nukleinsäure ist und ein Abschnitt am 3'-Ende in Bezug auf X ist.

12. Verfahren zur Herstellung doppelsträngiger DNA gemäß Anspruch 11, wobei die zu spaltende doppelsträngige DNA eine Vielzahl von Strukturen aufweist, dargestellt durch die Formel (1), und die Anzahl der Nukleotide zwischen den Strukturen 10 oder weniger beträgt.

13. Vorrichtung zur Herstellung doppelsträngiger DNA zum Herstellen doppelsträngiger DNA mit einem überlappenden Ende, wobei die Vorrichtung Hilfsmittel zur Präparation doppelsträngiger DNA einschließt, konfiguriert um doppelsträngige, zu spaltende DNA zu präparieren, die einen Sense-Strang und einen Antisense-Strang mit einer zum Sense-Strang komplementären Sequenz einschließt, wobei die zu spaltende doppelsträngige DNA in mindestens einem aus dem Sense-Strang und dem Antisense-Strang eine Struktur, dargestellt durch die folgende Formel (1), aufweist, und ein Mittel zur Erzeugung eines überlappenden Endes, das die zu spaltende doppelsträngige DNA mit einem Spaltungsmittel umsetzt, um den Sense-Strang und/oder den Antisense-Strang an einem Abschnitt X in Formel (1) zu spalten, um doppelsträngige DNA mit einer durch die folgende Formel (2) dargestellten Struktur und mit einem überlappenden Ende zu erzeugen, wobei
das Spaltungsmittel ein Metallnanopartikel ist, das ein Atom, ausgewählt aus der Gruppe bestehend aus Silber, Quecksilber und Cadmium, enthält, 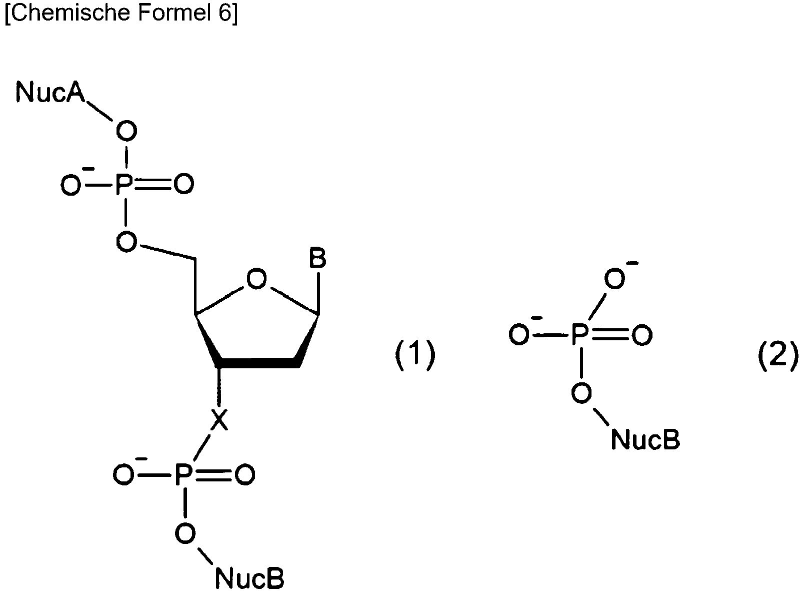
wobei B eine Base ist, X Schwefel oder Selen ist, NucA aus mindestens einem Nukleotid besteht, Teil der zu spaltenden Nukleinsäure ist und ein Abschnitt am 5'-Ende in Bezug auf X ist und NucB aus mindestens einem Nukleotid besteht, Teil der zu spaltenden Nukleinsäure ist und ein Abschnitt am 3'-Ende in Bezug auf X ist.

14. Vorrichtung zum Herstellen doppelsträngiger DNA gemäß Anspruch 13, wobei die zu spaltende doppelsträngige DNA eine Vielzahl von Strukturen, dargestellt durch Formel (1), aufweist und die Anzahl der Nukleotide zwischen den Strukturen 10 oder weniger beträgt.

## Revendications

1. Procédé de clivage d'un brin d'acide nucléique, comprenant :
une étape de préparation d'acide nucléique consistant à préparer un acide nucléique à cliver ayant une structure représentée par la Formule (1) suivante ; et
une étape de clivage consistant à faire réagir l'acide nucléique à cliver avec un agent de clivage pour cliver l'acide nucléique à cliver au niveau de la partie X de la Formule (1) afin de générer un acide nucléique ayant une structure représentée par la Formule (2) suivante,
l'agent de clivage est une nanoparticule de métal contenant un atome choisi dans le groupe constitué par l'argent, le mercure et le cadmium, 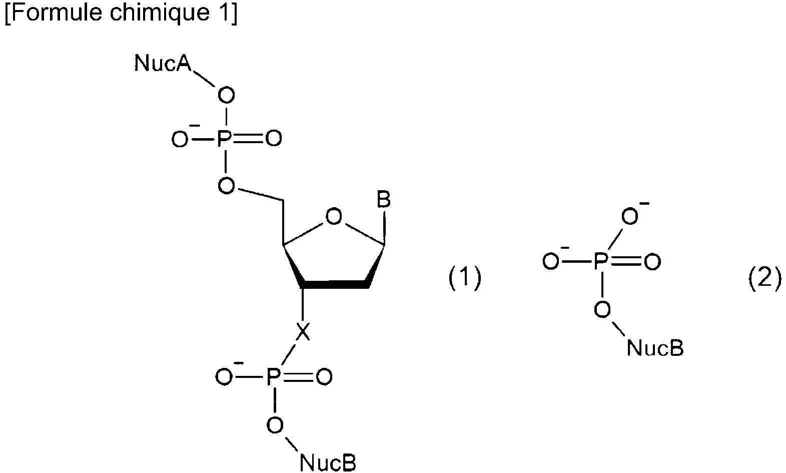
où B représente une base, X représente le soufre ou le sélénium, NucA est composé d'au moins un nucléotide, est une partie de l'acide nucléique à cliver et représente une partie du côté de l'extrémité 5' par rapport à X, et NucB est composé d'au moins un nucléotide, est une partie de l'acide nucléique à cliver et représente une partie du côté de l'extrémité 3' par rapport à X.

2. Procédé de clivage d'un brin d'acide nucléique selon la revendication 1, dans lequel l'agent de clivage est des nanoparticules d'argent, et X est le soufre.

3. Procédé de clivage d'un brin d'acide nucléique selon la revendication 1, dans lequel les nanoparticules de métal ont une granulométrie moyenne dans une plage de 1 à 20 nm.

4. Procédé de clivage d'un brin d'acide nucléique selon la revendication 1, dans lequel du polyéthylène glycol est lié à la surface de la nanoparticule de métal.

5. Procédé de clivage d'un brin d'acide nucléique selon la revendication 1, dans lequel à l'étape de préparation de l'acide nucléique, tout ou partie de l'acide nucléique à cliver est synthétisée par un procédé au phosphoramidite à l'aide d'un réactif amidite représenté par la Formule (3) et la Formule (4) suivantes, 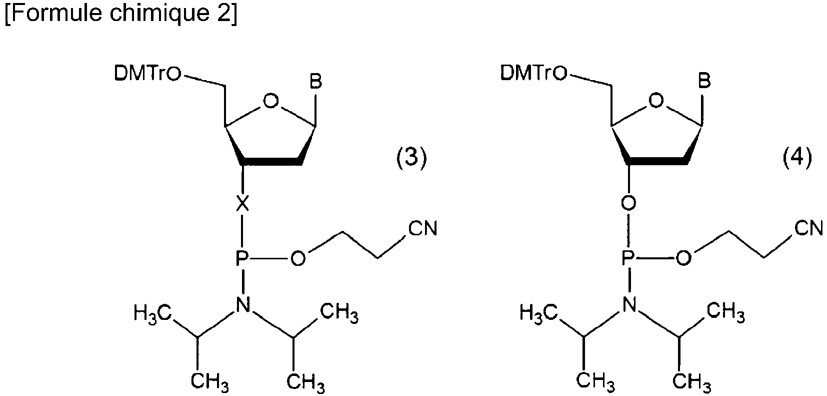 où B représente une base, X représente le soufre ou le sélénium et DMTr représente un groupe diméthoxytrityle.

6. Procédé de clivage d'un brin d'acide nucléique selon la revendication 5, dans lequel l'étape de préparation de l'acide nucléique comporte la synthèse de 5-[3,5-bis(trifluorométhyl)phényl]-1H-tétrazole en tant qu'activateur par un procédé au phosphoramidite.

7. Procédé de clivage d'un brin d'acide nucléique selon la revendication 5, dans lequel à l'étape de préparation de l'acide nucléique, une partie de l'acide nucléique à cliver est synthétisée par un procédé au phosphoramidite afin de former une amorce, une pluralité de cycles d'amplification en chaîne par polymérase est effectuée à l'aide d'un ADN matrice ayant une séquence complémentaire de l'acide nucléique à cliver en tant que matrice, et l'amorce est amplifiée le long de l'ADN matrice afin de générer l'acide nucléique à cliver.

8. Dispositif de clivage d'un brin d'acide nucléique, comprenant :
un moyen de préparation d'acide nucléique configuré pour préparer un acide nucléique à cliver ayant une structure représentée par la Formule (1) suivante ; et
un moyen de clivage configuré pour faire réagir l'acide nucléique à cliver avec un agent de clivage pour cliver l'acide nucléique à cliver au niveau de la partie X de la Formule (1) afin de générer un acide nucléique ayant une structure représentée par la Formule (2) suivante,
dans lequel
l'agent de clivage est une nanoparticule de métal contenant un atome choisi dans le groupe constitué par l'argent, le mercure et le cadmium, 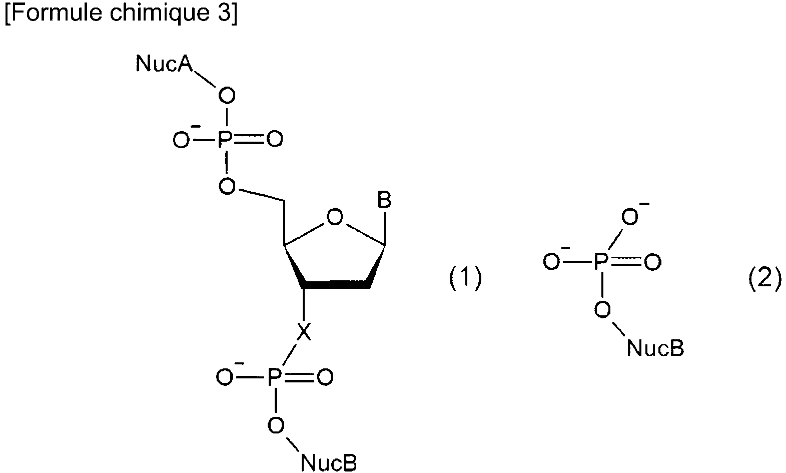
où B représente une base, X représente du soufre ou du sélénium, NucA est composé d'au moins un nucléotide, est une partie de l'acide nucléique à cliver et représente une partie du côté de l'extrémité 5' par rapport à X, et NucB est composé d'au moins un nucléotide, est une partie de l'acide nucléique à cliver et représente une partie du côté de l'extrémité 3' par rapport à X.

9. Dispositif de clivage d'un brin d'acide nucléique selon la revendication 8, dans lequel le moyen de préparation de l'acide nucléique est configuré pour synthétiser tout ou partie de l'acide nucléique à cliver par un procédé au phosphoramidite à l'aide d'un réactif amidite représenté par la Formule (3) et la Formule (4) suivantes, 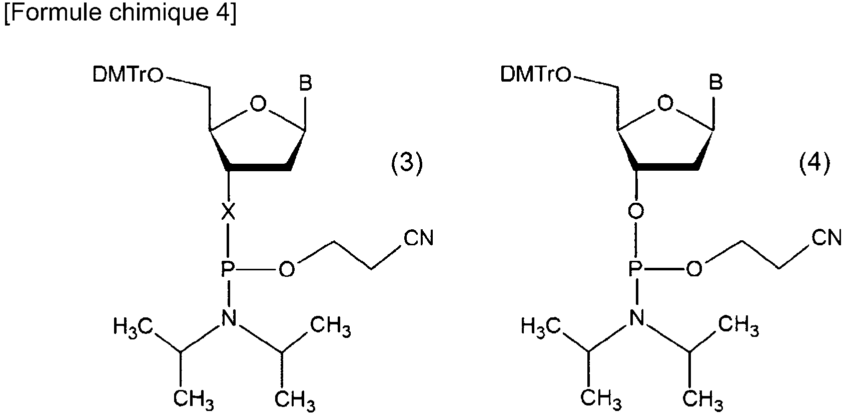 où B représente une base, X représente le soufre ou le sélénium et DMTr représente un groupe diméthoxytrityle.

10. Dispositif de clivage d'un brin d'acide nucléique selon la revendication 9, dans lequel le moyen de préparation de l'acide nucléique est configuré pour synthétiser du 5-[3,5-bis(trifluorométhyl)]]-1H-tétrazole en tant qu'activateur par un procédé au phosphoramidite.

11. Procédé de production d'ADN double brin permettant de produire de l'ADN double brin ayant une extrémité collante, le procédé comprenant :
une étape de préparation d'ADN double brin consistant à préparer un ADN double brin à cliver comportant un brin sens et un brin antisens ayant une séquence complémentaire du brin sens, l'ADN double brin à cliver ayant une structure représentée par la Formule (1) suivante dans l'un au moins du brin sens et du brin antisens ; et
une étape de génération d'extrémité collante consistant à faire réagir l'ADN double brin à cliver avec un agent de clivage pour cliver le brin sens et/ou le brin antisens au niveau d'une partie X dans la Formule (1) afin de générer de l'ADN double brin ayant une structure représentée par la Formule (2) suivante et ayant une extrémité collante,
l'agent de clivage est une nanoparticule de métal contenant un atome choisi dans le groupe constitué par l'argent, le mercure et le cadmium, 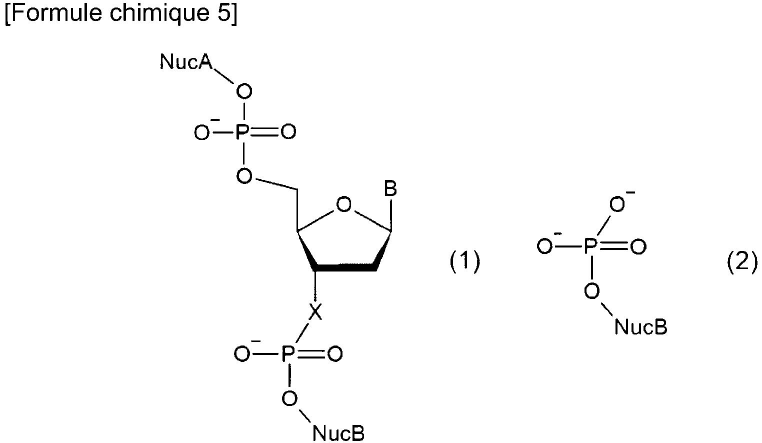
où B représente une base, X représente le soufre ou le sélénium, NucA est composé d'au moins un nucléotide, est une partie de l'acide nucléique à cliver et représente une partie du côté de l'extrémité 5' par rapport à X, et NucB est composé d'au moins un nucléotide, est une partie de l'acide nucléique à cliver et représente une partie du côté de l'extrémité 3' par rapport à X.

12. Procédé de production d'ADN double brin selon la revendication 11, dans lequel l'ADN double brin à cliver a une pluralité de structures représentées par la Formule (1), et le nombre de nucléotides entre les structures est inférieur ou égal à 10.

13. Dispositif de production d'ADN double brin permettant de produire de l'ADN double brin ayant une extrémité collante, le dispositif comportant un moyen de préparation d'ADN double brin configuré pour préparer de l'ADN double brin à cliver comportant un brin sens et un brin antisens ayant une séquence ayant une séquence complémentaire du brin sens, l'ADN double brin à cliver ayant une structure représentée par la Formule (1) suivante dans l'un au moins du brin sens et du brin antisens, et un moyen de génération d'extrémité collante qui fait réagir l'ADN double brin à cliver avec un agent de clivage pour cliver le brin sens et/ou le brin antisens au niveau d'une partie X dans la Formule (1) afin de générer de l'ADN double brin ayant une structure représentée par la Formule (2) suivante et ayant une extrémité collante, dans lequel
l'agent de clivage est une nanoparticule de métal contenant un atome choisi dans le groupe constitué par l'argent, le mercure et le cadmium, 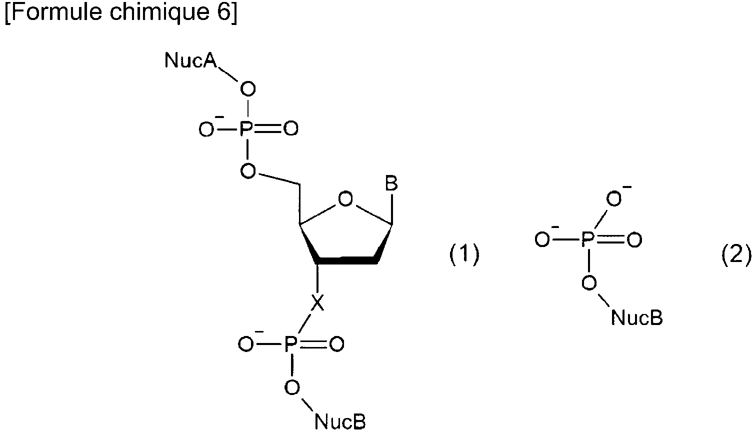 où B représente une base, X représente le soufre ou le sélénium, NucA est composé d'au moins un nucléotide, est une partie de l'acide nucléique à cliver et représente une partie du côté de l'extrémité 5' par rapport à X, et NucB est composé d'au moins un nucléotide, est une partie de l'acide nucléique à cliver et représente une partie du côté de l'extrémité 3' par rapport à X.

14. Dispositif de production d'ADN double brin selon la revendication 13, dans lequel l'ADN double brin à cliver a une pluralité de structures représentées par la Formule (1), et le nombre de nucléotides entre les structures est inférieur ou égal à 10.
